(19) Europäisches Patentamt
European Patent Office
Office européen des brevets

(11) EP 1 485 378 B1

(12) **EUROPEAN PATENT SPECIFICATION**

(45) Date of publication and mention of the grant of the patent:
**18.06.2008 Bulletin 2008/25**

(21) Application number: **03711979.9**

(22) Date of filing: **11.03.2003**

(51) Int Cl.:
***C07D 413/04*** *(2006.01)* ***C07D 403/04*** *(2006.01)*
***C07D 401/04*** *(2006.01)* ***A61K 31/506*** *(2006.01)*
***A61P 35/00*** *(2006.01)*

(86) International application number:
**PCT/EP2003/002510**

(87) International publication number:
**WO 2003/076438 (18.09.2003 Gazette 2003/38)**

(54) **PIPERAZINYL-, PIPERIDINYL- AND MORPHOLINYL-DERIVATIVES AS NOVEL INHIBITORS OF HISTONE DEACETYLASE**

PIPERAZINYL-, PIPERIDINYL- UND MORPHOLINYLDERIVATE ALS NEUE INHIBITOREN VON HISTON-DEACETYLASE

DERIVES PIPERAZINYL-, PIPERIDINYL- ET MORPHOLINYL- UTILISES EN TANT QUE NOUVEAUX INHIBITEURS D'HISTONE DEACETYLASE

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **13.03.2002 US 363799 P**
**23.12.2002 PCT/EP02/14833**

(43) Date of publication of application:
**15.12.2004 Bulletin 2004/51**

(73) Proprietor: **Janssen Pharmaceutica NV**
**2340 Beerse (BE)**

(72) Inventor: **VAN EMELEN, Kristof,**
**Janssen Pharmaceutica N.V.**
**B-2340 Beerse (BE)**

(56) References cited:
**WO-A-01/38322** **WO-A-98/55449**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 485 378 B1

Printed by Jouve, 75001 PARIS (FR)

## Description

This invention concerns compounds having histone deacetylase (HDAC) inhibiting enzymatic activity. It further relates to processes for their preparation, to compositions comprising them, as well as their use, both *in vitro* and *in vivo,* to inhibit HDAC and as a medicine, for instance as a medicine to inhibit proliferative conditions, such as cancer and psoriasis.

In all eukaryotic cells, genomic DNA in chromatine associates with histones to form nucleosomes. Each nucleosome consists of a protein octamer made up of two copies of each histones H2A, H2B, H3 and H4. DNA winds around this protein core, with the basic amino acids of the histones interacting with the negatively charged phosphate groups of the DNA. The most common posttranslational modification of these core histones is the reversible acetylation of the ε-amino groups of conserved, highly basic N-terminal lysine residues. The steady state of histone acetylation is established by the dynamic equilibrium between competing histone acetyltransferase(s) and histone deacetylase(s) herein referred to as "HDAC". Histone acetylation and deacetylation has long been linked to transcriptional control. The recent cloning of the genes encoding different histone acetyltransferases and histone deacetylases provided a possible explanation for the relationship between histone acetylation and transcriptional control. The reversible acetylation of histones can result in chromatin remodelling and as such act as a control mechanism for gene transcription. In general, hyperacetylation of histones facilitates gene expression, whereas histone deacetylation is correlated with transcriptional repression. Histone acetyltransferases were shown to act as transcriptional coactivators, whereas histone deacetylases were found to belong to transcriptional repression pathways.

The dynamic equilibrium between histone acetylation and deacetylation is essential for normal cell growth. Inhibition of histone deacetylase results in cell cycle arrest, cellular differentiation, apoptosis and reversal of the transformed phenotype. Therefore HDAC inhibitors can have great therapeutic potential in the treatment of cell proliferative diseases or conditions (Marks et al., Nature Reviews: Cancer 1: 194-202, 2001)

The study of inhibitors of histone deacetylases (HDAC) indicates that indeed these enzymes play an important role in cell proliferation and differentiation. The inhibitor Trichostatin A (TSA) causes cell cycle arrest at both G1 and G2 phases, reverts the transformed phenotype of different cell lines, and induces differentiation of Friend leukemia cells and other. TSA (and suberoylanilide hydroxamic acid SAHA) have been reported to inhibit cell growth, induce terminal differentiation, and prevent the formation of tumours in mice (Finnin et al., Nature, 401: 188-193, 1999).

Trichostatin A has also been reported to be useful in the treatment of fibrosis, e.g. liver fibrosis and liver chirrhosis. (Geerts et al., European Patent Application EP 0 827 742, published 11 March, 1998).

Patent application WO01/38322 published on May 31, 2001 discloses amongst others inhibitors of histone deacetylase of general formula Cy-$L^1$-Ar-$Y^1$-C(O)-NH-Z, providing compositions and methods for treating cell proliferative diseases and conditions.

Patent application WO01/70675 published on 27 September, 2001 discloses inhibitors of histone deacetylase of formula $Cy^2$-$Cy^1$-X- $Y^1$-W and Cy-$S(O)_2$-NH-$Y^3$-W and further provides compositions and methods for treating cell proliferative diseases and conditions.

The problem to be solved is to provide histone deacetylase inhibitors with high enzymatic activity and also show advantageous properties such as cellular activity and increased bioavailability, preferably oral bioavailability, and have little or no side effects.

The novel compounds of the present invention solve the above described problem. The compounds differ from the prior art in structure.

The compounds of the present invention show excellent *in-vitro* histone deacetylase inhibiting enzymatic activity. The present compounds have advantageous properties with regard to cellular activity and specific properties with regard to inhibition of cell cycle progression at both G1 and G2 checkpoints (p21 induction capacity). The compounds of the present invention show good metabolic stability and high bioavailability and more particular they show oral bioavailability.

This invention concerns compounds of formula (I)

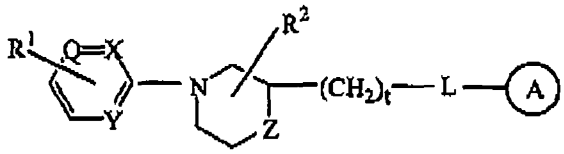 (I)

the *N*-oxide forms, the pharmaceutically acceptable addition salts and the stereochemically isomeric forms thereof, wherein

t is 0, 1, 2, 3 or 4 and when t is 0 then a direct bond is intended;
each Q is nitrogen or

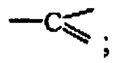

each X is nitrogen or

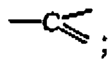

each Y is nitrogen or

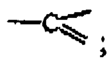

each Z is -NH-, -0- or $-CH_2-$;

$R^1$ is $-C(O)NR^3R^4$, $-NHC(O)R^7$, -C(O)-$C_{1-6}$alkanediylS$R^7$, $-NR^8C(O)N(OH)R^7$, $-NR^8C(O)C_{1-6}$alkanediyl$SR^7$ or $-NR^8C(O)C=N(OH)R^7$
wherein $R^3$ and $R^4$ are each independently selected from hydrogen, hydroxy, $C_{1-6}$alkyl, hydroxy$C_{1-6}$alkyl, amino$C_{1-6}$alkyl or aminoaryl;
$R^7$ is hydrogen, $C_{1-6}$alkyl, $C_{1-6}$alkylcarbonyl, aryl$C_{1-6}$alkyl, $C_{1-6}$alkylpyrazinyl, pyridinone, pyrrolidinone or methylimidazolyl;
$R^8$ is hydrogen or $C_{1-6}$alkyl;

$R^2$ is hydrogen, hydroxy, amino, hydroxy$C_{1-6}$alkyl, $C_{1-6}$alkyl, $C_{1-6}$alkyloxy, aryl$C_{1-6}$alkyl, aminocarbonyl, hydroxycarbonyl, amino$C_{1-6}$alkyl, aminocarbonyl$C_{1-6}$alkyl, hydroxycarbonyl$C_{1-6}$alkyl, hydroxyaminocarbonyl, $C_{1-6}$alkyloxycarbonyl, $C_{1-6}$alkylamino$C_{1-6}$alkyl or di($C_{1-6}$alkyl)amino$C_{1-6}$alkyl;

-L- is a bivalent radical selected from $-NR^9C(O)-$, $-NR^9SO_2-$ or $-NR^9CH_2-$wherein $R^9$ is hydrogen, $C_{1-6}$alkyl, $C_{3-10}$cycloalkyl, hydroxy$C_{1-6}$alkyl, $C_{1-6}$alkyloxy$C_{1-6}$alkyl or di($C_{1-6}$alkyl)amino$C_{1-6}$alkyl;

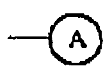

is a radical selected from

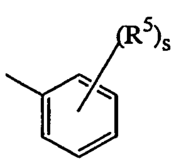

(a-1)

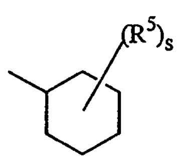

(a-2)

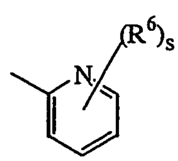

(a-3)

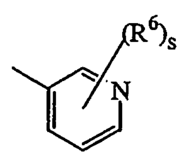

(a-4)

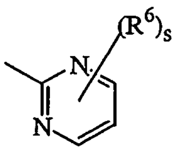

(a-5)

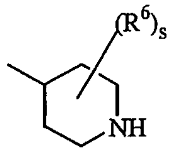

(a-6)

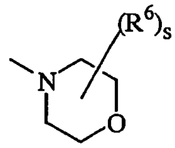

(a-7)

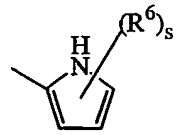

(a-8)

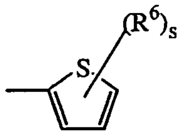

(a-9)

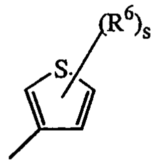

(a-10)

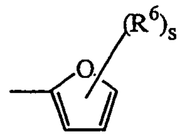

(a-11)

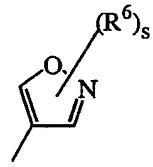

(a-12)

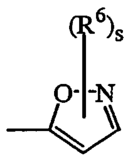

(a-13)

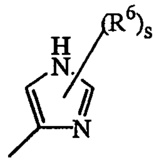

(a-14)

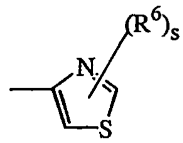

(a-15)

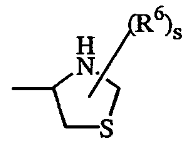

(a-16)

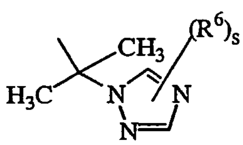

(a-17)

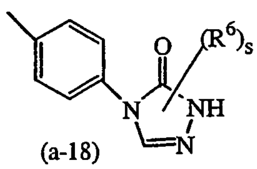

(a-18)

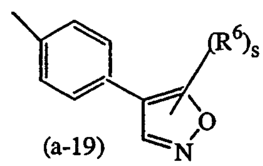

(a-19)

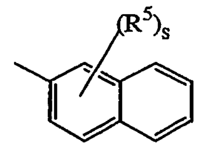

(a-20)

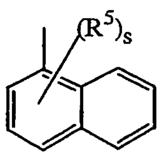

(a-21)

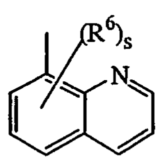

(a-22)

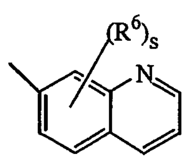

(a-23)

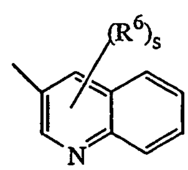

(a-24)

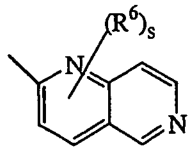
(a-25)

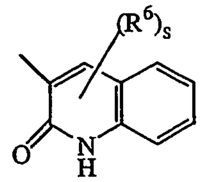
(a-26)

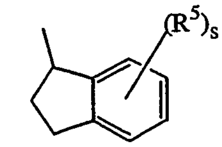
(a-27)

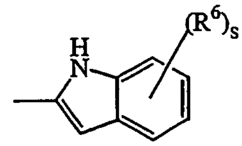
(a-28)

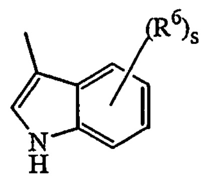
(a-29)

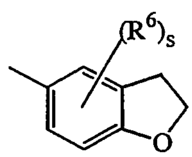
(a-30)

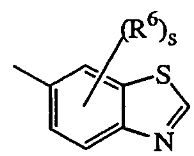
(a-31)

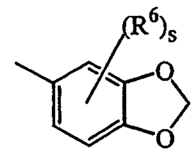
(a-32)

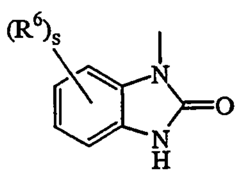
(a-33)

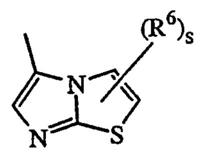
(a-34)

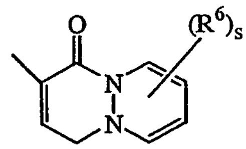
(a-35)

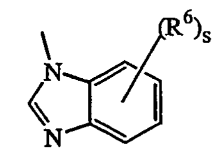
(a-36)

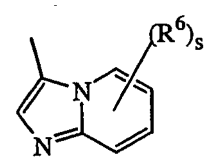
(a-37)

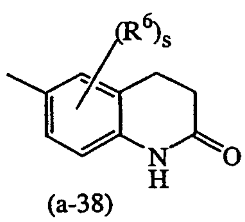
(a-38)

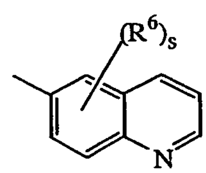
(a-39)

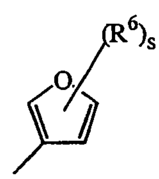
(a-40)

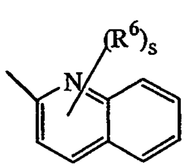
(a-41)

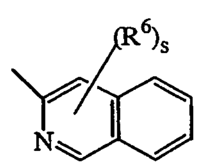
(a-42)

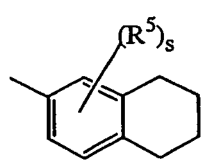
(a-43)

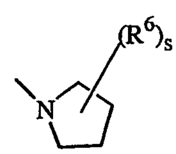
(a-44)

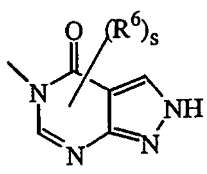

(a-45)

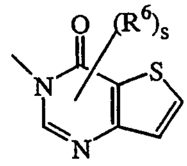

(a-46)

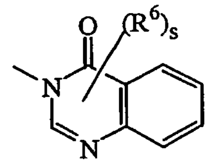

(a-47)

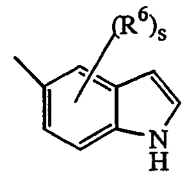

(a-48)

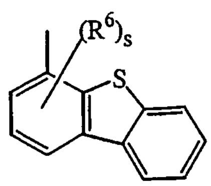

(a-49)

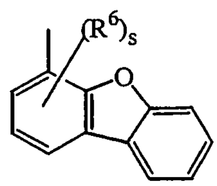

(a-50)

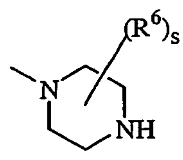

(a-51)

wherein each s is independently 0, 1, 2, 3, 4 or 5;

each $R^5$ and $R^6$ are independently selected from hydrogen; halo; hydroxy; amino; nitro; trihalo$C_{1-6}$alkyl; trihalo$C_{1-6}$alkyloxy; $C_{1-6}$alkyl; $C_{1-6}$alkyl substituted with aryl and $C_{3-10}$cycloalkyl; $C_{1-6}$alkyloxy; $C_{1-6}$alkyloxy$C_{1-6}$alkyloxy; $C_{1-6}$alkylcarbonyl; $C_{1-6}$alkyloxycarbonyl; $C_{1-6}$alkylsulfonyl; cyano$C_{1-6}$alkyl; hydroxy$C_{1-6}$alkyl; hydroxy$C_{1-6}$alkyloxy; hydroxy$C_{1-6}$alkylamino; amino$C_{1-6}$alkyloxy; di($C_{1-6}$alkyl)aminocarbonyl; di(hydroxy$C_{1-6}$alkyl)amino; (aryl)($C_{1-6}$alkyl)amino; di($C_{1-6}$alkyl)amino$C_{1-6}$alkyloxy; di($C_{1-6}$alkyl)amino$C_{1-6}$alkylamino; di($C_{1-6}$alkyl)amino$C_{1-6}$alkylamino$C_{1-6}$alkyl; arylsulfonyl; arylsulfonylamino; aryloxy; aryloxy$C_{1-6}$alkyl; aryl$C_{2-6}$alkenediyl; di($C_{1-6}$alkyl)amino; di($C_{1-6}$alkyl)amino$C_{1-6}$alkyl; di($C_{1-6}$alkyl)amino($C_{1-6}$alkyl)amino; di($C_{1-6}$alkyl)amino($C_{1-6}$alkyl)amino$C_{1-6}$alkyl; di($C_{1-6}$alkyl)amino$C_{1-6}$alkyl($C_{1-6}$alkyl)amino; di($C_{1-6}$alkyl)amino$C_{1-6}$alkyl($C_{1-6}$alkyl)amino$C_{1-6}$alkyl; aminosulfonylamino($C_{1-6}$alkyl)amino; aminosulfonylamino($C_{1-6}$alkyl)amino$C_{1-6}$alkyl; di($C_{1-6}$alkyl)aminosulfonylamino($C_{1-6}$alkyl)amino; di($C_{1-6}$alkyl)aminosulfonylamino($C_{1-6}$alkyl)amino$C_{1-6}$alkyl; cyano; thiophenyl; thiophenyl substituted with di($C_{1-6}$alkyl)amino$C_{1-6}$alkyl($C_{1-6}$alkyl)amino$C_{1-6}$alkyl, di($C_{1-6}$alkyl)amino$C_{1-6}$alkyl, $C_{1-6}$alkylpiperazinyl$C_{1-6}$alkyl, hydroxy$C_{1-6}$alkylpiperazinyl$C_{1-6}$alkyl, hydroxy$C_{1-6}$alkyloxy $C_{1-6}$alkylpiperazinyl$C_{1-6}$alkyl, di($C_{1-6}$alkyl)aminosulfonylpiperazinyl$C_{1-6}$alkyl, $C_{1-6}$alkyloxypiperidinyl, $C_{1-6}$alkyloxypiperidinyl$C_{1-6}$alkyl, morpholinyl$C_{1-6}$alkyl, hydroxy$C_{1-6}$alkyl($C_{1-6}$alkyl)amino$C_{1-6}$alkyl, or di(hydroxy$C_{1-6}$alkyl)amino$C_{1-6}$alkyl; furanyl; furanyl substituted with hydroxy$C_{1-6}$alkyl; benzofuranyl; imidazolyl; oxazolyl; oxazolyl substituted with aryl and $C_{1-6}$alkyl; $C_{1-6}$alkyltriazolyl; tetrazolyl; pyrrolidinyl; pyrrolyl; piperidinyl$C_{1-6}$alkyloxy; morpholinyl; $C_{1-6}$alkylmorpholinyl; morpholinyl$C_{1-6}$alkyloxy; morpholinyl$C_{1-6}$alkyl; morpholinyl$C_{1-6}$alkylamino; morpholinyl$C_{1-6}$ alkylamino$C_{1-6}$alkyl; piperazinyl; $C_{1-6}$alkylpiperazinyl; $C_{1-6}$alkylpiperazinyl$C_{1-6}$alkyloxy; piperazinyl$C_{1-6}$alkyl; naphtalenylsulfonylpiperazinyl; naphtalenylsulfonylpiperidinyl; naphtalenylsulfonyl: $C_{1-6}$alkylpiperazinyl$C_{1-6}$alkyl; $C_{1-6}$alkylpiperazinyl$C_{1-6}$alkylamino; $C_{1-6}$alkylpiperazinyl$C_{1-6}$alkylamino$C_{1-6}$alkyl; $C_{1-6}$alkylpiperazinylsulfonyl; aminosulfonylpiperazinyl$C_{1-6}$alkyloxy; aminosulfonylpiperazinyl; aminosulfonylpiperazinyl$C_{1-6}$alkyl; di($C_{1-6}$alkyl)aminosulfonylpiperazinyl; di($C_{1-6}$alkyl)aminosulfonylpiperazinyl$C_{1-6}$alkyl; hydroxy$C_{1-6}$alkylpiperazinyl; hydroxy$C_{1-6}$alkylpiperazinyl $C_{1-6}$alkyl; $C_{1-6}$alkyloxypiperidinyl; $C_{1-6}$alkyloxypiperidinyl$C_{1-6}$alkyl; piperidinylamino$C_{1-6}$alkylamino; piperidinylamino$C_{1-6}$ alkylamino$C_{1-6}$alky; ($C_{1-6}$alkylpiperidinyl)(hydroxy$C_{1-6}$alkyl)amino$C_{1-6}$alkylamino; ($C_{1-6}$alkylpiperidinyl)(hydroxy$C_{1-6}$alkyl)amino$C_{1-6}$alkylamino$C_{1-6}$alkyl; hydroxy$C_{1-6}$alkyloxy$C_{1-6}$alkylpiperazinyl; hydroxy$C_{1-6}$alkyloxy$C_{1-6}$alkylpiperazinyl$C_{1-6}$ alkyl; (hydroxy$C_{1-6}$alkyl)($C_{1-6}$alkyl)amino; (hydroxy$C_{1-6}$alkyl)($C_{1-6}$alkyl)amino$C_{1-6}$alkyl; hydroxy $C_{1-6}$alkylamino$C_{1-6}$alkyl; di(hydroxy$C_{1-6}$alkyl)amino$C_{1-6}$alkyl; pyrrolidinyl$C_{1-6}$alkyl; pyrrolidinyl$C_{1-6}$alkyloxy; pyrazolyl; thiopyrazolyl; pyrazolyl substituted with two substituents selected from $C_{1-6}$alkyl or trihalo$C_{1-6}$alkyl; pyridinyl; pyridinyl substituted with $C_{1-6}$alkyloxy, aryloxy or aryl; pyrimidinyl; tetrahydropyrimidinylpiperazinyl; tetrahydro- pyrimidinylpiperazinyl$C_{1-6}$alkyl; quinolinyl; indole; phenyl; phenyl substituted with one, two or three substituents independently selected from halo, amino, nitro, $C_{1-6}$alkyl, $C_{1-6}$alkyloxy, hydroxy$C_{1-4}$alkyl, trifluoromethyl, trifluoromethyloxy, hydroxy$C_{1-4}$alkyloxy, $C_{1-4}$alkylsulfonyl, $C_{1-4}$alkyloxy$C_{1-4}$ alkyloxy, $C_{1-4}$alkyloxycarbonyl, aminoe$C_{1-4}$alkyloxy, di($C_{1-4}$alkyl)amino$C_{1-4}$alkyloxy, di($C_{1-4}$alkyl)amino, di($C_{1-4}$alkyl)aminocarbonyl, di($C_{1-4}$alkyl)amino$C_{1-4}$alkyl, di($C_{1-4}$alkyl)amino$C_{1-4}$alkylamino$C_{1-4}$alkyl, di($C_{1-4}$alkyl)amino($C_{1-4}$alkyl)amino, di($C_{1-4}$alkyl)amino($C_{1-4}$alkyl)amino$C_{1-4}$alkyl, di($C_{1-4}$alkyl)amino$C_{1-4}$alkyl($C_{1-4}$alkyl)amino, di($C_{1-4}$alkyl)amino$C_{1-4}$alkyl ($C_{1-4}$alkyl)amino$C_{1-4}$alkyl, aminosulfonylamino($C_{1-4}$alkyl)amino, aminosulfonylamino($C_{1-4}$alkyl)amino$C_{1-4}$alkyl, di ($C_{1-4}$alkyl)aminosulfonylamino($C_{1-4}$alkyl)amino, di($C_{1-4}$alkyl)aminosulfonylamino($C_{1-4}$alkyl)amino$C_{1-6}$alkyl, cyano,

piperidinyl$C_{1-4}$alkyloxy, pyrrolidinyl$C_{1-4}$alkyloxy, aminosulfonylpiperazinyl, aminosulfonylpiperazinyl$C_{1-4}$alkyl, di($C_{1-4}$alkyl)aminosulfonylpiperazinyl, di($C_{1-4}$alkyl)aminosulfonylpiperazinyl$C_{1-4}$alkyl, hydroxy$C_{1-4}$alkylpiperazinyl, hydroxy$C_{1-4}$alkylpiperazinyl$C_{1-4}$alkyl, $C_{1-4}$alkyloxypiperidinyl, $C_{1-4}$alkyloxypiperidinyl$C_{1-4}$alkyl, hydroxy$C_{1-4}$alkyloxy$C_{1-4}$alkylpiperazinyl, hydroxy$C_{1-4}$alkyloxy$C_{1-4}$alkylpiperazinyl $C_{1-4}$alkyl, (hydroxy$C_{1-4}$alkyl)($C_{1-4}$alkyl)amino, (hydroxy$C_{1-4}$alkyl)($C_{1-4}$alkyl)amino$C_{1-4}$alkyl, di(hydroxy$C_{1-4}$alkyl)amino, di(hydroxy$C_{1-4}$alkyl)amino$C_{1-4}$alkyl, furanyl, furanyl substituted with -CH=CH-CH=CH-, pyrrolidinyl$C_{1-4}$alkyl, pyrrolidinyl$C_{1-4}$alkyloxy, morpholinyl, morpholinyl$C_{1-4}$alkyloxy, moxpholinyl$C_{1-4}$alkyl, morpholinyl$C_{1-4}$alkylamino, morpholinyl$C_{1-4}$alkylamino$C_{1-4}$alkyl, piperazinyl, $C_{1-4}$alkylpiperazinyl, $C_{1-4}$alkylpiperazinyl$C_{1-4}$alkyloxy, piperazinyl $C_{1-4}$alkyl, $C_{1-4}$alkylpiperazinyl$C_{1-4}$alkyl, $C_{1-4}$alkylpiperazinyl$C_{1-4}$alkylamino, $C_{1-4}$alkylpiperazinyl $C_{1-4}$alkylamino$C_{1-6}$alkyl, tetrahydropyrimidinylpiperazinyl, tetrahydropyrimidinylpiperazinyl$C_{1-4}$alkyl, piperidinylamino$C_{1-4}$alkylamino, piperidinylamino$C_{1-4}$alkylamino$C_{1-4}$alkyl, ($C_{1-4}$alkylpiperidinyl)(hydroxy$C_{1-4}$alkyl)amino$C_{1-4}$alkylamino, ($C_{1-4}$alkylpiperi- dinyl)(hydroxy$C_{1-4}$alkyl)amino$C_{1-4}$alkylamino$C_{1-4}$alkyl, pyridinyl$C_{1-4}$alkyloxy, hydroxy$C_{1-4}$alkylamino, hydroxy $C_{1-4}$alkylamino$C_{1-4}$alkyl, di($C_{1-4}$alkyl)amino$C_{1-4}$alkylamino, aminothiadiazolyl, aminosulfonylpiperazinyl$C_{1-4}$alkyloxy, or thiophenyl$C_{1-4}$alkylamino;

each $R^5$ and $R^6$ can be placed on the nitrogen in replacement of the hydrogen;

aryl in the above is phenyl, or phenyl substituted with one or more substituents each independently selected from halo, $C_{1-6}$alkyl, $C_{1-6}$alkyloxy, trifluoromethyl, cyano or hydroxycarbonyl.

The term "histone deacetylase inhibitor" or "inhibitor of histone deacetylase" is used to identify a compound, which is capable of interacting with a histone deacetylase and inhibiting its activity, more particularly its enzymatic activity. Inhibiting histone deacetylase enzymatic activity means reducing the ability of a histone deacetylase to remove an acetyl group from a histone. Preferably, such inhibition is specific, i.e. the histone deacetylase inhibitor reduces the ability of a histone deacetylase to remove an acetyl group from a histone at a concentration that is lower than the concentration of the inhibitor that is required to produce some other, unrelated biological effect.

As used in the foregoing definitions and hereinafter, halo is generic to fluoro, chloro, bromo and iodo; $C_{1-4}$alkyl defines straight and branched chain saturated hydrocarbon radicals having from 1 to 4 carbon atoms such as, e.g. methyl, ethyl, propyl, butyl, 1-methylethyl, 2-methylpropyl and the like; $C_{1-6}$alkyl includes $C_{1-4}$alkyl and the higher homologues thereof having 5 to 6 carbon atoms such as, for example, pentyl, 2-methylbutyl, hexyl, 2-methylpentyl and the like; $C_{1-6}$alkanediyl defines bivalent straight and branched chained saturated hydrocarbon radicals having from 1 to 6 carbon atoms such as, for example, methylene, 1,2-ethanediyl, 1,3-propanediyl 1,4-butanediyl, 1,5-pentanediyl, 1,6-hexanediyl and the branched isomers thereof such as, 2-methylpentanediyl, 3-methylpentanediyl, 2,2-dimethylbutanediyl, 2,3-diraethylbutanediyl and the like; trihalo$C_{1-6}$alkyl defines $C_{1-6}$alkyl containing three identical or different halo substituents for example trifluoromethyl; $C_{2-6}$alkenediyl defines bivalent straight and branched chain hydrocarbon radicals containing one double bond and having from 2 to 6 carbon atoms such as, for example, ethenediyl, 2-propenediyl, 3-butenediyl, 2-pentenediyl, 3-pentenediyl, 3-methyl-2-butenediyl, and the like; aminoaryl defines aryl substituted with amino; and $C_{3-10}$cycloalkyl includes cyclic hydrocarbon groups having from 3 to 10 carbons, such as cyclopropyl, cyclobutyl, cyclopentyl, cyclopentenyl, cyclohexyl, cyclohexenyl, cycloheptyl, cyclooctyl and the like.

Pharmaceutically acceptable addition salts encompass pharmaceutically acceptable acid addition salts and pharmaceutically acceptable base addition salts. The pharmaceutically acceptable acid addition salts as mentioned hereinabove are meant to comprise the therapeutically active non-toxic acid addition salt forms, which the compounds of formula (I) are able to form. The compounds of formula (I) which have basic properties can be converted in their pharmaceutically acceptable acid addition salts by treating said base form with an appropriate acid. Appropriate acids comprise, for example, inorganic acids such as hydrohalic acids, e.g. hydrochloric or hydrobromic acid; sulfuric; nitric; phosphoric and the like acids; or organic acids such as, for example, acetic, trifluoroacetic, propanoic, hydroxyacetic, lactic, pyruvic, oxalic, malonic, succinic (*i.e.* butanedioic acid), maleic, fumaric, malic, tartaric, citric, methanesulfonic, ethanesulfonic, benzenesulfonic, *p*-toluenesulfonic, cyclamic, salicylic, *p*-amino-salicylic, pamoic and the like acids.

The compounds of formula (I) which have acidic properties may be converted in their pharmaceutically acceptable base addition salts by treating said acid form with a suitable organic or inorganic base. Appropriate base salt forms comprise, for example, the ammonium salts, the alkali and earth alkaline metal salts, e.g. the lithium, sodium, potassium, magnesium, calcium salts and the like, salts with organic bases, e.g. the benzathine, *N*-methyl-D-glucamine, hydrabamine salts, and salts with amino acids such as, for example, arginine, lysine and the like.

The term "acid or base addition salts" also comprises the hydrates and the solvent addition forms, which the compounds of formula (I) are able to form. Examples of such forms are e.g. hydrates, alcoholates and the like.

The term "stereochemically isomeric forms of compounds of formula (I)", as used herein, defines all possible compounds made up of the same atoms bonded by the same sequence of bonds but having different three-dimensional structures, which are not interchangeable, which the compounds of formula (I) may possess. Unless otherwise mentioned or indicated, the chemical designation of a compound encompasses the mixture of all possible stereochemically isomeric forms, which said compound might possess. Said mixture may contain all diastereomers and/or enantiomers of the basic molecular structure of said compound. All stereochemically isomeric forms of the compounds of formula (I) both in pure form or in admixture with each other are intended to be embraced within the scope of the present invention.

The *N*-oxide forms of the compounds of formula (I) are meant to comprise those compounds of formula (I) wherein one or several nitrogen atoms are oxidized to the so-called *N*-oxide, particularly those *N*-oxides wherein one or more of the piperidine-, piperazine or pyridazinyl-nitrogens are *N*-oxidized.

Some of the compounds of formula (I) may also exist in their tautomeric forms. Such forms although not explicitly indicated in the above formula are intended to be included within the scope of the present invention.

Whenever used hereinafter, the term "compounds of formula (I)" is meant to include also the pharmaceutically acceptable addition salts and all stereoisomeric forms.

As used herein, the terms "histone deacetylase" and "HDAC" are intended to refer to any one of a family of enzymes that remove acetyl groups from the ε-amino groups of lysine residues at the N-terminus of a histone. Unless otherwise indicated by context, the term "histone" is meant to refer to any histone protein, including H1, H2A, H2B, H3, H4, and H5, from any species. Human HDAC proteins or gene products, include, but are not limited to, HDAC-1, HDAC-2, HDAC-3, HDAC-4, HDAC-5, HDAC-6, HDAC-7, HDAC-8, HDAC-9 and HDAC-10. The histone deacetylase can also be derived from a protozoal or fungal source.

A first group of interesting compounds consists of those compounds of formula (I) wherein one or more of the following restrictions apply:

a) t is 0 or 1;
b) each Q is

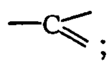

c) each X is nitrogen;
d) $R^1$ is -C(O)NH(OH);
e) $R^2$ is hydrogen, hydroxy, $C_{1-6}$alkyl, or aryl$C_{1-6}$alkyl;
f) -L- is a bivalent radical selected from -NHC(O)- or $-NHSO_2-$;
g)

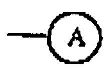

is a radical selected from (a-1) or (a-20);
h) each s is independently 0 or 1;
i) each $R^5$ is independently selected from hydrogen or phenyl.

A second group of interesting compounds consists of those compounds of formula (I) wherein one or more of the following restrictions apply:

a) t is 1;
b) each Q is

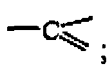

c) each X is nitrogen;
d) each Y is nitrogen;
e) each Z is -O- or $-CH_2-$;
f) $R^1$ is -C(O)NH(OH);
g) $R^2$ is hydrogen;
h) -L- is a bivalent radical selected from -NHC(O)- or $-NHSO_2-$;
i)

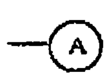

is a radical selected from (a-1) or (a-20);
j)each s is independently 0 or 1;
k) each $R^5$ is independently selected from hydrogen or phenyl.

A third group of interesting compounds consists of those compounds of formula (I) wherein $R^1$ is -C(O)NH(OH).
A fourth group of interesting compounds consists of those compounds of formula (I) wherein $R^1$ is -C(O)NH (OH) and $R^2$ is hydrogen.
A fifth group of interesting compounds consists of those compounds of formula (I) wherein one or more of the following restrictions apply:

a) t is 0;
b) $R^1$ is -C(O)NR$^3$R$^4$, -C(O)-$C_{1-6}$alkanediylSR$^7$, -NR$^8$C(O)N(OH)R$^7$, -NR$^8$C(O)$C_{1-6}$alkanediylSR$^7$ or -NR$^8$C(O)C=N(OH)R$^7$ wherein $R^3$ and $R^4$ are each independently selected from hydrogen, hydroxy, hydroxy$C_{1-6}$alkyl or amiano$C_{1-6}$alkyl;
c) $R^2$ is hydrogen, hydroxy, amino, hydroxy$C_{1-6}$alkyl, $C_{1-6}$alkyl, $C_{1-6}$alkyloxy, aryl$C_{1-6}$alkyl, aminocarbonyl, amino$C_{1-6}$alkyl, $C_{1-6}$alkylamino$C_{1-6}$alkyl or di($C_{1-6}$alkyl)amino$C_{1-6}$alkyl;
d) -L- is a bivalent radical selected from -NHC(O)- or -NHSO$_2$-;
e)

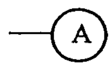

is a radical selected from (a-1), (a-3), (a-4), (a-5), (a-6), (a-7), (a-8), (a-9), (a-10), (a-11), (a-12), (a-13), (a-14), (a-15), (a-16), (a-17), (a-18), (a-19), (a-20), (a-21), (a-22), (a-23), (a-24), (a-25), (a-26), (a-28), (a-29), (a-30), (a-31), (a-32), (a-33), (a-34), (a-35), (a-36), (a-37), (a-38), (a-39), (a-40), (a-41), (a-42), (a-44), (a-45), (a-46), (a-47), (a-48) or (a-51);
f) each s is independently 0, 1, 2, 3 or 4;
g) $R^5$ is hydrogen; halo; hydroxy; amino; nitro; trihalo$C_{1-6}$alkyl; trihalo$C_{1-6}$alkyloxy; $C_{1-6}$alkyl; $C_{1-6}$alkyloxy; $C_{1-6}$alkylcarbonyl; $C_{1-6}$alkyloxycarbonyl; $C_{1-6}$alkylsulfonyl, hydroxy$C_{1-6}$alkyl; aryloxy; di($C_{1-6}$alkyl)amino; cyano; thiophenyl; furanyl; furanyl substituted with hydroxy$C_{1-6}$alkyl; benzofuranyl; imidazolyl; oxazolyl; oxazolyl substituted with aryl and $C_{1-6}$alkyl; $C_{1-6}$alkyltriazolyl; tetrazolyl; pyrrolidinyl; pyrrolyl; morpholinyl; $C_{1-6}$alkylmorpholinyl; piperazinyl; $C_{1-6}$alkylpiperazinyl; hydroxy$C_{1-6}$alkylpiperazinyl; $C_{1-6}$alkyloxypiperidinyl; pyrazoly; pyrazolyl substituted with one or two substituents selected from $C_{1-6}$alkyl or trihalo$C_{1-6}$alkyl; pyridinyl; pyridinyl substituted with $C_{1-6}$alkyloxy, aryloxy or aryl; pyrimidinyl; quinolinyl; indole; phenyl; or phenyl substituted with one or two substituents independently selected from halo, $C_{1-6}$alkyl, $C_{1-6}$alkyloxy or trifluoromethyl;
h) $R^6$ is hydrogen; halo; hydroxy; amino; nitro; trihalo$C_{1-6}$alkyl; trihalo$C_{1-6}$alkyloxy; $C_{1-6}$alkyl; $C_{1-6}$alkyloxy; $C_{1-6}$alkylcarbonyl; $C_{1-6}$alkyloxycarbonyl; $C_{1-6}$alkylsulfonyl; hydroxy$C_{1-6}$alkyl; aryloxy; di($C_{1-6}$alkyl)amino; cyano; pyridinyl; phenyl; or phenyl substituted with one or two substituents independently selected from halo, $C_{1-6}$alkyl, $C_{1-6}$alkyloxy or trifluoromethyl.

A sixth group of interesting compounds consists of those compounds of formula (I) wherein one or more of the following restrictions apply:

a) $R^3$ and $R^4$ are each independently selected from hydrogen, hydroxy, hydroxy$C_{1-6}$alkyl, amino$C_{1-6}$alkyl or aminoaryl;
b)

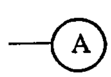

is a radical selected from (a-1), (a-2), (a-3), (a-4), (a-5), (a-6), (a-7), (a-8), (a-9), (a-10), (a-11), (a-12), (a-13), (a-14), (a-15), (a-16), (a-17), (a-18), (a-19), (a-20), (a-21), (a-22), (a-23), (a-24), (a-25), (a-26), (a-27), (a-28), (a-29), (a-30), (a-31), (a-32), (a-33), (a-34), (a-35), (a-36), (a-37), (a-38), (a-39), (a-40), (a-41), (a-42) (a-43) or (a-44);
c) each $R^5$ and $R^6$ are independently selected from hydrogen; halo; hydroxy; amino; nitro; trihalo$C_{1-6}$alkyl; trihalo$C_{1-6}$alkyloxy; $C_{1-6}$alkyl; $C_{1-6}$alkyloxy; $C_{1-6}$alkyloxy$C_{1-6}$alkyloxy; $C_{1-6}$alkylcarbonyl; $C_{1-6}$alkylsulfonyl; cyano $C_{1-6}$alkyl; hydroxy$C_{1-6}$alkyl; hydroxy$C_{1-6}$alkyloxy; hydroxy$C_{1-6}$alkylamino; amino$C_{1-6}$alkyloxy; di($C_{1-6}$alkyl)aminoc-

arbonyl; di(hydroxy$C_{1-6}$alkyl)amino; aryl$C_{1-6}$alkyl)amino; di($C_{1-6}$alkyl)amino$C_{1-6}$alkyloxy; di($C_{1-6}$alkyl)amino $C_{1-6}$alkylamino; arylsulfonyl; arylsulfonylamino; aryloxy; aryl$C_{2-6}$alkenediyl; di($C_{1-6}$alkyl)amino; di($C_{1-6}$alkyl)amino$C_{1-6}$alkyl; di($C_{1-6}$alkyl)amino$C_{1-6}$alkyl($C_{1-6}$alkyl)amino$C_{1-6}$alkyl; cyano; thiophenyl; thiophenyl substituted with di($C_{1-6}$alkyl)amino$C_{1-6}$alkyl($C_{1-6}$alkyl)amino$C_{1-6}$alkyl, di($C_{1-6}$alkyl)amino$C_{1-6}$alkyl, $C_{1-6}$alkylpiperazinyl$C_{1-6}$alkyl or di(hydroxy$C_{1-6}$alkyl)amino$C_{1-6}$alkyl; furanyl; imidazolyl; $C_{1-6}$alkyltriazolyl; tetrazolyl; pyrrolidinyl; piperidinyl $C_{1-6}$alkyloxy; morpholinyl; $C_{1-6}$alkylmorpholinyl; morpholinyl$C_{1-6}$alkyloxy; morpholinyl$C_{1-6}$alkyl; $C_{1-6}$alkylpiperazinyl; $C_{1-6}$alkylpiperazinyl$C_{1-6}$alkyloxy; $C_{1-6}$alkylpiperazinyl$C_{1-6}$alkyl; $C_{1-6}$alkylpiperazinylsulfonyl; aminosulfonylpiperazinyl$C_{1-6}$alkyloxy; aminosulfonylpiperazinyl; aminosulfonylpiperazinyl$C_{1-6}$alkyl; di($C_{1-6}$alkyl)aminosulfonylpiperazinyl; di($C_{1-6}$alkyl)aminosulfonylpiperazinyl$C_{1-6}$alkyl; hydroxy$C_{1-6}$alkylpiperazinyl; hydroxy$C_{1-6}$alkylpiperazinyl$C_{1-6}$alkyl; $C_{1-6}$alkyloxypiperidinyl; $C_{1-6}$alkyloxypiperidinyl$C_{1-6}$alkyl; hydroxy$C_{1-6}$alkyloxy$C_{1-6}$alkylpiperazinyl; hydroxy$C_{1-6}$alkyloxy$C_{1-6}$alkylpiperazinyl$C_{1-6}$alkyl; (hydroxy$C_{1-6}$alkyl)($C_{1-6}$alkyl)amino; (hydroxy$C_{1-6}$alkyl)($C_{1-6}$alkyl)amino$C_{1-6}$alkyl; pyrrolidinyl$C_{1-6}$alkyloxy; pyrazolyl; thiopyrazolyl; pyrazolyl substituted with two substituents selected from $C_{1-6}$alkyl or trihalo$C_{1-6}$alkyl; pyridinyl; pyridinyl substituted with $C_{1-6}$alkyloxy or aryl; pyrimidinyl; quinolinyl; indole; phenyl; phenyl substituted with one, two or three substituents independently selected from halo, amino, $C_{1-6}$alkyl, $C_{1-6}$alkyloxy, hydroxy$C_{1-4}$alkyl, trifluoromethyl, trifluoromethyloxy, hydroxy$C_{1-4}$alkyloxy, $C_{1-4}$alkyloxy$C_{1-4}$alkyloxy, amino$C_{1-4}$alkyloxy, di($C_{1-4}$alkyl)amino$C_{1-4}$alkyloxy, di($C_{1-4}$alkyl)amino, di($C_{1-4}$alkyl)amino$C_{1-4}$alkyl, di($C_{1-4}$alkyl)amino$C_{1-4}$alkyl($C_{1-4}$alkyl)amino$C_{1-4}$alkyl, piperidinyl$C_{1-4}$alkyloxy, pyrrolidinyl$C_{1-4}$alkyloxy, aminosulfonylpiperazinyl, aminosulfonylpiperazinyl$C_{1-4}$alkyl, di($C_{1-4}$alkyl)aminosulfonylpiperazinyl, di($C_{1-4}$alkyl)aminosulfonylpiperazinyl$C_{1-4}$alkyl, hydroxy$C_{1-4}$alkylpiperazinyl, hydroxy$C_{1-4}$alkylpiperazinyl$C_{1-4}$alkyl, $C_{1-4}$alkyloxypiperidinyl, $C_{1-4}$alkyloxypiperidinyl$C_{1-4}$alkyl, hydroxy$C_{1-4}$alkyloxy$C_{1-4}$alkylpiperazinyl, hydroxy$C_{1-4}$alkyloxy$C_{1-4}$alkylpiperazinyl $C_{1-4}$alkyl, (hydroxy$C_{1-4}$alkyl)($C_{1-4}$alkyl)amino, (hydroxy$C_{1-4}$alkyl)($C_{1-4}$alkyl)amino$C_{1-4}$alkyl, pyrrolidinyl$C_{1-4}$alkyloxy, morpholinyl$C_{1-4}$alkyloxy, morpholinyl$C_{1-4}$alkyl, $C_{1-4}$alkylpiperazinyl, $C_{1-4}$alkylpiperazinyl$C_{1-4}$alkyloxy, $C_{1-4}$alkylpiperazinyl$C_{1-4}$alkyl, hydroxy$C_{1-4}$alkylamino, di(hydroxy$C_{1-4}$alkyl)amino, di($C_{1-4}$alkyl)amino$C_{1-4}$alkylamino, aminothiadiazolyl, aminosulfonylpiperazinyl$C_{1-4}$alkyloxy, or thiophenyl$C_{1-4}$alkylamino.

A group of preferred compounds consists of those compounds of formula (I) wherein $R^3$ and $R^4$ are each independently selected from hydrogen, hydroxy,
hydroxy$C_{1-6}$alkyl, amino$C_{1-6}$alkyl or aminoaryl;

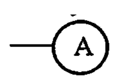

is a radical selected from (a-1), (a-2), (a-3), (a-4), (a-5), (a-6), (a-7), (a-8), (a-9), (a-10), (a-11), (a-12), (a-13), (a-14), (a-15), (a-16), (a-17), (a-18), (a-19), (a-20), (a-21), (a-22), (a-23), (a-24), (a-25), (a-26), (a-27), (a-28), (a-29), (a-30), (a-31), (a-32), (a-33), (a-34), (a-35), (a-36), (a-37), (a-38), (a-39), (a-40), (a-41), (a-42) (a-43) or (a-44);
each $R^5$ and $R^6$ are independently selected from hydrogen; halo; hydroxy; amino; nitro; trihalo$C_{1-6}$alkyl; trihalo$C_{1-6}$alkyloxy; $C_{1-6}$alkyl; $C_{1-6}$alkyloxy; $C_{1-6}$alkyloxy$C_{1-6}$alkyloxy; $C_{1-6}$alkylcarbonyl; $C_{1-6}$alkylsulfonyl; cyano$C_{1-6}$alkyl; hydroxy$C_{1-6}$alkyl; hydroxy$C_{1-6}$alkyloxy; hydroxy$C_{1-6}$alkylamino; amino$C_{1-6}$alkyloxy; di($C_{1-6}$alkyl)aminocarbonyl; di(hydroxy$C_{1-6}$alkyl)amino; aryl$C_{1-6}$alkyl)amino; di($C_{1-6}$alkyl)amino$C_{1-6}$alkyloxy; di($C_{1-6}$alkyl)amino$C_{1-6}$alkylamino; arylsulfonyl; arylsulfonylamino; aryloxy; aryl$C_{2-6}$alkenediyl; di($C_{1-6}$alkyl)amino; di($C_{1-6}$alkyl)amino$C_{1-6}$alkyl; di($C_{1-6}$alkyl)amino$C_{1-6}$alkyl($C_{1-6}$alkyl)amino$C_{1-6}$alkyl; cyano; thiophenyl; thiophenyl substituted with di($C_{1-6}$alkyl)amino$C_{1-6}$alkyl($C_{1-6}$alkyl)amino$C_{1-6}$alkyl, di($C_{1-6}$alkyl)amino$C_{1-6}$alkyl, $C_{1-6}$alkylpiperazinyl$C_{1-6}$alkyl or di(hydroxy$C_{1-6}$alkyl)amino$C_{1-6}$alkyl; furanyl; imidazolyl; $C_{1-6}$alkyltriazolyl; tetrazolyl; pyrrolidinyl; piperidinyl$C_{1-6}$alkyloxy; morpholinyl; $C_{1-6}$alkylmorpholinyl; morpholinyl$C_{1-6}$alkyloxy; morpholinyl$C_{1-6}$alkyl; $C_{1-6}$alkylpiperazinyl; $C_{1-6}$alkylpiperazinyl$C_{1-6}$alkyloxy; $C_{1-6}$alkylpiperazinyl$C_{1-6}$alkyl; $C_{1-6}$alkylpiperazinylsulfonyl; aminosulfonylpiperazinyl$C_{1-6}$alkyloxy; aminosulfonylpiperazinyl; aminosulfonylpiperazinyl$C_{1-6}$alkyl; di($C_{1-6}$alkyl)aminosulfonylpiperazinyl; di($C_{1-6}$alkyl)aminosulfonylpiperazinyl$C_{1-6}$alkyl; hydroxy$C_{1-6}$alkylpiperazinyl; hydroxy$C_{1-6}$alkylpiperazinyl$C_{1-6}$alkyl; $C_{1-6}$alkyloxypiperidinyl; $C_{1-6}$alkyloxypiperidinyl$C_{1-6}$alkyl; hydroxy$C_{1-6}$alkyloxy$C_{1-6}$alkylpiperazinyl; hydroxy$C_{1-6}$alkyloxy$C_{1-6}$alkylpiperazinyl$C_{1-6}$alkyl; (hydroxy$C_{1-6}$alkyl)($C_{1-6}$alkyl)amino; (hydroxy$C_{1-6}$alkyl)($C_{1-6}$alkyl)amino$C_{1-6}$alkyl; pyrrolidinyl $C_{1-6}$alkyloxy; pyrazolyl; thiopyrazolyl; pyrazolyl substituted with two substituents selected from $C_{1-6}$alkyl or trihalo$C_{1-6}$alkyl; pyridinyl; pyridinyl substituted with $C_{1-6}$alkyloxy or aryl; pyrimidinyl; quinolinyl; indole; phenyl; phenyl substituted with one, two or three substituents independently selected from halo, amino, $C_{1-6}$alkyl, $C_{1-6}$alkyloxy, hydroxy$C_{1-4}$alkyl, trifluoromethyl, trifluoromethyloxy, hydroxy$C_{1-4}$alkyloxy, $C_{1-4}$alkyloxy$C_{1-4}$alkyloxy, amino$C_{1-4}$alkyloxy, di($C_{1-4}$alkyl)amino$C_{1-4}$alkyloxy, di($C_{1-4}$alkyl)amino, di($C_{1-4}$alkyl)amino$C_{1-4}$alkyl, di($C_{1-4}$alkyl)amino$C_{1-4}$alkyl($C_{1-4}$alkyl)amino$C_{1-4}$alkyl, piperidinyl$C_{1-4}$alkyloxy, pyrrolidinyl$C_{1-4}$alkyloxy, aminosulfonylpiperazinyl, aminosulfonylpiperazinyl $C_{1-4}$alkyl, di($C_{1-4}$alkyl)aminosulfonylpiperazinyl, di($C_{1-4}$alkyl)aminosulfonylpiperazinyl$C_{1-4}$alkyl, hydroxy$C_{1-4}$alkylpiperazinyl, hydroxy$C_{1-4}$alkylpiperazinyl$C_{1-4}$alkyl, $C_{1-4}$alkyloxypiperidinyl, $C_{1-4}$alkyloxypiperidinyl$C_{1-4}$alkyl, hydroxy

$C_{1-4}$alkyloxy$C_{1-4}$alkylpiperazinyl, hydroxy$C_{1-4}$alkyloxy$C_{1-4}$alkylpiperazinyl$C_{1-4}$alkyl, (hydroxy$C_{1-4}$alkyl)($C_{1-4}$alkyl)amino, (hydroxy$C_{1-4}$alkyl)($C_{1-4}$alkyl)amino$C_{1-4}$alkyl, pyrrolidinyl$C_{1-4}$alkyloxy, morpholinyl$C_{1-4}$alkyloxy, morpholinyl$C_{1-4}$alkyl, $C_{1-4}$alkylpiperazinyl, $C_{1-4}$alkylpiperazinyl$C_{1-4}$alkyloxy, $C_{1-4}$alkylpiperazinyl$C_{1-4}$alkyl, hydroxy$C_{1-4}$alkylamino, di (hydroxy$C_{1-4}$alkyl)amino, di($C_{1-4}$alkyl)amino$C_{1-4}$alkylamino, aminothiadiazolyl, aminosulfonylpiperazinyl$C_{1-4}$alkyloxy, or thiophenyl$C_{1-4}$alkylamino.

A further group of preferred compounds consists of those compounds of formula (I) wherein t is 0;

$R^1$ is -C(O)N$R^3R^4$, -C(C)-$C_{1-6}$alkanediylS$R^7$, -N$R^8$C(O)N(OH)$R^7$, -N$R^8$C(O)$C_{1-6}$alkanediylS$R^7$ or -N$R^8$C(O)C=N(OH)$R^7$ wherein $R^3$ and $R^4$ are each independently selected from hydrogen, hydroxy, hydroxy$C_{1-6}$alkyl or amino$C_{1-6}$alkyl; $R^2$ is hydrogen, hydroxy, amino, hydxaxy$C_{1-6}$alkyl, $C_{1-6}$alkyl, $C_{1-6}$alkyloxy, aryl$C_{1-6}$alkyl, aminocarbonyl, amino$C_{1-6}$alkyl, $C_{1-6}$alkylamino$C_{1-6}$alkyl or di($C_{1-6}$alkyl) amino$C_{1-6}$alkyl;

-L- is a bivalent radical selected from -NHC(O)- or -NH$SO_2$-;

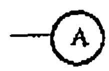

is a radical selected from (a-1), (a-3), (a-4), (a-5), (a-6), (a-7), (a-8), (a-9), (a-10), (a-11), (a-12), (a-13), (a-14), (a-15), (a-16), (a-17), (a-18), (a-19), (a-20), (a-21), (a-22), (a-23), (a-24), (a-25), (a-26), (a-28), (a-29), (a-30), (a-31), (a-32), (a-33), (a-34), (a-35), (a-36), (a-37), (a-38), (a-39), (a-40), (a-41), (a-42), (a-44), (a-45), (a-46), (a-47), (a-48) or (a-51); each s is independently 0, 1, 2, 3 or 4;

$R^5$ is hydrogen; halo; hydroxy; amino; nitro; trihalo$C_{1-6}$alkyl; trihalo$C_{1-6}$alkyloxy; $C_{1-6}$alkyl; $C_{1-6}$alkyloxy; $C_{1-6}$alkylcarbonyl; $C_{1-6}$alkyloxycarbonyl; $C_{1-6}$alkylsulfonyl; hydroxy$C_{1-6}$alkyl; aryloxy; di($C_{1-6}$alkyl)amino; cyano; thiophenyl; furanyl; furanyl substituted with hydroxy$C_{1-6}$alkyl; benzofuranyl; imidazolyl; oxazolyl; oxazolyl substituted with aryl and $C_{1-6}$alkyl; $C_{1-6}$alkyltriazolyl; tetrazolyl; pyrrolidinyl; pyrrolyl; morpholinyl; $C_{1-6}$alkylmorpholinyl; piperazinyl; $C_{1-6}$alkylpiperazinyl; hydroxy$C_{1-6}$alkylpiperazinyl; $C_{1-6}$alkyloxypiperidinyl; pyrazoly; pyrazolyl substituted with one or two substituents selected from $C_{1-6}$alkyl or trihalo$C_{1-6}$alkyl; pyridinyl; pyridinyl substituted with $C_{1-6}$alkyloxy or aryl; pyrimidinyl; quinolinyl; indole; phenyl; or phenyl substituted with one or two substituents independently selected from halo, $C_{1-6}$alkyl, $C_{1-6}$alkyloxy or trifluoromethyl;

and $R^6$ is hydrogen; halo; hydroxy; amino; nitro; trihalo$C_{1-6}$alkyl; trihalo$C_{1-6}$alkyloxy; $C_{1-6}$alkyl; $C_{1-6}$alkyloxy; $C_{1-6}$alkylcarbonyl; $C_{1-6}$alkyloxycarbonyl; $C_{1-6}$alkylsulfonyl; hydroxy$C_{1-6}$alkyl; aryloxy; di($C_{1-6}$alkyl)amino; cyano; pyridinyl; phenyl; or phenyl substituted with one or two substituents independently selected from halo, $C_{1-6}$alkyl, $C_{1-6}$alkyloxy or trifluoromethyl.

Another group of preferred compounds consists of those compounds of formula (I) wherein t is 0 or 1; each Q is

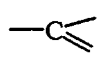 ;

each X is nitrogen; $R^1$ is -C(O)NH(OH); $R^2$ is hydrogen, hydroxy, $C_{1-6}$alkyl or aryl$C_{1-6}$alkyl; -L- is a bivalent radical selected from -NHC(O)- or -NH$SO_2$-;

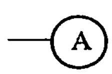

is a radical selected from (a-1) or (a-20); each s is independently 0 or 1; and each $R^5$ is independently selected from hydrogen or phenyl.

A group of more preferred compounds consists of those compounds of formula (I) wherein t is 1; each Q is

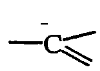 ;

each X is nitrogen; each Y is nitrogen; each Z is - O- or $-CH_2-$; $R^1$ is -C(O)NH(OH); $R^2$ is hydrogen; -L- is a bivalent radical selected from -NHC(O)- or $-NHSO_2-$;

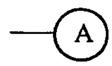

is a radical selected from (a-1) or (a-20); each s is independently 0 or 1; and each $R^5$ is independently selected from hydrogen or phenyl.

Most preferred compounds are compounds No 4, No 10, No 8, No 6, No 1, No 12 and No 14 .

| | |
|---|---|
| 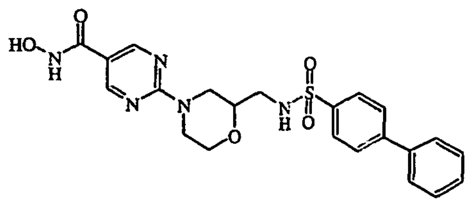 | 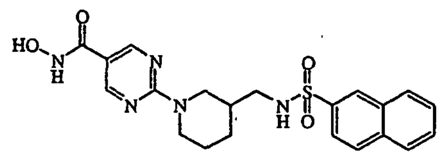 |
| Co. No. 4 | Co. No. 10 |
| 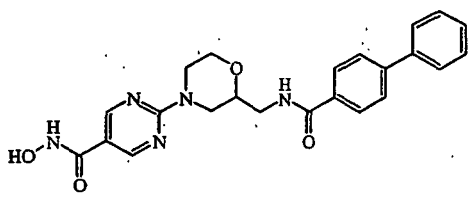 | 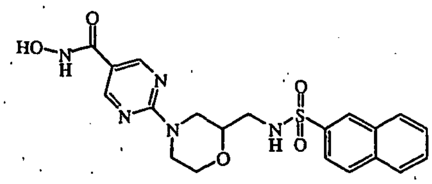 |
| Co. No. 8 | Co. No. 6 |
| 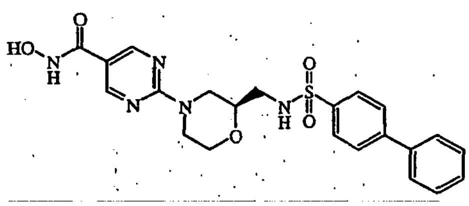 | 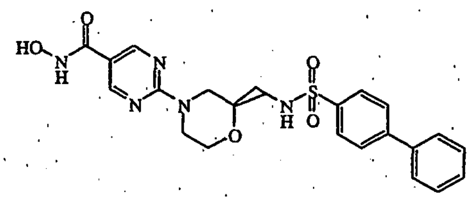 |
| (B); Co. No.1 | (A); Co. No. 12 |
| 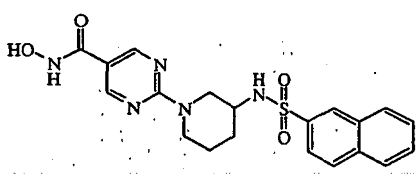 | |
| Co. No: 14 | |

The compounds of formula (I) and their pharmaceutically acceptable salts and N-oxides and stereochemically isomeric forms thereof may be prepared in a conventional manner. A general synthesis route is encompassed as example:

a) Hydroxamic acids of formula (I) wherein $R^1$ is -C(O)NH(OH), said compounds being referred to as compounds of formula (I-a), may be prepared by reacting an intermediate of formula (II) with an appropriate acid, such as for example, trifluoro acetic acid. Said reaction is performed in an appropriate solvent, such as, for example, methanol.

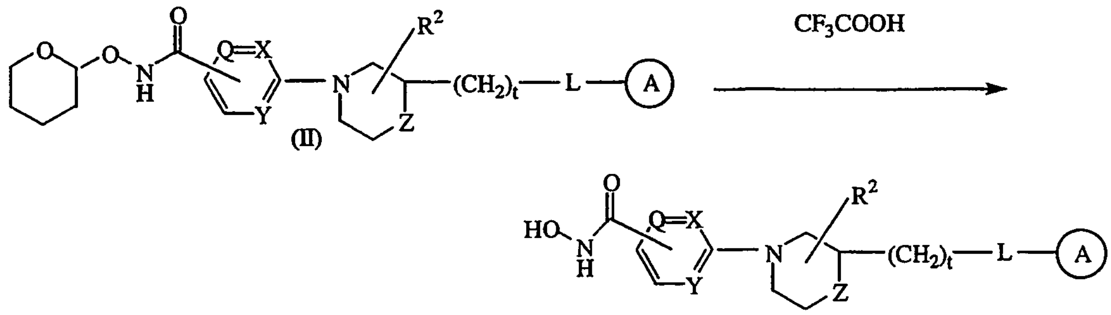

(II)

(I-a)

b) intermediates of formula (II) may be prepared by reacting an intermediate of formula (III) with an intermediate of formula (IV) in the presence of appropriate reagents such as *N'*-(ethylcarbonimidoyl)-*N*,*N*-dimethyl-1,3-propanediamine, monohydrochloride (EDC) and 1-hydroxy-1*H*-benzotriazole (HOBT). The reaction may be performed in a suitable solvent such as a mixture of DCM and THF.

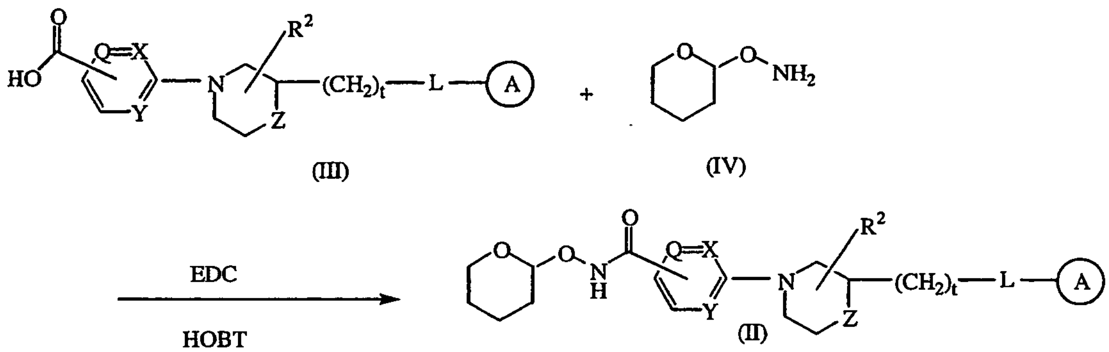

(III) + (IV)

(II)

c) intermediates of formula (III) may be prepared by reacting an intermediate of formula (V) with an appropriate base such as NaOH in the presence of a suitable solvent such as ethanol.

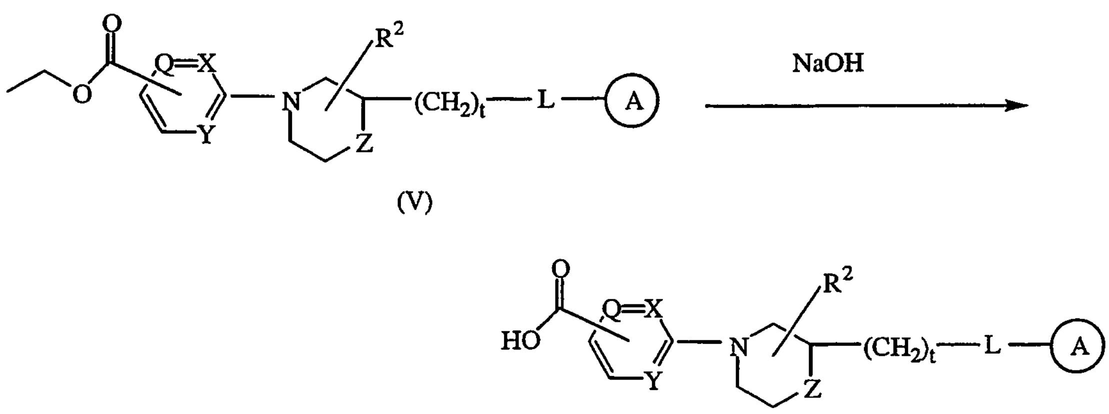

(V)

(III)

The compounds of formula (I) can also conveniently be prepared using solid phase synthesis techniques. In general, solid phase synthesis involves reacting an intermediate in a synthesis with a polymer support. This polymer-supported intermediate can then be carried on through a number of synthesis steps. After each step, filtering the resin and washing it numerous times with various solvents remove impurities. At each step the resin can be split up to react with various intermediates in the next step thus allowing for the synthesis of a large number of compounds. After the

last step in the procedure the resin is treated with a reagent or process to cleave the resin from the sample. More detailed explanation of the techniques used in solid phase chemistry is described in for example "The Combinatorial Index" (B.Bunin, Academic Press) and Novabiochem's 1999 Catalogue & Peptide Synthesis Handbook (Novabiochem AG, Switzerland) both incorporated herein by reference.

The compounds of formula (I) and some of the intermediates have at least one stereogenic centre in their structure. This stereogenic centre may be present in an R or an S configuration.

The compounds of formula (I) as prepared in the hereinabove described processes are generally racemic mixtures of enantiomers, which can be separated from one another following art-known resolution procedures. The racemic compounds of formula (I) may be converted into the corresponding diastereomeric salt forms by reaction with a suitable chiral acid. Said diastereomeric salt forms are subsequently separated, for example, by selective or fractional crystallization and the enantiomers are liberated there from by alkali. An alternative manner of separating the enantiomeric forms of the compounds of formula (I) involves liquid chromatography using a chiral stationary phase. Said pure stereochemically isomeric forms may also be derived from the corresponding pure stereochemically isomeric forms of the appropriate starting materials, provided that the reaction occurs stereospecifically. Preferably if a specific stereoisomer is desired, said compound would be synthesized by stereospecific methods of preparation. These methods will advantageously employ enantiomerically pure starting materials.

The compounds of formula (I), the pharmaceutically acceptable acid addition salts and stereoisomeric forms thereof have valuable pharmacological properties in that they have a histone deacetylase (HDAC) inhibitory effect.

This invention provides a method for inhibiting the abnormal growth of cells, including transformed cells, by administering an effective amount of a compound of the invention. Abnormal growth of cells refers to cell growth independent of normal regulatory mechanisms (e.g. loss of contact inhibition). This includes the inhibition of tumour growth both directly by causing growth arrest, terminal differentiation and/or apoptosis of cancer cells, and indirectly, by inhibiting neovascularization of tumours.

This invention also provides a method for inhibiting tumour growth by administering an effective amount of a compound of the present invention, to a subject, e.g. a mammal (and more particularly a human) in need of such treatment. In particular, this invention provides a method for inhibiting the growth of tumours by the administration of an effective amount of the compounds of the present invention. Examples of tumours which may be inhibited, but are not limited to, lung cancer (e.g. adenocarcinoma and including non-small cell lung cancer), pancreatic cancers (e.g. pancreatic carcinoma such as, for example exocrine pancreatic carcinoma), colon cancers (e.g. colorectal carcinomas, such as, for example, colon adenocarcinoma and colon adenoma), prostate cancer including the advanced disease, hematopoietic tumours of lymphoid lineage (e.g. acute lymphocytic leukemia, B-cell lymphoma, Burkitt's lymphoma), myeloid leukemias (for example, acute myelogenous leukemia (AML)), thyroid follicular cancer, myelodysplastic syndrome (MDS), tumours of mesenchymal origin (e.g. fibrosarcomas and rhabdomyosarcomas), melanomas, teratocarcinomas, neuroblastomas, gliomas, benign tumour of the skin (e.g. keratoacanthomas), breast carcinoma (e.g. advanced breast cancer), kidney carcinoma, ovary carcinoma, bladder carcinoma and epidermal carcinoma.

The compound according to the invention may be used for other therapeutic purposes, for example:

a) the sensitisation of tumours to radiotherapy by administering the compound according to the invention before, during or after irradiation of the tumour for treating cancer;
b) treating arthropathies and osteopathological conditions such as rheumatoid arthritis, osteoarthritis, juvenile arthritis, gout, polyarthritis, psoriatic arthritis, ankylosing spondylitis and systemic lupus erythematosus;
c) inhibiting smooth muscle cell proliferation including vascular proliferative disorders, atherosclerosis and restenosis;
d) treating inflammatory conditions and dermal conditions such as ulcerative colitis, Crohn's disease, allergic rhinitis, graft vs. host disease, conjunctivitis, asthma, ARDS, Behcets disease, transplant rejection, uticaria, allergic dermatitis, alopecia areata, scleroderma, exanthema, eczema, dermatomyositis, acne, diabetes, systemic lupus erythematosis, Kawasaki's disease, multiple sclerosis, emphysema, cystic fibrosis and chronic bronchitis;
e) treating endometriosis, uterine fibroids, dysfunctional uterine bleeding and endometrial hyperplasia;
f) treating ocular vascularisation including vasculopathy affecting retinal and choroidal vessels;
g) treating a cardiac dysfunction;
h) inhibiting immunosuppressive conditions such as the treatment of HIV infections;
i) treating renal dysfunction;
j) suppressing endocrine disorders;
k) inhibiting dysfunction of gluconeogenesis;
l) treating a neuropathology for example Parkinson's disease or a neuropathology that results in a cognitive disorder, for example, Alzheimer's disease or polyglutamine related neuronal diseases;
m) inhibiting a neuromuscular pathology, for example, amylotrophic lateral sclerosis;
n) treating spinal muscular atrophy;
o) treating other pathologic conditions amenable to treatment by potentiating expression of a gene;

p) enhancing gene therapy.

Hence, the present invention discloses the compounds of formula (I) for use as a medicine as well as the use of these compounds of formula (I) for the manufacture of a medicament for treating one or more of the above mentioned conditions.

The compounds of formula (I), the pharmaceutically acceptable acid addition salts and stereoisomeric forms thereof can have valuable diagnostic properties in that they can be used for detecting or identifying a HDAC in a biological sample comprising detecting or measuring the formation of a complex between a labelled compound and a HDAC.

The detecting or identifying methods can use compounds that are labelled with labelling agents such as radioisotopes, enzymes, fluorescent substances, luminous substances, etc. Examples of the radioisotopes include $^{125}I$, $^{131}I$, $^{3}H$ and $^{14}C$. Enzymes are usually made detectable by conjugation of an appropriate substrate which, in turn catalyses a detectable reaction. Examples thereof include, for example, beta-galactosidase, beta-glucosidase, alkaline phosphatase, peroxidase and malate dehydrogenase, preferably horseradish peroxidase. The luminous substances include, for example, luminol, luminol derivatives, luciferin, aequorin and luciferase.

Biological samples can be defined as body tissue or body fluids. Examples of body fluids are cerebrospinal fluid, blood, plasma, serum, urine, sputum, saliva and the like.

In view of their useful pharmacological properties, the subject compounds may be formulated into various pharmaceutical forms for administration purposes.

To prepare the pharmaceutical compositions of this invention, an effective amount of a particular compound, in base or acid addition salt form, as the active ingredient is combined in intimate admixture with a pharmaceutically acceptable carrier, which carrier may take a wide variety of forms depending on the form of preparation desired for administration. These pharmaceutical compositions are desirably in unitary dosage form suitable, preferably, for administration orally, rectally, percutaneously, or by parenteral injection. For example, in preparing the compositions in oral dosage form, any of the usual pharmaceutical media may be employed, such as, for example, water, glycols, oils, alcohols and the like in the case of oral liquid preparations such as suspensions, syrups, elixirs and solutions; or solid carriers such as starches, sugars, kaolin, lubricants, binders, disintegrating agents and the like in the case of powders, pills, capsules and tablets.

Because of their ease in administration, tablets and capsules represent the most advantageous oral dosage unit form, in which case solid pharmaceutical carriers are obviously employed. For parenteral compositions, the carrier will usually comprise sterile water, at least in large part, though other ingredients, to aid solubility for example, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. In the compositions suitable for percutaneous administration, the carrier optionally comprises a penetration enhancing agent and/or a suitable wetting agent, optionally combined with suitable additives of any nature in minor proportions, which additives do not cause a significant deleterious effect to the skin. Said additives may facilitate the administration to the skin and/or may be helpful for preparing the desired compositions. These compositions may be administered in various ways, e.g., as a transdermal patch, as a spot-on or as an ointment.

It is especially advantageous to formulate the aforementioned pharmaceutical compositions in dosage unit form for ease of administration and uniformity of dosage. Dosage unit form as used in the specification and claims herein refers to physically discrete units suitable as unitary dosages, each unit containing a predetermined quantity of active ingredient, calculated to produce the desired therapeutic effect, in association with the required pharmaceutical carrier. Examples of such dosage unit forms are tablets (including scored or coated tablets), capsules, pills, powder packets, wafers, injectable solutions or suspensions, teaspoonfuls, tablespoonfuls and the like, and segregated multiples thereof.

Those skilled in the art could easily determine the effective amount from the test results presented hereinafter. In general it is contemplated that a therapeutically effective amount would be from 0.005 mg/kg to 100 mg/kg body weight, and in particular from 0.005 mg/kg to 10 mg/kg body weight. It may be appropriate to administer the required dose as two, three, four or more sub-doses at appropriate intervals throughout the day. Said sub-doses may be formulated as unit dosage forms, for example, containing 0.5 to 500 mg, and in particular 10 mg to 500 mg of active ingredient per unit dosage form.

As another aspect of the present invention a combination of a HDAC-inhibitor with another anticancer agent is envisaged, especially for use as a medicine, more specifically in the treatment of cancer or related diseases.

For the treatment of the above conditions, the compounds of the invention may be advantageously employed in combination with one or more other medicinal agents, more particularly, with other anti-cancer agents. Examples of anti-cancer agents are:

- platinum coordination compounds for example cisplatin, carboplatin or oxalyplatin;
- taxane compounds for example paclitaxel or docetaxel;

- topoisomerase I inhibitors such as camptothecin compounds for example irinotecan or topotecan;
- topoisomerase II inhibitors such as anti-tumour podophyllotoxin derivatives for example etoposide or teniposide;
- anti-tumour vinca alkaloids for example vinblastine, vincristine or vinorelbine;
- anti-tumour nucleoside derivatives for example 5-fluorouracil, gemcitabine or capecitabine;
- alkylating agents such as nitrogen mustard or nitrosourea for example cyclophosphamide, chlorambucil, carmustine or lomustine;
- anti-tumour anthracycline derivatives for example daunorubicin, doxorubicin, idarubicin or mitoxantrone;
- HER2 antibodies for example trastuzumab;
- estrogen receptor antagonists or selective estrogen receptor modulators for example tamoxifen, toremifene, droloxifene, faslodex or raloxifene;
- aromatase inhibitors such as exemestane, anastrozole, letrazole and vorozole;
- differentiating agents such as retinoids, vitamin D and retinoic acid metabolism blocking agents (RAMBA) for example accutane;
- DNA methyl transferase inhibitors for example azacytidine;
- kinase inhibitors for example flavoperidol, imatinib mesylate or gefitinib;
- farnesyltransferase inhibitors; or
- other HDAC inhibitors.

The term "platinum coordination compound" is used herein to denote any tumor cell growth inhibiting platinum coordination compound which provides platinum in the form of an ion.

The term "taxane compounds" indicates a class of compounds having the taxane ring system and related to or derived from extracts from certain species of yew (Taxus) trees.

The term "topisomerase inhibitors" is used to indicate enzymes that are capable of altering DNA topology in eukaryotic cells. They are critical for important cellular functions and cell proliferation. There are two classes of topoisomerases in eukaryotic cells, namely type I and type II. Topoisomerase I is a monomeric enzyme of approximately 100,000 molecular weight. The enzyme binds to DNA and introduces a transient single-strand break, unwinds the double helix (or allows it to unwind) and subsequently reseals the break before dissociating from the DNA strand. Topisomerase II has a similar mechanism of action which involves the induction of DNA strand breaks or the formation of free radicals.

The term "camptothecin compounds" is used to indicate compounds that are related to or derived from the parent camptothecin compound which is a water-insoluble alkaloid derived from the Chinese tree Camptothecin acuminata and the Indian tree Nothapodytes foetida.

The term "podophyllotoxin compounds" is used to indicate compounds that are related to or derived from the parent podophyllotoxin, which is extracted from the mandrake plant.

The term "anti-tumor vinca alkaloids" is used to indicate compounds that are related to or derived from extracts of the periwinkle plant (Vinca rosea).

The term "alkylating agents" encompass a diverse group of chemicals that have the common feature that they have the capacity to contribute, under physiological conditions, alkyl groups to biologically vital macromolecules such as DNA. With most of the more important agents such as the nitrogen mustards and the nitrosoureas, the active alkylating moieties are generated *in vivo* after complex degradative reactions, some of which are enzymatic. The most important pharmacological actions of the alkylating agents are those that disturb the fundamental mechanisms concerned with cell proliferation in particular DNA synthesis and cell division. The capacity of alkylating agents to interfere with DNA function and integrity in rapidly proliferating tissues provides the basis for their therapeutic applications and for many of their toxic properties.

The term "anti-tumour anthracycline derivatives" comprise antibiotics obtained from the fungus *Strep. peuticus var. caesius* and their derivatives, characterised by having a tetracycline ring structure with an unusual sugar, daunosamine, attached by a glycosidic linkage.

Amplification of the human epidermal growth factor receptor 2 protein (HER 2) in primary breast carcinomas has been shown to correlate with a poor clinical prognosis for certain patients. Trastuzumab is a highly purified recombinant DNA-derived humanized monoclonal IgG1 kappa antibody that binds with high affiniity and specificity to the extracellular domain of the HER2 receptor.

Many breast cancers have estrogen receptors and growth of these tumors can be stimulated by estrogen. The terms "estrogen receptor antagonists" and "selective estrogen receptor modulators" are used to indicate competitive inhibitors of estradiol binding to the estrogen receptor (ER). Selective estrogen receptor modulators, when bound to the ER, induces a change in the three-dimensional shape of the receptor, inhibiting its binding to the estrogen responsive element (ERE) on DNA.

In postmenopausal women, the principal source of circulating estrogen is from conversion of adrenal and ovarian androgens (androstenedione and testosterone) to estrogens (estrone and estradiol) by the aromatase enzyme in peripheral tissues. Estrogen deprivation through aromatase inhibition or inactivation is an effective and selective

treatment for some postmenopausal patients with hormone-dependent breast cancer.

The term "antiestrogen agent" is used herein to include not only estrogen receptor antagonists and selective estrogen receptor modulators but also aromatase inhibitors as discussed above.

The term "differentiating agents" encompass compounds that can, in various ways, inhibit cell proliferation and induce differentiation. Vitamin D and retinoids are known to play a major role in regulating growth and differentiation of a wide variety of normal and malignant cell types. Retinoic acid metabolism blocking agents (RAMBA's) increase the levels of endogenous retinoic acids by inhibiting the cytochrome P450-mediated catabolism of retinoic acids.

DNA methylation changes are among the most common abnormalities in human neoplasia. Hypermethylation within the promotors of selected genes is usually associated with inactivation of the involved genes. The term "DNA methyl transferase inhibitors" is used to indicate compounds that act through pharmacological inhibition of DNA methyl transferase and reactivation of tumour suppressor gene expression.

The term "kinase inhibitors" comprises potent inhibitors of kinases that are involved in cell cycle progression and programmed cell death (apoptosis)

The term "farnesyltransferase inhibitors" is used to indicate compounds that were designed to prevent farnesylation of Ras and other intracellular proteins. They have been shown to have effect on malignant cell proliferation and survival.

The term "other HDAC inhibitors" comprises but is not limited to:

- short-chain fatty acids for example butyrate, 4-phenylbutyrate or valproic acid;
- hydroxamic acids for example suberoylanilide hydroxamic acid (SAHA), biaryl hydroxamate A-161906, bicyclic aryl-*N*-hydroxycarboxamides, pyroxamide, CG-1521, PXD-101, sulfonamide hydroxamic acid, LAQ-824, trichostatin A (TSA), oxamflatin, scriptaid, m-carboxy cinnamic acid bishydroxamic acid, or trapoxin-hydroxamic acid analogue;
- cyclic tetrapeptides for example trapoxin, apidicin or depsipeptide;
- benzamides for example MS-275 or CI-994, or
- depudecin.

For the treatment of cancer the compounds according to the present invention may be administered to a patient as described above, in conjunction with irradiation. Irradiation means ionising radiation and in particular gamma radiation, especially that emitted by linear accelerators or by radionuclides that are in common use today. The irradiation of the tumour by radionuclides can be external or internal.

The present invention also relates to a combination according to the invention of an anti-cancer agent and a HDAC inhibitor according to the invention.

The present invention also relates to a combination according to the invention for use in medical therapy for example for inhibiting the growth of tumour cells.

The present invention also relates to a combinations according to the invention for inhibiting the growth of tumour cells.

The present invention also relates to a method of inhibiting the growth of tumour cells in a human subject which comprises administering to the subject an effective amount of a combination according to the invention.

This invention further provides a method for inhibiting the abnormal growth of cells, including transformed cells, by administering an effective amount of a combination according to the invention.

The other medicinal agent and HDAC inhibitor may be administered simultaneously (e.g. in separate or unitary compositions) or sequentially in either order. In the latter case, the two compounds will be administered within a period and in an amount and manner that is sufficient to ensure that an advantageous or synergistic effect is achieved. It will be appreciated that the preferred method and order of administration and the respective dosage amounts and regimes for each component of the combination will depend on the particular other medicinal agent and HDAC inhibitor being administered, their route of administration, the particular tumour being treated and the particular host being treated. The optimum method and order of administration and the dosage amounts and regime can be readily determined by those skilled in the art using conventional methods and in view of the information set out herein.

The platinum coordination compound is advantageously administered in a dosage of 1 to 500mg per square meter ($mg/m^2$) of body surface area, for example 50 to 400 $mg/m^2$, particularly for cisplatin in a dosage of about 75 $mg/m^2$ and for carboplatin in about 300$mg/m^2$ per course of treatment.

The taxane compound is advantageously administered in a dosage of 50 to 400 mg per square meter ($mg/m^2$) of body surface area, for example 75 to 250 $mg/m^2$, particularly for paclitaxel in a dosage of about 175 to 250 $mg/m^2$ and for docetaxel in about 75 to 150 $mg/m^2$ per course of treatment.

The camptothecin compound is advantageously administered in a dosage of 0.1 to 400 mg per square meter ($mg/m^2$) of body surface area, for example 1 to 300 $mg/m^2$, particularly for irinotecan in a dosage of about 100 to 350 $mg/m^2$ and for topotecan in about 1 to 2 $mg/m^2$ per course of treatment.

The anti-tumor podophyllotoxin derivative is advantageously administered in a dosage of 30 to 300 mg per

square meter ($mg/m^2$) of body surface area, for example 50 to 250$mg/m^2$, particularly for etoposide in a dosage of about 35 to 100 $mg/m^2$ and for teniposide in about 50 to 250 $mg/m^2$ per course of treatment.

The anti-tumor vinca alkaloid is advantageously administered in a dosage of 2 to 30 mg per square meter ($mg/m^2$) of body surface area, particularly for vinblastine in a dosage of about 3 to 12 $mg/m^2$ , for vincristine in a dosage of about 1 to 2 $mg/m^2$, and for vinorelbine in dosage of about 10 to 30 $mg/m^2$ per course of treatment.

The anti-tumor nucleoside derivative is advantageously administered in a dosage of 200 to 2500 mg per square meter ($mg/m^2$) of body surface area, for example 700 to1500 $mg/m^2$, particularly for 5-FU in a dosage of 200 to 500$mg/m^2$, for gemcitabine in a dosage of about 800 to 1200 $mg/m^2$ and for capecitabine in about 1000 to 2500 $mg/m^2$ per course of treatment.

The alkylating agents such as nitrogen mustard or nitrosourea is advantageously administered in a dosage of 100 to 500 mg per square meter ($mg/m^2$) of body surface area, for example 120 to 200 $mg/m^2$, particularly for cyclophosphamide in a dosage of about 100 to 500 $mg/m^2$ , for chlorambucil in a dosage of about 0.1 to 0.2 mg/kg, for carmustine in a dosage of about 150 to 200 $mg/m^2$ , and for lomustine in a dosage of about 100 to 150 $mg/m^2$ per course of treatment.

The anti-tumor anthracycline derivative is advantageously administered in a dosage of 10 to 75 mg per square meter ($mg/m^2$) of body surface area, for example 15 to 60 $mg/m^2$, particularly for doxorubicin in a dosage of about 40 to 75 $mg/m^2$, for daunorubicin in a dosage of about 25 to 45$mg/m^2$ , and for idarubicin in a dosage of about 10 to 15 $mg/m^2$ per course of treatment.

Trastuzumab is advantageously administered in a dosage of 1 to 5mg per square meter ($mg/m^2$) of body surface area, particularly 2 to 4$mg/m^2$ per course of treatment.

The antiestrogen agent is advantageously administered in a dosage of about 1 to 100mg daily depending on the particular agent and the condition being treated. Tamoxifen is advantageously administered orally in a dosage of 5 to 50 mg, preferably 10 to 20 mg twice a day, continuing the therapy for sufficient time to achieve and maintain a therapeutic effect. Toremifene is advantageously administered orally in a dosage of about 60mg once a day, continuing the therapy for sufficient time to achieve and maintain a therapeutic effect. Anastrozole is advantageously administered orally in a dosage of about 1mg once a day. Droloxifene is advantageously administered orally in a dosage of about 20-100mg once a day. Raloxifene is advantageously administered orally in a dosage of about 60mg once a day. Exemestane is advantageously administered orally in a dosage of about 25mg once a day.

These dosages may be administered for example once, twice or more per course of treatment, which may be repeated for example every 7,14, 21 or 28 days.

In view of their useful pharmacological properties, the components of the combinations according to the invention, i.e. the other medicinal agent and the HDAC inhibitor may be formulated into various pharmaceutical forms for administration purposes. The components may be formulated separately in individual pharmaceutical compositions or in a unitary pharmaceutical composition containing both components.

The present invention therefore also relates to a pharmaceutical composition comprising the other medicinal agent and the HDAC inhibitor together with one or more pharmaceutical carriers.

The present invention also relates to a combination according to the invention in the form of a pharmaceutical composition comprising an anti-cancer agent and a HDAC inhibitor according to the invention together with one or more pharmaceutical carriers.

The present invention further relates to the use of a combination according to the invention in the manufacture of a pharmaceutical composition for inhibiting the growth of tumour cells.

The present invention further relates to a product containing as first active ingredient a HDAC inhibitor according to the invention and as second active ingredient an anticancer agent, as a combined preparation for simultaneous, separate or sequential use in the treatment of patients suffering from cancer.

## Experimental part

The following examples are provided for purposes of illustration.

Hereinafter "DCM" means dichloromethane, "DMF" means dimethylformamide, "EtOAc" means ethyl acetate, "iPrOH" means isopropyl, "MeOH" means methanol, "EtOH" means ethanol, "TEA"means triethylamine, "TFA" means trifluoroacetic acid, "THF" means tetrahydrofuran., "BSA" means bovine serum albumine, "DMSO" means dimethylsulfoxide, and "Hepes" means 4-(-2-hydroxyethyl)-1-piperazineethanesulfonic acid.

$[\alpha]_D^{20}$ indicates the optical rotation measured with light at the wavelenght of the D-line of sodium at a temperature of 20°C. Behind the actual value the concentration and solvent of the solution which was used to measure the optical rotation are mentioned.

A. Preparation of the intermediates

Example A1

a) Preparation of

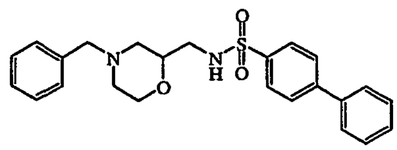

intermediate 1

A solution of [1,1'-biphenyl]-4-sulfonyl chloride (0.016 mol) in DCM (50ml) was added dropwise at 0˚C to a solution of 4-(phenylmethyl)-2-morpholinemethanamine (0.0145 mol) and TEA (0.023 mol) in DCM (50ml). The mixture was brought to room temperature, then stirred overnight, poured out into ice water and extracted with DCM. The organic layer was separated, dried ($MgSO_4$), filtered, and the solvent was evaporated. The residue (6g) was crystallized from diethyl ether. The precipitate was filtered off and dried, yielding 3.1g (62%) of intermediate 1, melting point 128˚C.

b) Preparation of

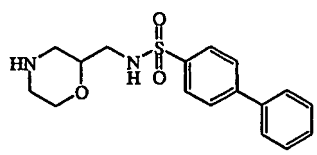

intermediate 2

A mixture of intermediate 1 (0.0071 mol) and Pd/C (0.5g) in MeOH (50ml) and acetic acid (5ml) was hydrogenated at room temperature for 5 days under a 3 bar pressure, then filtered over celite. Celite was washed with DCM/MeOH. The filtrate was evaporated. The residue (3g) was taken up in diethyl ether. The precipitate was filtered, washed with diethyl ether and dried, yielding 2.6g (100%) of intermediate 2, melting point 151˚C.

c) Preparation of

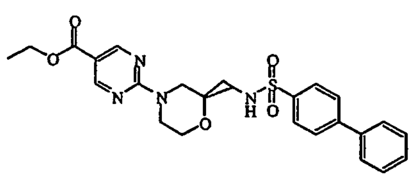

(A)
intermediate 3

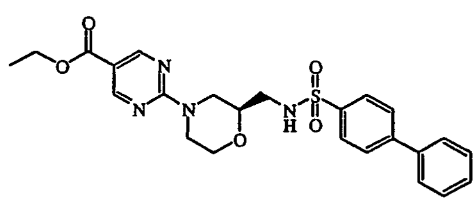

(B)
intermediate 4

Sodium hydride 60% (0.014 mol) was added portionwise at 0˚C to a mixture of intermediate 2 (0.0069 mol) in THF (30ml) under $N_2$ flow. The mixture was stirred at 0˚C for 1 hour. A solution of 2-(methylsulfonyl)-5-pyrimidinecarboxylic acid, ethyl ester (0.009 mol) in THF (20ml) was added dropwise. The mixture was stirred at room temperature for 4 hours, poured out into ice water and extracted with EtOAc. The organic layer was washed with water, dried ($MgSO_4$),

filtered and the solvent was evaporated. The residue (3g) was purified by column chromatography over silica gel (15-40μm)(eluent: DCM/MeOH 99/5). The pure fractions were collected and the solvent was evaporated. The residue (0.6g) was purified by column chromatography over silica gel (chiralpak)(eluent: $CH_3CN$ 100). Two fractions were collected and the solvent was evaporated, yielding 0.255g (8%) of intermediate 3 (A), $[\alpha]_D^{20}$ = -32.6 (c=0.00485 DMF) and 0.25g (8%) of intermediate 4 (B), $[\alpha]_D^{20}$= +33.8 (c=0.005, DMF).

Example A2

a) Preparation of

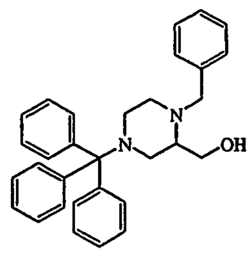

intermediate 5

Tetrahydro- aluminate(1-), lithium (0.04 mol) was added portionwise at 5°C to THF (40ml) under $N_2$ flow. A solution of 1-benzyl-4-triphenylmethylpiperazine-2-carboxylic acid ethyl ester (0.01 mol) in THF (40ml) was added dropwise. The mixture was stirred for 2 hours, poured out into EtOAc/water and filtered over celite. The organic layer was separated, dried ($MgSO_4$), filtered and the solvent was evaporated, yielding 4.05g of intermediate 5. This product was used directly in the next reaction step.

b) Preparation of

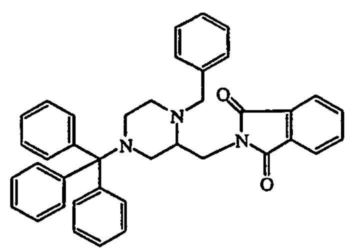

intermediate 6

A solution of diazenedicarboxylic acid, bis(1-methylethyl) ester (0.0097 mol) in THF (10ml) was added dropwise at 5°C to a solution of intermediate 5 (0.0064 mol), 1*H*-isoindole-1,3(2*H*)-dione (0.0097 mol) and triphenyl-phosphine (0.0097 mol) in THF (50ml) under $N_2$ flow. The mixture was stirred at room temperature for 6 hours, poured out into ice water and extracted with EtOAc. The organic layer was separated, dried ($MgSO_4$), filtered, and the solvent was evaporated. The residue (11g) was purified by column chromatography over silica gel (15-40μm)(eluent: DCM/EtOAc 99/1). The pure fractions were collected and the solvent was evaporated, yielding: 2.8g (75%) of intermediate 6, melting point 100°C.

c) Preparation of

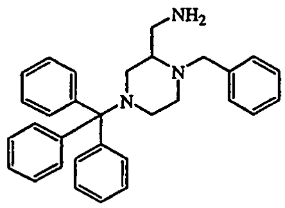

intermediate 7

Hydrazine monohydrobromide (0.005 mol) was added to a solution of intermediate 6 (0.0025 mol) in EtOH (25ml). The mixture was stirred and refluxed for 4 hours, then cooled to room temperature. The solvent was evaporated. The residue was taken up in NaCl and extracted with EtOAc/DCM. The organic layer was separated, dried ($MgSO_4$), filtered, and the solvent was evaporated, yielding 3.55g of intermediate 7. This product was used directly in the next reaction step.

d) Preparation of

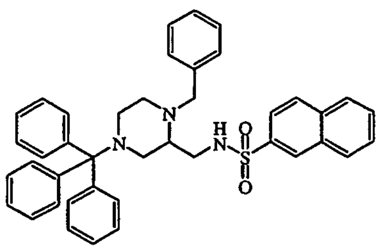

intermediate 8

A mixture of intermediate 7 (0.0025 mol), 2-naphthalenesulfonyl chloride (0.0027 mol) and TEA (0.004 mol) in DCM (20ml) was stirred at room temperature overnight, poured out into ice water and extracted with DCM. The organic layer was separated, dried ($MgSO_4$), filtered, and the solvent was evaporated. The residue (3.8g) was purified by column chromatography over silica gel (15-40μm)(eluent: DCM/EtOAc 98/2). The pure fractions were collected and the solvent was evaporated. The residue (0.8g) was crystallized from diethyl ether. The precipitate was filtered off and dried, yielding 0.693g (41 %) of intermediate 8, melting point 219°C.

e) Preparation of

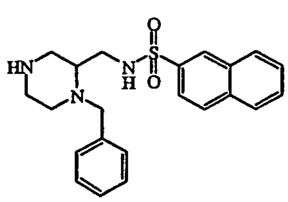

intermediate 9

A mixture of intermediate 8 (0.0009 mol) in HCl 12N (0.6ml) and 2-propanone (18ml) was stirred at room temperature for 4 hours. The solvent was evaporated. The residue was taken up in water. The aqueous layer was washed with diethyl ether, basified with potassium carbonate and extracted with EtOAc. The organic layer was separated, dried ($MgSO_4$), filtered, and the solvent was evaporated. The residue (0.5g) was crystallized from diethyl ether. The

precipitate was filtered off and dried. The residue (0.38g) was taken up in water/EtOAc. The organic layer was evaporated. The residue was taken up in diethyl ether. The precipitate was filtered off and dried, yielding 0.044g (48%) of intermediate 9, melting point 210°C.

f) Preparation of

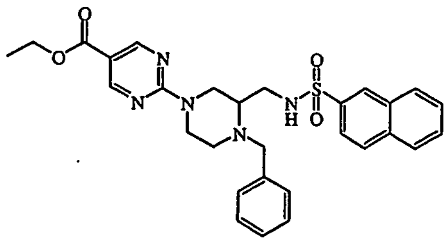

intermediate 10

2-(Methylsulfonyl)- 5-pyrimidinecarboxylic acid, ethyl ester (0.0029 mol) was added at room temperature to a solution of intermediate 9 (0.002 mol) and potassium carbonate (0.007 mol) in acetonitrile (50ml). The mixture was stirred at room temperature for 3 hours, then stirred at 80°C overnight, cooled to room temperature, poured out into ice water and extracted with EtOAc. The organic layer was separated, dried ($MgSO_4$), filtered, and the solvent was evaporated. The residue (2g) was purified by column chromatography over silica gel (15-40μm)(eluent: DCM/MeOH 99/1). The pure fractions were collected and the solvent was evaporated, yielding 0.76g (48%) of intermediate 10.

Example A3

a) Preparation of

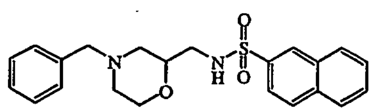

intermediate 11

A solution of 2-naphthalenesulfonyl chloride (0.016 mol) in DCM (50ml) was added dropwise at 0°C to a solution of 4-(phenylmethyl)-2-morpholinemethanamine (0.015 mol) and TEA (0.024 mol) in DCM (50ml) under $N_2$ flow. The mixture was stirred at room temperature overnight, poured out into ice water and extracted with DCM. The organic layer was separated, dried ($MgSO_4$), filtered, and the solvent was evaporated, yielding 6g (100%) of intermediate 11. This product was used directly in the next reaction step.

b) Preparation of

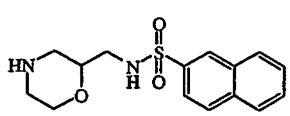

intermediate 12

A solution of carbonochloridic acid, 1-chloroethyl ester (0.016 mol) in 1,2-dichloroethane (2ml) was added at room temperature to a mixture of intermediate 11 (0.414 mol) in 1,2-dichloro- ethane (48ml). The mixture was stirred at room temperature for 45 minutes, then at 80˚C for 3 hours. MeOH (100m1) was added. The mixture was stirred at 80˚C for 4 days, poured out into water and extracted with EtOAc. The organic layer was separated, dried ($MgSO_4$), filtered, and the solvent was evaporated. The residue (3.5g) was purified by column chromatography over silica gel (15-40μm) (eluent: DCM/MeOH/$NH_4OH$ 92/8/0.5). The pure fractions were collected and the solvent was evaporated. The residue (0.38g, 9%) was crystallized from diethyl ether. The precipitate was filtered off and dried, yielding 0.21g of intermediate 12, melting point 140˚C.

Example A4

a) Preparation of

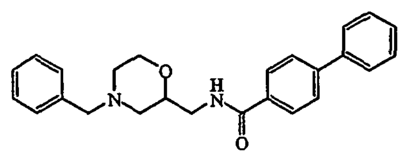

intermediate 13

A solution of [1,1'-biphenyl]-4-carbonyl chloride (0.016 mol) in DCM (50ml) was added dropwise at 0˚C to a solution of 4-(phenylmethyl)-2-morpholinemethanamine (0.0145 mol) and TEA (0.023 mol) in DCM (50ml). The mixture was stirred at room temperature for 12 hours, poured out into ice water and extracted with DCM. The organic layer was washed with potassium carbonate 10%, separated, dried ($MgSO_4$), filtered, and the solvent was evaporated. The residue (6.2g) was crystallized from $CH_3CN$/diethyl ether. The precipitate was filtered off and dried. The mother layer was evaporated. The residue (3.3g)was purified by column chromatography over silica gel (15-40μm)(eluent: DCM/MeOH/$NH_4OH$ 98/2/0.1). The pure fractions were collected and the solvent was evaporated, yielding 1.5g (27%). A fraction (0.39g) was crystallized from $CH_3CN$/diethyl ether. The precipitate was filtered off and dried, yielding 0.12g of intermediate 13, melting point 133˚C.

b) Preparation of

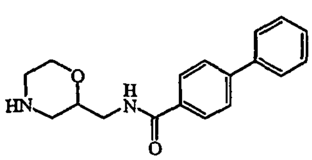

intermediate 14

Carbonochloridic acid, 1-chloroethyl ester (0.0066 mol) was added at room temperature to a mixture of intermediate 13 (0.006 mol) in 1,2-dichloro- ethane (35ml). The mixture was stirred at room temperature for 45 minutes, then stirred at 80˚C for 3 hours. MeOH (60ml) was added. The mixture was stirred at 80˚C for 8 days, then cooled to room temperature. The solvent was evaporated. EtOAc was added. The precipitate was filtered, washed with diethyl ether and dried, yielding 1.8g (100%) of intermediate 14, melting point 284˚C.

Example A5

a) Preparation of

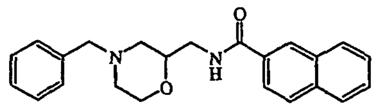

intermediate 15

A solution of 2-naphthalenecarbonyl chloride (0.017 mol) in DCM (60ml) was added dropwise at 0°C to a mixture of 4-(phenylmethyl)-2-morpholinemethanamine (0.015 mol) and TEA (0.026 mol) in DCM (60ml). The mixture was brought to room temperature overnight and poured out into ice water. The organic layer was separated, washed with potassium carbonate 10%, dried ($MgSO_4$), filtered and the solvent was evaporated. The residue was crystallized from diethyl ether/DIPE. The precipitate was filtered off and dried, yielding 4.6g (85%) of intermediate 15, melting point 104°C.

b) Preparation of

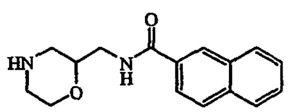

intermediate 16

A mixture of intermediate 15 (0.0109 mol) and Pd/C (2g) in MeOH (80ml) and acetic acid (8ml) was hydrogenated at room temperature for 4 days under a 3 bar pressure, then filtered over celite. Celite was washed with MeOH/DCM. The filtrate was evaporated. The residue (7.2g) was purified by column chromatography over silica gel (15-40μm)(eluent: DCM/MeOH/$NH_4OH$ 97/3/0.1). The pure fractions were collected and the solvent was evaporated, yielding 0.8g of intermediate 16.

Example A6

a) Preparation of

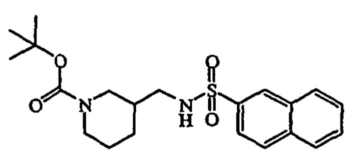

intermediate 17

A solution of 2-naphthalenesulfonyl chloride (0.011 mol) in DCM (5ml) was added at 5°C to a mixture of 3-(aminomethyl)-1-piperidinecarboxylic acid, 1,1-dimethylethyl ester (0.01 mol) and TEA (0.014 mol) in DCM (15ml).

The mixture was stirred at 20˚C for 18 hours. Potassium carbonate 10% was added. The organic layer was separated, dried ($MgSO_4$), filtered, and the solvent was evaporated till dryness, yielding 4.6g (>100%) of intermediate 17.

b) Preparation of

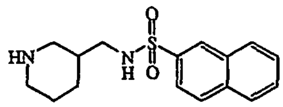

intermediate 18

A mixture of intermediate 17 (0.0089 mol) in HCl/iPrOH 5N (40ml) was stirred at 50˚C for 15 minutes, basified with $NH_4OH$. The solvent was evaporated till dryness. The residue was taken up in DCM and filtered. The filtrate was dried ($MgSO_4$), filtered and the solvent was evaporated till dryness, yielding 2.9g (>100%) of intermediate 18.

Example A7

a) preparation of

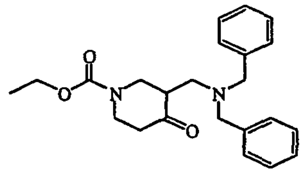

intermediate 19

N-(phenylmethyl)- benzenemethanamine (1 mol) was dissolved in diethyl ether and converted into the hydrochloric acid salt (1:1) with 6 N HCl/2-propanol. The precipitate was filtered off and dried to give 234g which was added with 4-oxo-1-piperidinecarboxylic acid, ethyl ester and $(CH_2O)_n$ (30 g) in acetic acid (1600 ml). The mixture was stirred for 110 min at 60-65 ˚C, then cooled to room temperature and poured out onto ice/water/$NH_4OH$. The resultant white oily precipitate was extracted with diethyl ether. The separated organic layer was washed with water, dried ($MgSO_4$), filtered and the solvent evaporated at room temperature, yielding 436 g of intermediate 19.

b) Preparation of

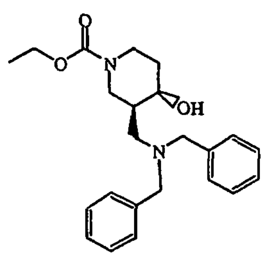

(TRANS)
intermediate 20

Intermediate 19 (max. 0.55 mol crude residue) was stirred in EtOH (1500 ml). Part of sodium hydroborate was added portionwise resulting in an exothermic temperature rise to 30 ˚C. Therefore the reaction mixture was cooled on

an ice-water bath and more sodium hydroborate (in total, 1.5 mol) was added while the reaction temperature remained at ± 16 ˚C. The reaction mixture was stirred for one hour at ± 16 ˚C. The mixture was concentrated to half the initial volume by evaporation. The concentrate was cooled. Water was added. The mixture was concentrated further (heavy foaming!) until all ethanol was evaporated. The aqueous concentrate was cooled, then extracted with diethyl ether. The organic layer was separated, washed with water, dried ($MgSO_4$), filtered and the solvent was evaporated. The oily residue was dissolved in DIPE, and treated with HCl/2-propanol. A sticky precipitation resulted. The solvent was evaporated. The residue was suspended in warm acetonitrile, then cooled and the precipitate was removed by filtration. The filtrate was evaporated. The residue was dissolved in water, alkalised with $NH_4OH$, then extracted with diethyl ether. The separated organic layer was dried ($MgSO_4$), filtered and the solvent evaporated. The residue (267g) was separated by HPLC (eluent: toluene/ethanol 98.5/1.5). The pure factions were collected and the solvent was evaporated. One of the fractions was crystallized from acetonitrile, filtered off and dried, yielding 107 g (TRANS) of intermediate 20.

c) Preparation of

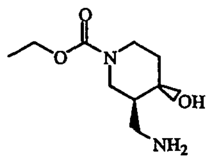

(TRANS)
intermediate 21

A mixture of intermediate 20 (0.01 mol) and Pd/C (1.5g) in EtOH (200ml) was hydrogenated at 50˚C overnight under a 3 bar pressure, then filtered over celite. The filtrate was evaporated till dryness, yielding 2.1g (>100%) of intermediate 21.

d) Preparation of

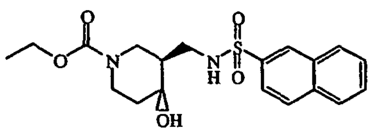

(TRANS)
intermediate 22

A solution of 2-naphthalenesulfonyl chloride (0.0054 mol) in DCM (2ml) was added at 5˚C to a mixture of intermediate 21 (0.0049 mol) and TEA (0.0069 mol) in DCM (10ml) under $N_2$ flow. The mixture was stirred at room temperature for 18 hours. Potassium carbonate 10% was added. The mixture was extracted with DCM. The organic layer was separated, dried ($MgSO_4$), filtered, and the solvent was evaporated till dryness, yielding 2g (100%) of intermediate 22 (TRANS).

e) Preparation of

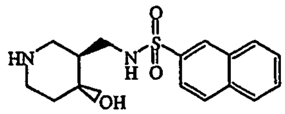

(TRANS)
intermediate 23

A mixture of intermediate 22 (0.0043 mol) in HCL 6N (20ml) was stirred and refluxed for 24 hours. The solvent was evaporated till dryness. The residue was basified with NaOH 3N. EtOAc was added. The mixture was stirred at room temperature overnight. The precipitate was filtered, washed with diethyl ether and dried, yielding 1.37g (97%) of intermediate 23 (TRANS).

B. Preparation of the final compounds

Example B1

a) Preparation of

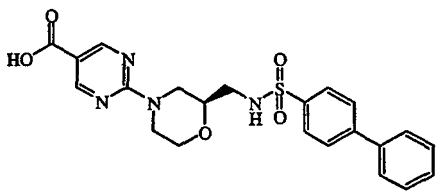

.Na
(B)
intermediate 24

A mixture of intermediate 4 (0.0004 mol) and NaOH (0.0008 mol) in EtOH (10ml) was stirred at 80°C for 48 hours, then cooled to room temperature. The precipitate was filtered, washed with diethyl ether and dried, yielding 0.188g (90%) of intermediate 24 (B) Na.

b) Preparation of

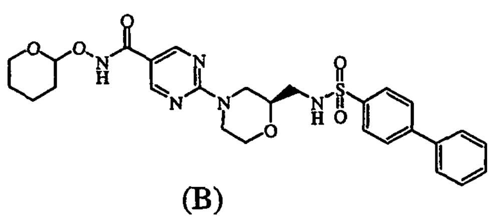

(B)
intermediate 25

A solution of *N'*-(ethylcarbonimidoyl)-*N,N*-dimethyl-1,3-propanediamine, monohydrochloride (0.0005 mol) in DCM (5ml) then a solution of 1-hydroxy-1*H*-benzotriazole (0.0005 mol) in THF (5ml) were added at room temperature to a mixture of intermediate 24 (0.0003 mol) and O-(tetrahydro-2*H*-pyran-2-yl)- hydroxylamine (0.0005 mol) in DCM (10ml). The mixture was stirred at room temperature overnight, poured out into water and extracted with DCM. The organic layer was separated, dried ($MgSO_4$), filtered, and the solvent was evaporated. The residue (0.29g) was purified by column chromatography over silica gel (10μm)(eluent: DCM/MeOH 99/1). The pure fractions were collected and the solvent was evaporated, yielding 0.142g (66%) of intermediate 25 (B).

c) Preparation of

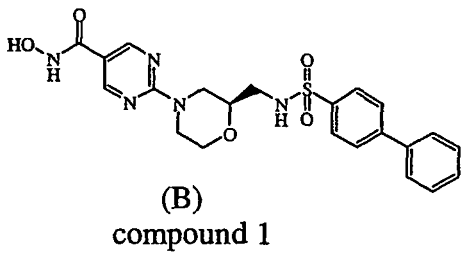

(B)
compound 1

A mixture of intermediate 25 (B) (0.0002 mol) in TFA (1ml) and MeOH (15ml) was stirred at room temperature for 4 days. The precipitate was filtered off and dried. The residue (0.08g) was taken up in MeOH/$CH_3CN$. The precipitate was filtered, washed with MeOH, then with diethyl ether and dried, yielding 0.046g of compound 1 (B), $[\alpha]_D^{20}$=+32.85 (c=0.0047, DMF), melting point 164˚C.

Example B2

Preparation of

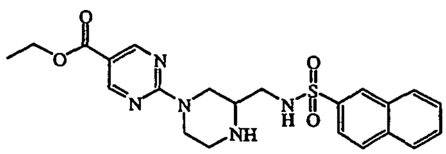

intermediate 26

A mixture of intermediate 10 (0.0088 mol) and Pd/C 10% (1.3g) in acetic acid (2ml) and EtOH (200ml) was stirred at room temperature for 9 days under a 3 bar pressure, then filtered over celite. The filtrate was evaporated till dryness. The mixture was filtered over celite. The filtrate was evaporated till dryness. The residue was taken up in DCM. The organic layer was washed with potassium carbonate 10%, dried ($MgSO_4$), filtered, and the solvent was evaporated. The residue (2.7g) was purified by column chromatography over silica gel (15-40μm)(eluent: toluene/iPrOH/$NH_4OH$ 90/10/0.2). The pure fractions were collected and the solvent was evaporated, yielding 0.29g (8%) of intermediate 26. Intermediate 26 was handled analogously as described in example [B1] to give 0.065g (51%) of compound 2, melting point 243˚C.

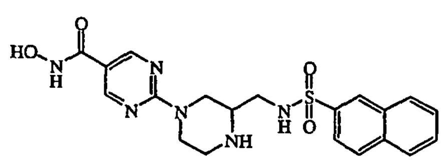

compound 2

Example B3

Intermediate 10 was handled analogously as described in example [B1] to give 0.26g (100%) of compound 3, melting point 135˚C.

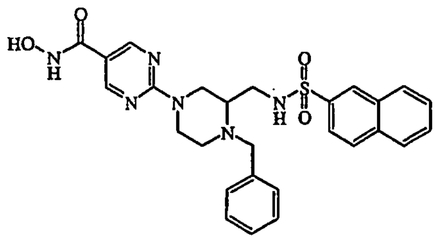

compound 3

Example B4

Preparation of

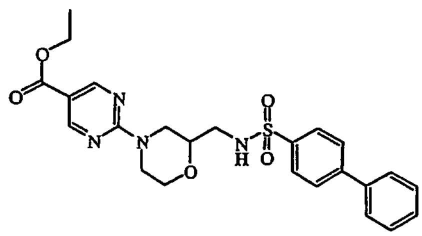

intermediate 27

Sodium hydride 60% (0.0059 mol) was added at 0˚C to a mixture of intermediate 2 (0.0039 mol) in THF (15ml) under $N_2$ flow. The mixture was stirred at 0˚C for 1 hour. A solution of 2-(methylsulfonyl)- 5-pyrimidinecarboxylic acid, ethyl ester (0.0051 mol) in THF (10ml) was added dropwise. The mixture was stirred at 0˚C for 2 hours, then brought to room temperature for 2 hours, poured out into ice water and extracted with EtOAc. The organic layer was separated, dried ($MgSO_4$) filtered, and the solvent was evaporated. The residue (2g) was purified by column chromatography over silica gel (15-40μm)(eluent: DCM/MeOH/$NH_4OH$ 97/3/0.1). The pure fractions were collected and the solvent was evaporated. The residue was crystallized from diethyl ether. The precipitate was filtered off and dried, yielding 0.075g of intermediate 27, melting point 170˚C.
Intermediate 27 was handled analogously as described in example [B1] to give 0.236g (64%) of compound 4, melting point 163˚C.

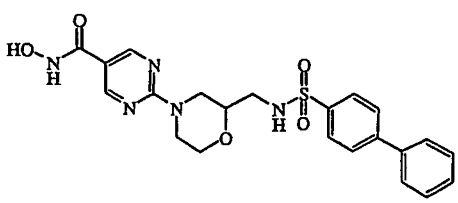

· compound 4

Example B5

Preparation of

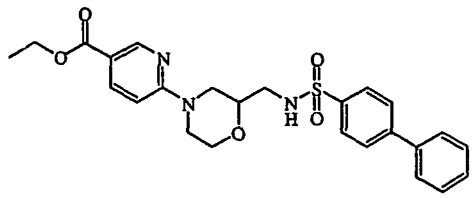

intermediate 28

Sodium hydride 60% (0.0043 mol) was added portionwise at 0˚C to a mixture of intermediate 2 (0.0036 mol) and 6-chloro-3-pyridinecarboxylic acid, ethyl ester (0.0047 mol) in DMF (20ml) under $N_2$ flow. The mixture was stirred at 90˚C for 12 hours, then cooled to room temperature and poured out into ice water. EtOAc was added. The mixture was extracted with EtOAc. The organic layer was washed with water, dried ($MgSO_4$), filtered, and the solvent was evaporated. The residue (1.7g) was purified by column chromatography over silica gel (15-40μm)(eluent: DCM/EtOAc 85/15 then DCM/MeOH/$NH_4OH$ 97/3/0.1). The pure fractions were collected and the solvent was evaporated. The residue (0.42g) was crystallized from diethyl ether. The precipitate was filtered off and dried, yielding 0.36g (22%) of intermediate 28, melting point 186˚C.
Intermediate 28 was handled analogously as described in example [B1] to give 0.155g (62%) of compound 5, melting point 210˚C.

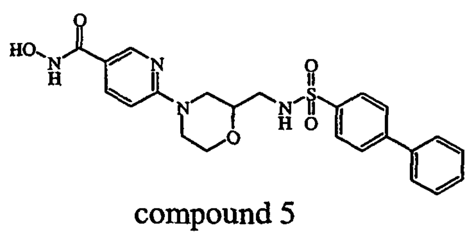

compound 5

Example B6

Preparation of

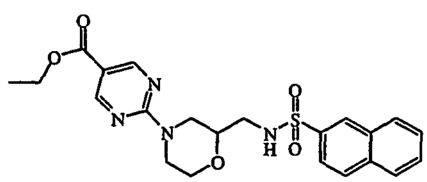

intermediate 29

Sodium hydride (0.0032 mol) was added at 0˚C to a mixture of intermediate 12 (0.0016 mol) in THF (10ml) under $N_2$ flow. The mixture was stirred at room temperature for 1 hour. A solution of 2-(methylsulfonyl)- 5-pyrimidinecarboxylic acid, ethyl ester (0.0019 mol) in THF (10ml) was added dropwise at 0˚C. The mixture was stirred at room temperature for 2 hours. Ice and EtOAc were added. The mixture was extracted with EtOAc. The organic layer was separated, dried ($MgSO_4$), filtered, and the solvent was evaporated. The residue (0.75g) was purified by column chromatography over silica gel (15-40μm)(eluent: DCM/MeOH 99/1). The pure fractions were collected and the solvent was evaporated, yielding 0.22g (73%) of intermediate 29.
Intermediate 29 was handled analogously as described in example [B1] to give 0.055g (50%) of compound 6, melting point 179˚C.

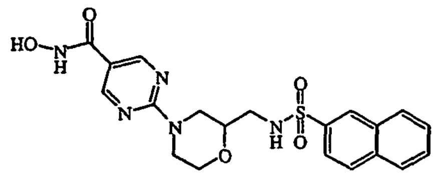

compound 6

Example B7

Preparation of

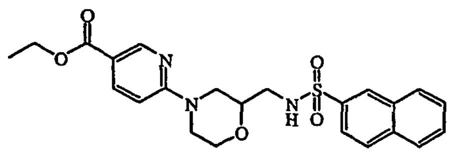

intermediate 30

Sodium hydride 60% (0.0052 mol) was added at 0˚C to a mixture of intermediate 12 (0.0026 mol) in DMF (20ml) under $N_2$ flow. The mixture was stirred at 0˚C for 1 hour. A solution of 6-chloro-3-pyridinecarboxylic acid, ethyl ester (0.0034 mol) in DMF (10ml) was added dropwise at 0˚C. The mixture was brought to room temperature, then stirred at 90˚C for 12 hours, poured out into ice water and extracted with DCM. The organic layer was washed with water, dried ($MgSO_4$), filtered and the solvent was evaporated. The residue (1.4g) was purified by column chromatography over silica gel (15-40μm)(eluent: cyclohexane/EtOAc 60/40). The pure fractions were collected and the solvent was evaporated, yielding 0.5g (42%) of intermediate 30.
Intermediate 30 was handled analogously as described in example [B1] to give 0.23g (66%) of compound 7, melting point 157˚C.

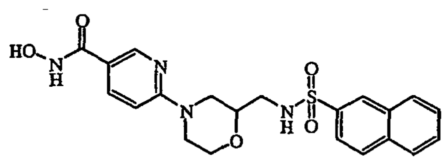

compound 7

Example B8

Preparation of

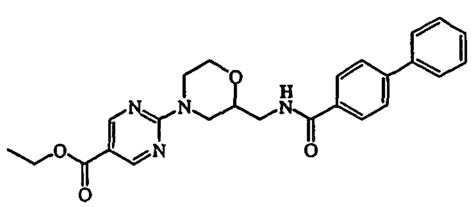

intermediate 31

A mixture of intermediate 14 (0.003 mol), 2-(methylsulfonyl)- 5-pyrimidinecarboxylic acid, ethyl ester (0.004 mol) and potassium carbonate (0.0061 mol) in acetonitrile (30ml) was stirred at room temperature for 12 hours, poured out into water and extracted with EtOAc. The organic layer was separated, washed with water, dried ($MgSO_4$), filtered and the solvent was evaporated. The residue (1.4g)was crystallized from $CH_3CN$/diethyl ether. The precipitate was filtered off and dried, yielding: 1.1g (79%) of intermediate 31, melting point 184˚C.
Intermediate 31 was handled analogously as described in example [B1] to give 0.156g (47%) of compound 8, melting point 290˚C.

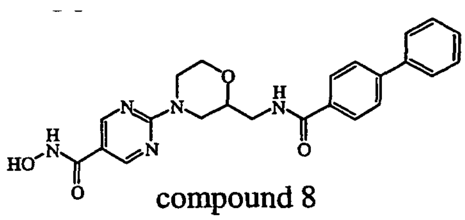

compound 8

Example B9

Preparation of

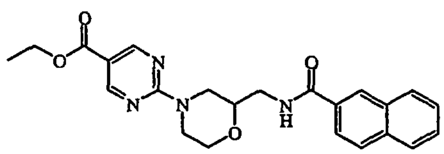

intermediate 32

Sodium hydride (0.0044 mol) was added at 0˚C to a mixture of intermediate 16 (0.0022 mol) in THF (15ml) under $N_2$ flow. The mixture was stirred at 0˚C for 1 hour. A solution of 2-(methylsulfonyl)- 5-pyrimidinecarboxylic acid, ethyl ester (0.0029 mol) in THF (5ml) was added dropwise. The mixture was stirred at room temperature for 4 hours, poured out into ice water and extracted with EtOAc. The organic layer was separated, dried ($MgSO_4$), filtered and the solvent was evaporated. The residue (1g) was purified by column chromatography over silica gel (15-40μm) (eluent: DCM/MeOH/$NH_4OH$ 97/3/0.1). The pure fractions were collected and the solvent was evaporated, yielding 0.58g (90%) of intermediate 32.
Intermediate 32 was handled analogously as described in example [B1] to give 0.091g (50%) of compound 9, melting point 246˚C.

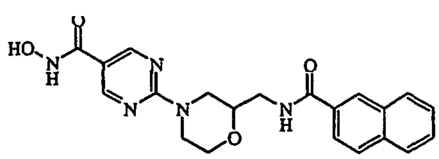

compound 9

Example B10

Preparation of

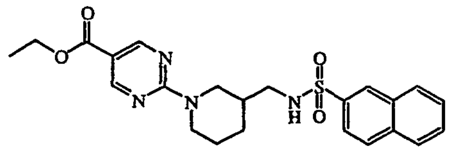

intermediate 33

Sodium hydride 60% in oil (0.0042 mol) was added portionwise at 5˚C to a mixture of intermediate 18 (0.0032 mol) in THF (10ml) under $N_2$ flow. The mixture was stirred for 30 minutes. A solution of 2-(methylsulfonyl)- 5-pyrimidinecarboxylic acid, ethyl ester (0.0039 mol) in THF (2ml) was added. The mixture was stirred at room temperature for 18 hours. Potassium carbonate 10% was added. The mixture was extracted with DCM. The organic layer was separated, dried ($MgSO_4$), filtered, and the solvent was evaporated till dryness. The residue (1.6g) was purified by column chromatography over silica gel (15-40μm)(eluent: DCM 100 to DCM/MeOH 98/2). The pure fractions were collected and the solvent was evaporated, yielding 0.9g (60%) of intermediate 33.
Intermediate 33 was handled analogously as described in example [B1] to give 0.19g (50%) of compound 10, melting point 210˚C.

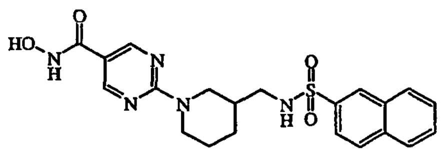

compound 10

Example B11

Preparation of

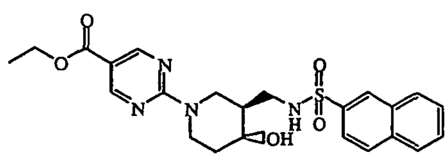

TRANS
Intermediate 34

Sodium hydride (0.0102 mol) was added portionwise at 5˚C to a mixture of intermediate 23 (0.004 mol) in THF (10ml) under $N_2$ flow. The mixture was stirred for 1 hour. A solution of 5-pyrimidinecarboxylic acid, 2-(methylsulfonyl)-, ethyl ester (0.0053 mol) in THF (5ml) was added. The mixture was stirred at room temperature for 24 hours. Potassium carbonate 10% was added. The mixture was extracted with DCM. The organic layer was separated, dried ($MgSO_4$), filtered, and the solvent was evaporated till dryness. The residue (1.1g) was purified by column chromatography over silica gel (15-40μm)(eluent: DCM/MeOH/$NH_4OH$ 95/5/0.1). The pure fractions were collected and the solvent was evaporated, yielding 0.17g (9%) of intermediate 34, melting point 189˚C.
Intermediate 34 was handled analogously as described in example [B1] to give 0.194g (68%) of compound 11 (TRANS), melting point 176˚C.

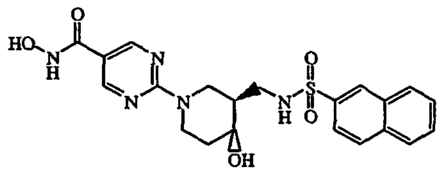

TRANS
compound 11

| | |
|---|---|
| 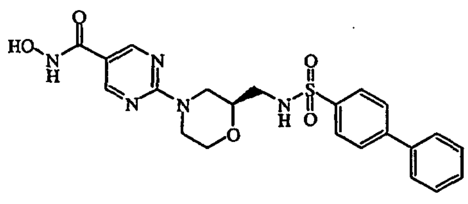 | 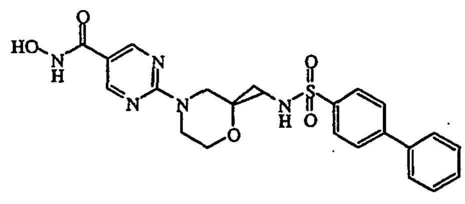 |
| (B); Co. No. 1; Ex. [B1]; mp. 164°C | (A); Co. No. 12; Ex. [B1]; mp. 175°C |
| 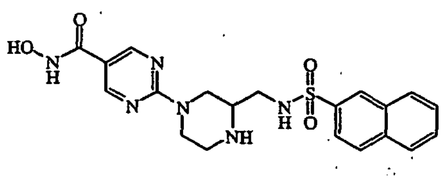 | 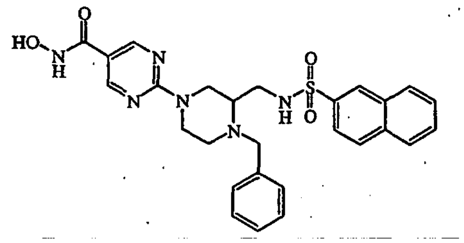 |
| Co. No. 2; Ex. [B2]; mp. 243°C | Co. No. 3; Ex. [B3]; mp. 135°C |
| 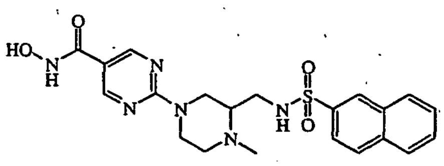 | 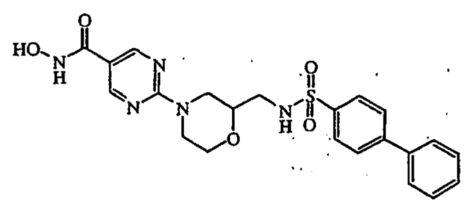 |
| $H_2O$ (1:1) .$C_2HF_3O_2$ (1:1); Co. No. 13; Ex. [B3] | Co. No. 4; Ex. [B4]; mp. 163°C |
| 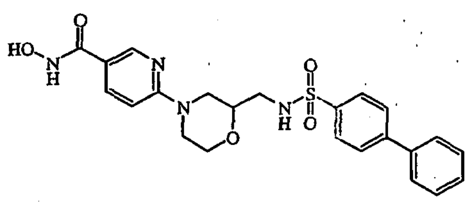 | 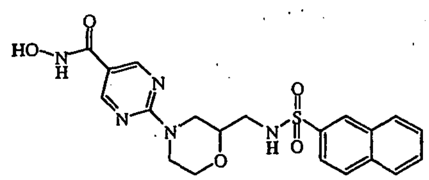 |
| Co. No. 5; Ex. [B5]; mp. 210°C | Co. No. 6; Ex. [B6]; mp 179°C |
| 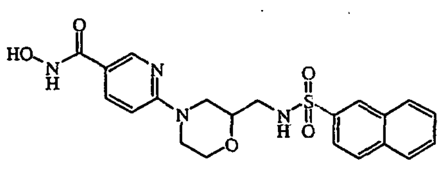 | 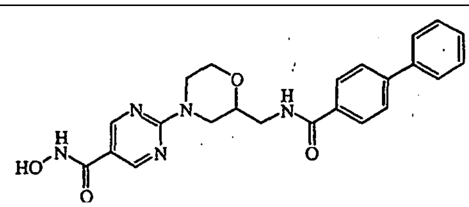 |
| Co. No. 7; Ex. [B7]; mp. 157°C | Co. No. 8; Ex. [B8]; mp. 290°C |

(continued)

| | |
|---|---|
| 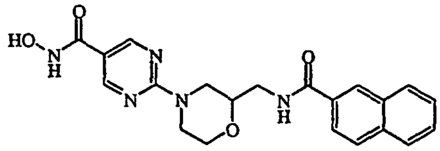 | 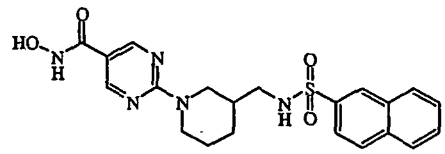 |
| Co. No. 9; Ex. [B9]; mp. 246˚C | Co. No. 10; Ex. [B10]; mp. 210˚C |
| 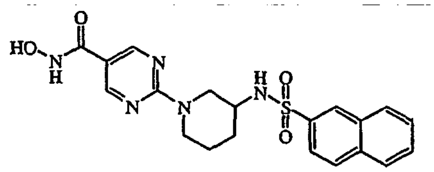 | 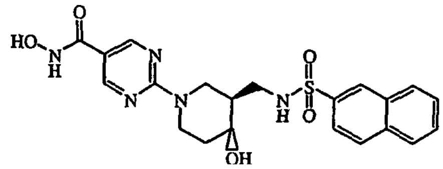 |
| Co. No. 14; Ex. [B10],mp. 150˚C | TRANS; Co. No.11; Ex. [B11]; mp. 176˚C |

C. Pharmacological example

The *in vitro* assay for inhibition of histone deacetylase (see example C.1) measures the inhibition of HDAC enzymatic activity obtained with the compounds of formula (I).

Cellular activity of the compounds of formula (I) was determined on A2780 tumour cells using a colorimetric assay for cell toxicity or survival (Mosmann Tim, Journal of Immunological Methods 65: 55-63, 1983)(see example C.2).

Kinetic solubility in aqueous media measures the ability of a compound to stay in aqueous solution upon dilution (see example C.3).
DMSO-stock solutions are diluted with a single aqueous buffer solvent in 3 consecutive steps. For every dilution turbidity is measured with a nephelometer.

Metabolism of drugs means that a lipid-soluble xenobiotic or endobiotic compound is enzymatically transformed into (a) polar, water-soluble, and excretable metabolite(s). The major organ for drug metabolism is the liver. The metabolic products are often less active than the parent drug or inactive. However, some metabolites may have enhanced activity or toxic effects. Thus drug metabolism may include both "detoxication" and "toxication" processes. One of the major enzyme systems that determine the organism's capability of dealing with drugs and chemicals is represented by the cytochrome P450 monooxygenases, which are NADPH dependent enzymes. Metabolic stability of compounds can be determined *in vitro* with the use of subcellular human tissue (see example C.4). Here metabolic stability of the compounds is expressed as % of drug metabolised after 15 minutes incubation of these compounds with microsomes. Quantitation of the compounds was determined by LC-MS analysis.

The tumour suppressor p53 transcriptionally activates a number of genes including the WAF1/CIP1 gene in response to DNA damage. The 21 kDa product of the WAF1 gene is found in a complex involving cyclins, cyclin dependent kinases (CDKs), and proliferating cell nuclear antigen (PCNA) in normal cells but not transformed cells and appears to be a universal inhibitor of CDK activity. One consequence of p21WAF1 binding to and inhibiting CDKs is to prevent CDK-dependent phosphorylation and subsequent inactivation of the Rb protein, which is essential for cell cycle progression. Induction of p21WAF1 in response to cellular contact with a HDAC inhibitor is therefore a potent and specific indicator of inhibition of cell cycle progression at both the G1 and G2 checkpoints.
The capacity of the compounds to induce p21WAF1 was measured with the p21WAF1 enzyme linked immunosorbent assay (WAF1 ELISA of Oncogene). The p21WAF1 assay is a "sandwich" enzyme immunoassay employing both mouse monoclonal and rabbit polyclonal antibodies. A rabbit polyclonal antibody, specific for the human WAF1 protein, has been immobilized onto the surface of the plastic wells provided in the kit. Any p21WAF present in the sample to be assayed will bind to the capture antibody. The biotinylated detector monoclonal antibody also recognizes human p21WAF1 protein, and will bind to any p21WAF1, which has been retained by the capture antibody. The detector antibody, in turn, is bound by horseradish peroxidase-conjugated streptavidin. The horseradish peroxidase catalyses the conversion of the chromogenic substrate tetra-methylbenzidine from a colorless solution to a blue solution (or yellow after the addition of stopping reagent), the intensity of which is proportional to the amount of p21WAF1 protein bound to the plate. The colored reaction product is quantified using a spectrophotometer. Quantitation is achieved by the construction of a standard curve using known concentrations of p21WAF1 (provided lyophilised)(see example C.5).

Example C.1: *In Vitro* Assay for Inhibition of histone deacetylase:

HeLa nuclear extracts (supplier: Biomol) were incubated at 60 μg/ml with $2x10^{-8}$ M of radiolabeled peptide substrate. As a substrate for measuring HDAC activity a synthetic peptide, i.e. the amino acids 14-21 of histone H4, was used. The substrate is biotinylated at the $NH_2$-terminal part with a 6-aminohexanoic acid spacer, and is protected at the COOH-terminal part by an amide group and specifically [$^3$H]acetylated at lysine 16. The substrate, biotin-(6-aminohexanoic)Gly-Ala-([$^3$H]-acetyl-Lys-Arg-His-Arg-Lys-Val-$NH_2$), was added in a buffer containing 25 mM Hepes, 1 M sucrose, 0.1 mg/ml BSA and 0.01% Triton X-100 at pH 7.4. After 30 min the deacetylation reaction was terminated by the addition of HCl and acetic acid. (final concentration 0.035 mM and 3.8 mM respectively). After stopping the reaction, the free $^3$H-acetate was extracted with ethylacetate. After mixing and centrifugation, the radioactivity in an aliquot of the upper (organic) phase was counted in a β-counter.
For each experiment, controls (containing HeLa nuclear extract and DMSO without compound), a blank incubation (containing DMSO but no HeLa nuclear extract or compound) and samples (containing compound dissolved in DMSO and HeLa nuclear extract) were run in parallel. In first instance, compounds were tested at a concentration of $10^{-5}$M. When the compounds showed activity at $10^{-5}$M, a concentration-response curve was made wherein the compounds were tested at concentrations between $10^{-5}$M and $10^{-12}$M. In each test the blank value was substracted from both the control and the sample values. The control sample represented 100% of substrate deactylation. For each sample the radioactivity was expressed as a percentage of the mean value of the controls. When appropriate $IC_{50}$-values (concentration of the drug, needed to reduce the amount of metabolites to 50% of the control) were computed using probit analysis for graded data. Herein the effects of test compounds are expressed as $pIC_{50}$ (the negative log value of the $IC_{50}$-value). All tested compounds had a $pIC_{50} \geq 7$ (see table F-2).

Example C.2: Determination of antiproliferative activity on A2780 cells

All compounds tested were dissolved in DMSO and further dilutions were made in culture medium. Final DMSO concentrations never exceeded 0.1 % (v/v) in cell proliferation assays. Controls contained A2780 cells and DMSO without compound and blanks contained DMSO but no cells. MTT was dissolved at 5 mg/ml in PBS. A glycine buffer comprised of 0.1 M glycine and 0.1 M NaCl buffered to pH 10.5 with NaOH (1 N) was prepared (all reagents were from Merck).
The human A2780 ovarian carcinoma cells (a kind gift from Dr. T.C. Hamilton [Fox Chase Cancer Centre, Pennsylvania, USA]) were cultured in RPMI 1640 medium supplemented with 2 mM L-glutamine, 50 μg/ml gentamicin and 10 % fetal calf serum. Cells were routinely kept as monolayer cultures at 37˚C in a humidified 5 % $CO_2$ atmosphere. Cells were passaged once a week using a trypsin/EDTA solution at a split ratio of 1:40. All media and supplements were obtained from Life Technologies. Cells were free of mycoplasma contamination as determined using the Gen-Probe Mycoplasma Tissue Culture kit (supplier: BioMérieux).
Cells were seeded in NUNC™ 96-well culture plates (Supplier: Life Technologies) and allowed to adhere to the plastic overnight. Densities used for plating were 1500 cells per well in a total volume of 200 μl medium. After cell adhesion to the plates, medium was changed and drugs and/or solvents were added to a final volume of 200 μl. Following four days of incubation, medium was replaced by 200 μl fresh medium and cell density and viability was assessed using an MTT-based assay. To each well, 25 μl MTT solution was added and the cells were further incubated for 2 hours at 37˚C. The medium was then carefully aspirated and the blue MTT-formazan product was solubilized by addition of 25 μl glycine buffer followed by 100 μl of DMSO. The microtest plates were shaken for 10 min on a microplate shaker and the absorbance at 540 nm was measured using an Emax 96-well spectrophotometer (Supplier: Sopachem). Within an experiment, the results for each experimental condition are the mean of 3 replicate wells. For initial screening purposes, compounds were tested at a single fixed concentration of $10^{-6}$ M. For active compounds, the experiments were repeated to establish full concentration-response curves. For each experiment, controls (containing no drug) and a blank incubation (containing no cells or drugs) were run in parallel. The blank value was subtracted from all control and sample values. For each sample, the mean value for cell growth (in absorbance units) was expressed as a percentage of the mean value for cell growth of the control. When appropriate, $IC_{50}$-values (concentration of the drug, needed to reduce cell growth to 50% of the control) were computed using probit analysis for graded data (Finney, D.J., Probit Analyses, 2nd Ed. Chapter 10, Graded Responses, Cambridge University Press, Cambridge 1962). Herein the effects of test compounds are expressed as $pIC_{50}$ (the negative log value of the $IC_{50}$-value). Most of the tested compounds showed cellular activity at a test concentration of $10^{-6}$ M and 12 compounds had a $pIC_{50} \geq 5$ (see table F-2)

Example C.3: Kinetic solubility in aqueous media

In the first dilution step, 10 μl of a concentrated stock-solution of the active compound, solubilized in DMSO (5mM), was added to 100 μl phosphate citrate buffer pH 7.4 and mixed. In the second dilution step, an aliquot (20 μl) of the first dilution step was further dispensed in 100 μl phosphate citrate buffer pH 7.4 and mixed. Finally, in the third

dilution step, a sample (20 μl) of the second dilution step was further diluted in 100 μl phosphate citrate buffer pH 7.4 and mixed. All dilutions were performed in 96-well plates. Immediately after the last dilution step the turbidity of the three consecutive dilution steps were measured with a nephelometer. Dilution was done in triplicate for each compound to exclude occasional errors. Based on the turbidity measurements a ranking is performed into 3 classes. Compounds with high solubility obtained a score of 3 and for this compounds the first dilution is clear. Compounds with medium solubility obtained a score of 2. For these compounds the first dilution is unclear and the second dilution is clear. Compounds with low solubility obtained a score of 1 and for these compounds both the first and the second dilution are unclear.

The solubility of 11 compounds was measured. Six compounds obtained a score of 3, two compounds showed a score of 2 and three compounds demonstrated a score of 1 (see table F-2).

Example C.4: Metabolic stability

Sub-cellular tissue preparations were made according to Gorrod et al. (Xenobiotica 5: 453-462, 1975) by centrifugal separation after mechanical homogenization of tissue. Liver tissue was rinsed in ice-cold 0.1 M Tris-HCl (pH 7.4) buffer to wash excess blood. Tissue was then blotted dry, weighed and chopped coarsely using surgical scissors. The tissue pieces were homogenized in 3 volumes of ice-cold 0.1 M phosphate buffer (pH 7.4) using either a Potter-S (Braun, Italy) equipped with a Teflon pestle or a Sorvall Omni-Mix homogeniser, for 7 x 10 sec. In both cases, the vessel was kept in/on ice during the homogenization process.

Tissue homogenates were centrifuged at 9000 x g for 20 minutes at 4 °C using a Sorvall centrifuge or Beckman Ultracentrifuge. The resulting supernatant was stored at -80 °C and is designated 'S9'.

The S9 fraction can be further centrifuged at 100.000 x g for 60 minutes (4 °C) using a Beckman ultracentrifuge. The resulting supernatant was carefully aspirated, aliquoted and designated 'cytosol'. The pellet was re-suspended in 0.1 M phosphate buffer (pH 7.4) in a final volume of 1 ml per 0.5 g original tissue weight and designated 'microsomes'. All sub-cellular fractions were aliquoted, immediately frozen in liquid nitrogen and stored at -80 °C until use.

For the samples to be tested, the incubation mixture contained PBS (0.1M), compound (5 μM), microsomes (1mg/ml) and a NADPH-generating system (0.8 mM glucose-6-phosphate, 0.8 mM magnesium chloride and 0.8 Units of glucose-6-phosphate dehydrogenase). Control samples contained the same material but the microsomes were replaced by heat inactivated (10 min at 95 degrees Celsius) microsomes. Recovery of the compounds in the control samples was always 100%.

The mixtures were preincubated for 5 min at 37 degrees Celsius. The reaction was started at timepoint zero (t = 0) by addition of 0.8 mM NADP and the samples were incubated for 15 min (t = 15). The reaction was terminated by the addition of 2 volumes of DMSO. Then the samples were centrifuged for 10 min at 900 x g and the supernatants were stored at room temperature for no longer as 24 h before analysis. All incubations were performed in duplo. Analysis of the supernatants was performed with LC-MS analysis. Elution of the samples was performed on a Xterra MS C18 (50 x 4.6 mm, 5 μm, Waters, US). An Alliance 2790 (Supplier: Waters, US) HPLC system was used. Elution was with buffer A (25 mM ammoniumacetate (pH 5.2) in $H_2O$/acetonitrile (95/5)), solvent B being acetonitrile and solvent C methanol at a flow rate of 2.4 ml/min. The gradient employed was increasing the organic phase concentration from 0 % over 50 % B and 50 % C in 5 min up to 100 % B in 1 min in a linear fashion and organic phase concentration was kept stationary for an additional 1.5 min. Total injection volume of the samples was 25 μl.

A Quattro (supplier: Micromass, Manchester, UK) triple quadrupole mass spectrometer fitted with and ESI source was used as detector. The source and the desolvation temperature were set at 120 and 350 °C respectively and nitrogen was used as nebuliser and drying gas. Data were acquired in positive scan mode (single ion reaction). Cone voltage was set at 10 V and the dwell time was 1 sec.

Metabolic stability was expressed as % metabolism of the compound after 15 min of incubation in the presence of active microsomes

$$(E(act))\ (\%\ metabolism = 100\ \% - ((\frac{\text{Total Ion Current (TIC) of E(act) at } t = 15}{\text{TIC of E(act) at } t = 0})\ x\ 100).$$

Compounds that had a percentage metabolism less than 20 % were defined as highly metabolic stable. Compound that had a metabolism between 20 and 70 % were defined as intermediately stable and compounds that showed a percentage metabolism higher than 70 were defined as low metabolic stable. Three reference compounds were always included whenever a metabolic stability screening was performed. Verapamil was included as a compound with low metabolic stability (% metabolism = 73 %). Cisapride was included as a compound with medium metabolic stability (% metabolism 45 %) and propanol was included as a compound with intermediate to high metabolic stability (25 % metabolism). These reference compounds were used to validate the metabolic stability assay.

Ten compounds were tested. Six compounds had a percentage metabolism less than 20 % and four compounds had

a percentage metabolism between 20 and 70 %.

Example C.5: p21 induction capacity

The following protocol has been applied to determine the p21 protein expression level in human A2780 ovarian carcinoma cells. The A2780 cells (20000 cells /180 μl) were seeded in 96 microwell plates in RPMI 1640 medium supplemented with 2 mM L-glutamine, 50 μg/ml gentamicin and 10 % fetal calf serum. 24 hours before the lysis of the cells, compounds were added at final concentrations of $10^{-5}$, $10^{-6}$, $10^{-7}$ and $10^{-8}$ M. All compounds tested were dissolved in DMSO and further dilutions were made in culture medium. 24 hours after the addition of the compound, the supernatants were removed from the cells. Cells were washed with 200 μl ice-cold PBS. The wells were aspirated and 30 μl of lysisbuffer (50 mM Tris.HCl (pH 7.6), 150 mM NaCl, 1 % Nonidet p40 and 10 % glycerol) was added. The plates were incubated overnight at -70 °C.
The appropriate number of microtiter wells were removed from the foil pouch and placed into an empty well holder. A working solution (1x) of the Wash Buffer (20x plate wash concentrate: 100 ml 20-fold concentrated solution of PBS and surfactant. Contains 2 % chloroacetamide) was prepared. The lyophilised p21WAF standard was reconstituted with distilled $H_2O$ and further diluted with sample diluent (provided in the kit)
The samples were prepared by diluting them 1:4 in sample diluent. The samples (100 μl) and the p21WAF1 standards (100 μl) were pipetted into the appropriate wells and incubated at room temperature for 2 hours. The wells were washed 3 times with 1x wash buffer and then 100 μl of detector antibody reagent (a solution of biotinylated monoclonal p21WAF1 antibody) was pipetted into each well. The wells were incubated at room temperature for 1 hour and then washed three times with 1x wash buffer. The 400x conjugate (peroxidase streptavidine conjugate: 400-fold concentrated solution) was diluted and 100 μl of the 1x solution was added to the wells. The wells were incubated at room temperature for 30 min and then washed 3 times with 1x wash buffer and 1 time with distilled $H_2O$. Substrate solution (chromogenic substrate) (100 μl) was added to the wells and the wells were incubated for 30 minutes in the dark at room temperature. Stop solution was added to each well in the same order as the previously added substrate solution. The absorbance in each well was measured using a spectrophotometric plate reader at dual wavelengths of 450/595 nm.
For each experiment, controls (containing no drug) and a blank incubation (containing no cells or drugs) were run in parallel. The blank value was substracted from all control and sample values. For each sample, the value for p21WAF1 induction (in absorbance units) was expressed as the percentage of the value for p21WAF1 present in the control. Percentage induction higher than 130 % was defined as significant induction. Eleven compounds were tested in this assay. They all showed significant induction.

Table F-2: Table F-2 lists the results of the compounds that were tested according to example C.1, C.2, and C.3.

| Co. No. | Enzyme activity pIC50 | Cellular activity pIC50 | Solubility Score |
|---|---|---|---|
| 1 | 7.723 | 6.232 | 2 |
| 2 | 8.059 | 5.691 | 1 |
| 3 | 7.647 | 5.62 | 1 |
| 4 | 8.097 | 6.248 | 1 |
| 5 | 7.549 | 5.689 | |
| 6 | 7.421 | 6.384 | 3 |
| 7 | 7.323 | 5.69 | 3 |
| 8 | 7.487 | 7.438 | 3 |
| 9 | 7.609 | <5 | 3 |
| 10 | 7.65 | 5.966 | 3 |
| 11 | 7.553 | <5 | 3 |
| 12 | 7.528 | 6.01 | 2 |
| 13 | 7.361 | 5.773 | |
| 14 | | 6.614 | |

D. Composition example: Film-coated tablets

Preparation of tablet core

A mixture of 100 g of a compound of formula (I), 570 g lactose and 200 g starch is mixed well and thereafter humidified with a solution of 5 g sodium dodecyl sulphate and 10 g polyvinyl-pyrrolidone in about 200 ml of water. The wet powder mixture is sieved, dried and sieved again. Then there is added 100 g microcrystalline cellulose and 15 g hydrogenated vegetable oil. The whole is mixed well and compressed into tablets, giving 10.000 tablets, each comprising 10 mg of a compound of formula (I).

Coating

To a solution of 10 g methyl cellulose in 75 ml of denaturated ethanol there is added a solution of 5 g of ethyl cellulose in 150 ml of dichloromethane. Then there are added 75 ml of dichloromethane and 2.5 ml 1,2,3-propanetriol 10 g of polyethylene glycol is molten and dissolved in 75 ml of dichloromethane. The latter solution is added to the former and then there are added 2.5 g of magnesium octadecanoate, 5 g of polyvinyl-pyrrolidone and 30 ml of concentrated colour suspension and the whole is homogenated. The tablet cores are coated with the thus obtained mixture in a coating apparatus.

## Claims

**1.** A compound of formula (I),

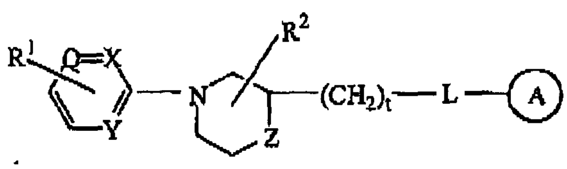

(I)

the *N*-oxide forms, the pharmaceutically acceptable addition salts and the stereochemically isomeric forms thereof, wherein
t is 0, 1, 2, 3 or 4 and when t is 0 then a direct bond is intended;
each Q is nitrogen or

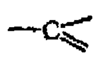

;

each X is nitrogen or

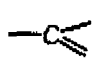

;

each Y is nitrogen or

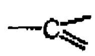

;

each Z is -NH-, -O- or $-CH_2-$;

$R^1$ is $-C(O)NR^3R^4$, $-NHC(O)R^7$, $-C(O)-C_{1-6}$alkanediyl$SR^7$, $-NR^8C(O)N(OH)R^7$, $-NR^8C(O)C_{1-6}$alkanediyl$SR^7$ or $-NR^8C(O)C=N(OH)R^7$

wherein $R^3$ and $R^4$ are each independently selected from hydrogen, hydroxy, $C_{1-6}$alkyl, hydroxy$C_{1-6}$alkyl, amino$C_{1-6}$alkyl or aminoaryl;

$R^7$ is hydrogen, $C_{1-6}$alkyl, $C_{1-6}$alkylcarbonyl, aryl$C_{1-6}$alkyl, $C_{1-6}$alkylpyrazinyl, pyridinone, pyrrolidinone or methylimidazolyl;

$R^8$ is hydrogen or $C_{1-6}$alkyl;

$R^2$ is hydrogen, hydroxy, amino, hydroxy$C_{1-6}$alkyl, $C_{1-6}$alkyl, $C_{1-6}$alkyloxy, aryl$C_{1-6}$alkyl, aminocarbonyl, hydroxycarbonyl, amino$C_{1-6}$alkyl, aminocarbonyl$C_{1-6}$alkyl, hydroxycarbonyl$C_{1-6}$alkyl, hydroxyaminocarbonyl, $C_{1-6}$alkyloxycarbonyl, $C_{1-6}$alkylamino$C_{1-6}$alkyl or di($C_{1-6}$alkyl)amino$C_{1-6}$alkyl;

-L- is a bivalent radical selected from $-NR^9C(O)-$, $-NR^9SO_2-$ or $-NR^9CH_2-$wherein $R^9$ is hydrogen, $C_{1-6}$alkyl, $C_{3-10}$cycloalkyl, hydroxy$C_{1-6}$alkyl, $C_{1-6}$alkyloxy$C_{1-6}$alkyl or di($C_{1-6}$alkyl)amino$C_{1-6}$alkyl;

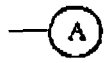

is a radical selected from

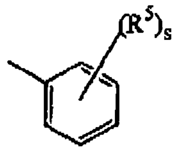
(a-1)

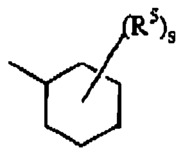
(a-2)

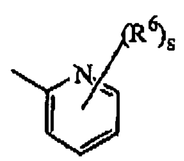
(a-3)

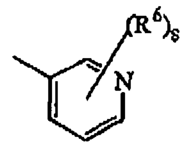
(a-4)

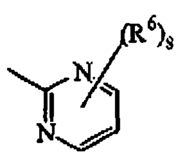
(a-5)

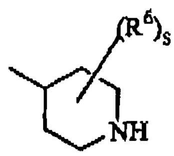
(a-6)

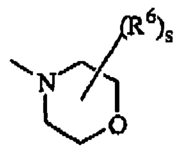
(a-7)

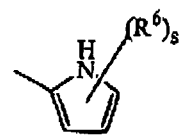
(a-8)

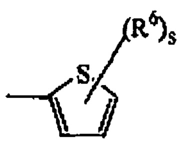
(a-9)

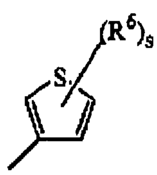
(a-10)

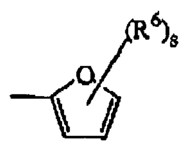
(a-11)

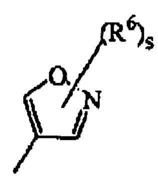
(a-12)

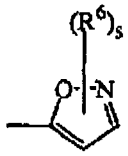
(a-13)

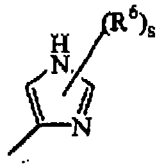
(a-14)

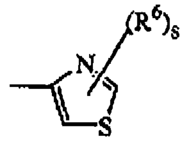
(a-15)

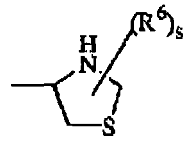
(a-16)

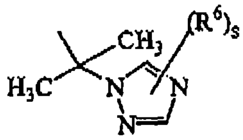
(a-17)

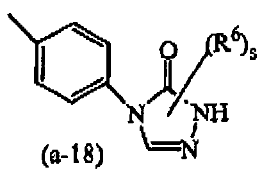
(a-18)

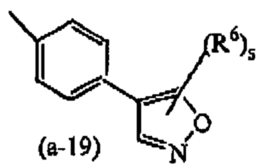
(a-19)

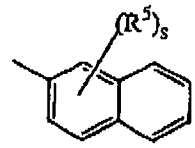
(a-20)

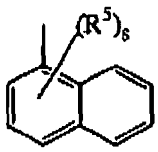
(a-21)

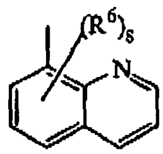
(a-22)

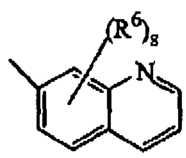
(a-23)

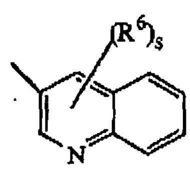
(a-24)

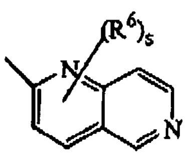
(a-25)

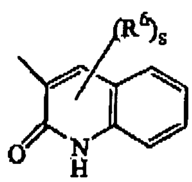
(a-26)

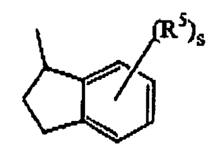
(a-27)

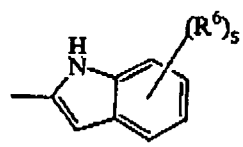
(a-28)

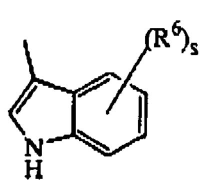
(a-29)

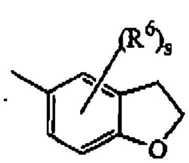
(a-30)

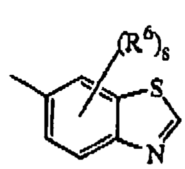
(a-31)

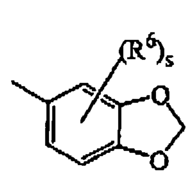
(a-32)

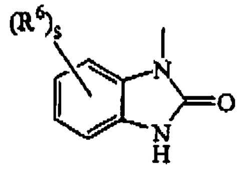

(a-33)

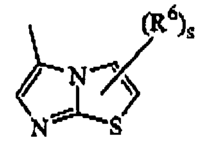

(a-34)

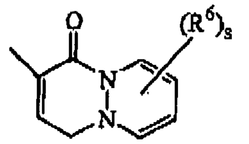

(a-35)

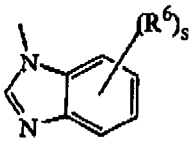

(a-36)

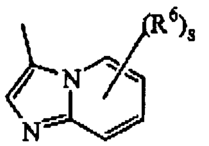

(a-37)

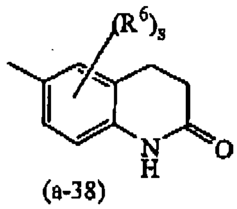

(a-38)

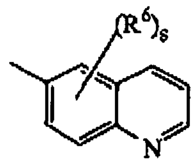

(a-39)

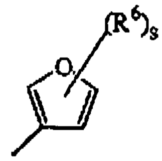

(a-40)

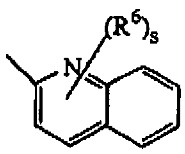

(a-41)

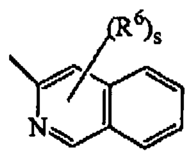

(a-42)

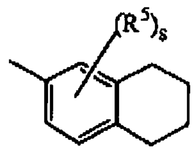

(a-43)

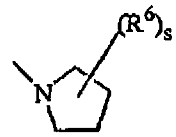

(a-44)

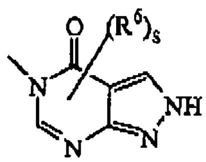

(a-45)

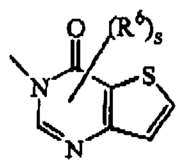

(a-46)

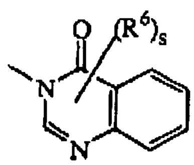

(a-47)

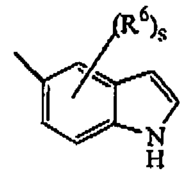

(a-48)

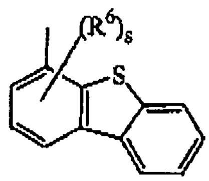

(a-49)

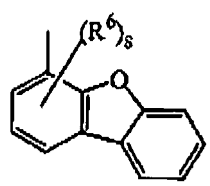

(a-50)

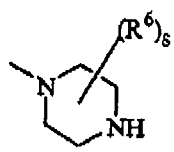

(a-51)

wherein each s is independently 0, 1, 2, 3, 4 or 5;

each $R^5$ and $R^6$ are independently selected from hydrogen; halo; hydroxy; amino; nitro; trihalo$C_{1-6}$alkyl; trihalo$C_{1-6}$alkyloxy; $C_{1-6}$alkyl; $C_{1-6}$alkyl substituted with aryl and $C_{3-10}$cycloalkyl; $C_{1-6}$alkyloxy; $C_{1-6}$alkyloxy $C_{1-6}$alkyloxy; $C_{1-6}$alkylcarbonyl; $C_{1-6}$alkyloxycarbonyl; $C_{1-6}$alkylsulfonyl; cyano$C_{1-6}$alkyl; hydroxy$C_{1-6}$alkyl; hydroxy$C_{1-6}$alkyloxy; hydroxy$C_{1-6}$alkylamino; amino$C_{1-6}$alkyloxy; di($C_{1-6}$alkyl)aminocarbonyl; di(hydroxy$C_{1-6}$alkyl) amino; (aryl)($C_{1-6}$alkyl)amino; di($C_{1-6}$alkyl)amino$C_{1-6}$alkyloxy; di($C_{1-6}$alkyl)amino$C_{1-6}$alkylamino; di($C_{1-6}$alkyl) amino$C_{1-6}$alkylamino$C_{1-6}$alkyl; arylsulfonyl; arylsulfonylamino; aryloxy; aryloxy$C_{1-6}$alkyl; aryl$C_{2-6}$alkenediyl; di ($C_{1-6}$alkyl)amino; di($C_{1-6}$alkyl)amino$C_{1-6}$alkyl; di($C_{1-6}$alkyl)amino($C_{1-6}$alkyl)amino; di($C_{1-6}$alkyl)amino($C_{1-6}$alkyl) amino$C_{1-6}$alkyl; di($C_{1-6}$alkyl)amino$C_{1-6}$alkyl($C_{1-6}$alkyl)amino; di($C_{1-6}$alkyl)amino$C_{1-6}$alkyl($C_{1-6}$alkyl)amino$C_{1-6}$ alkyl; aminosulfonylamino($C_{1-6}$alkyl)amino; aminosulfonylamino($C_{1-6}$alkyl)amino$C_{1-6}$alkyl; di($C_{1-6}$alkyl)aminosulfo-nyl- amino($C_{1-6}$alkyl)amino; di($C_{1-6}$alkyl)aminosulfonylamino($C_{1-6}$alkyl)amino$C_{1-6}$alkyl; cyano; thiophenyl; thiophe-

nyl substituted with di($C_{1-6}$alkyl)amino$C_{1-6}$alkyl($C_{1-6}$alkyl)amino$C_{1-6}$alkyl, di($C_{1-6}$alkyl)amino$C_{1-6}$alkyl, $C_{1-6}$alkylpiperazinyl$C_{1-6}$alkyl, hydroxy$C_{1-6}$alkylpiperazinyl$C_{1-6}$alkyl, hydroxy$C_{1-6}$alkyloxy$C_{1-6}$alkylpiperazinyl$C_{1-6}$ alkyl, di($C_{1-6}$alkyl)aminosulfonylpiperazinyl$C_{1-6}$alkyl, $C_{1-6}$alkyloxypiperidinyl, $C_{1-6}$alkyloxypiperidinyl$C_{1-6}$alkyl, morpho- linyl$C_{1-6}$alkyl, hydroxy$C_{1-6}$alkyl($C_{1-6}$alkyl)amino$C_{1-6}$alkyl, or di(hydroxy$C_{1-6}$alkyl)amino$C_{1-6}$alkyl; furanyl; furanyl substituted with hydroxy$C_{1-6}$alkyl; benzofuranyl; imidazolyl; oxazolyl; oxazolyl substituted with aryl and $C_{1-6}$alkyl; $C_{1-6}$alkyltriazolyl; tetrazolyl; pyrrolidinyl; pyrrolyl; piperidinyl$C_{1-6}$alkyloxy; morpholinyl; $C_{1-6}$alkylmorpholinyl; morpholinyl$C_{1-6}$alkyloxy; morpholinyl$C_{1-6}$alkyl; morpholinyl$C_{1-6}$alkylamino; morpholinyl$C_{1-6}$alkylamino$C_{1-6}$alkyl; piperazinyl; $C_{1-6}$alkylpiperazinyl; $C_{1-6}$alkylpiperazinyl$C_{1-6}$alkyloxy; piperazinyl$C_{1-6}$alkyl; naphtalenylsulfonylpiperazinyl; naphtalenylsulfonylpiperidinyl; naphtalenylsulfonyl; $C_{1-6}$alkylpiperazinyl$C_{1-6}$alkyl; $C_{1-6}$alkylpiperazinyl$C_{1-6}$ alkylamino; $C_{1-6}$alkylpiperazinyl$C_{1-6}$alkylamino$C_{1-6}$alkyl; $C_{1-6}$alkylpiperazinylsulfonyl; amínosulfonylpiperazinyl $C_{1-6}$alkyloxy; aminosulfonylpiperazinyl; aminosulfonylpiperazinyl$C_{1-6}$alkyl-, di($C_{1-6}$alkyl)aminosulfonylpiperazinyl; di($C_{1-6}$alkyl)aminosulfonylpiperazinyl$C_{1-6}$alkyl; hydroxy$C_{1-6}$alkylpiperazinyl; hydroxy$C_{1-6}$alkylpiperazinyl$C_{1-6}$alkyl; $C_{1-6}$alkyloxypiperidinyl; $C_{1-6}$alkyloxypiperidinyl$C_{1-6}$alkyl; piperidinylamino$C_{1-6}$alkylamino; piperidinylamino $C_{1-6}$alkylamino$C_{1-6}$alkyl; ($C_{1-6}$alkylpiperidinyl)(hydroxy$C_{1-6}$alkyl)amino$C_{1-6}$alkylamino; ($C_{1-6}$alkylpiperidinyl) (hydroxy$C_{1-6}$alkyl)amino$C_{1-6}$alkylamino$C_{1-6}$alkyl; hydroxy$C_{1-6}$alkyloxy$C_{1-6}$alkylpiperazinyl; hydroxy$C_{1-6}$alkyloxy $C_{1-6}$alkylpiperazinyl$C_{1-6}$alkyl; (hydroxy$C_{1-6}$alkyl)($C_{1-6}$alkyl)amino; (hydroxy$C_{1-6}$alkyl)($C_{1-6}$alkyl)amino$C_{1-6}$alkyl; hydroxy$C_{1-6}$alkylamino $C_{1-6}$alkyl; di(hydroxy$C_{1-6}$alkyl)amino$C_{1-6}$alkyl; pyrrolidinyl$C_{1-6}$alkyl; pyrrolidinyl$C_{1-6}$alkyloxy; pyrazolyl; thiopyrazolyl; pyrazolyl substituted with two substituents selected from $C_{1-6}$alkyl or trihalo$C_{1-6}$alkyl; pyridinyl; pyridinyl substituted with $C_{1-6}$alkyloxy, aryloxy or aryl; pyrimidinyl; tetrahydropyrimidinylpiperazinyl; tetrahydropyrimidinyl- piperazinyl$C_{1-6}$alkyl; quinolinyl; indole; phenyl; phenyl substituted with one, two or three substituents independently selected from halo, amino, nitro, $C_{1-6}$alkyl, $C_{1-6}$alkyloxy, hydroxy$C_{1-4}$alkyl, trifluoromethyl, trifluoromethyloxy, hydroxy$C_{1-4}$alkyloxy, $C_{1-4}$alkylsulfonyl, $C_{1-4}$alkyloxy$C_{1-4}$alkyloxy, $C_{1-4}$alkyloxycarbonyl, amina$C_{1-4}$ alkyloxy, di($C_{1-4}$alkyl)amino$C_{1-4}$alkyloxy, di($C_{1-4}$alkyl)amino, di($C_{1-4}$alkyl)aminocarbonyl, di($C_{1-4}$alkyl) amino$C_{1-4}$alkyl, di($C_{1-4}$alkyl)amino$C_{1-4}$alkylamino$C_{1-4}$alkyl, di($C_{1-4}$alkyl)amino($C_{1-4}$alkyl)amino, di($C_{1-4}$alkyl)amino ($C_{1-4}$alkyl)amino $C_{1-4}$alkyl, di($C_{1-4}$alkyl)amino$C_{1-4}$alkyl($C_{1-4}$alkyl)amino, di($C_{1-4}$alkyl)amino$C_{1-4}$alkyl($C_{1-4}$alkyl) amino$C_{1-4}$alkyl, aminosulfonylamino($C_{1-4}$alkyl)amino, aminosulfonylamino($C_{1-4}$alkyl)amino$C_{1-4}$alkyl, di($C_{1-4}$alkyl) aminosulfonylamino($C_{1-4}$alkyl)amino, di($C_{1-4}$alkyl)aminosulfonylamino($C_{1-4}$alkyl)amino$C_{1-6}$alkyl, cyano, piperidinyl$C_{1-4}$alkyloxy, pyrrolidinyl$C_{1-4}$alkyloxy, aminosulfonylpiperazinyl, aminosulfonylpiperazinyl$C_{1-4}$alkyl, di ($C_{1-4}$alkyl)aminosulfonylpiperazinyl, di($C_{1-4}$alkyl)aminosulfonylpiperazinyl$C_{1-4}$alkyl, hydroxy$C_{1-4}$alkylpiperazinyl, hydroxy$C_{1-4}$alkylpiperazinyl$C_{1-4}$alkyl, $C_{1-4}$alkyloxypiperidinyl, $C_{1-4}$alkyloxypiperidinyl$C_{1-4}$alkyl, hydroxy$C_{1-4}$alkyloxy $C_{1-4}$alkylpiperazinyl, hydroxy$C_{1-4}$alkyloxy$C_{1-4}$alkylpiperazinyl$C_{1-4}$alkyl, (hydroxy$C_{1-4}$alkyl)($C_{1-4}$alkyl)amino, (hydroxy $C_{1-4}$alkyl)($C_{1-4}$alkyl)amino$C_{1-4}$alkyl, hydroxy$C_{1-4}$alkylamino$C_{1-4}$alkyl, di(hydroxy$C_{1-4}$alkyl)amino$C_{1-4}$alkyl, furanyl, furanyl substituted with-CH=CH-CH=CH-, pyrrolidinyl$C_{1-4}$alkyl, pyrrolidinyl$C_{1-4}$alkyloxy, morpholinyl, morpholinyl$C_{1-4}$alkyloxy, morpholinyl$C_{1-4}$alkyl, morpbolinyl$C_{1-4}$alkylamino, morpholinyl$C_{1-4}$alkylamino$C_{1-4}$alkyl, piperazinyl, $C_{1-4}$alkylpiperazinyl, $C_{1-4}$alkylpiperazinyl$C_{1-4}$alkyloxy, piperazinyl$C_{1-4}$alkyl, $C_{1-4}$alkylpiperazinyl $C_{1-4}$alkyl, $C_{1-4}$alkylpiperazinyl $C_{1-4}$alkylamino, $C_{1-4}$alkylpiperazinyl$C_{1-4}$alkylamino$C_{1-6}$alkyl, pyrimidinylpiperazinyl, pyrimidinylpiperazinyl $C_{1-4}$alkyl, piperidinylamino$C_{1-4}$alkylamino, piperidinylamino$C_{1-4}$alkylamino$C_{1-4}$alkyl, ($C_{1-4}$alkylpiperidinyl)(hydroxy$C_{1-4}$alkyl)amino$C_{1-4}$alkyl amino, ($C_{1-4}$alkylpiperidinyl)(hydroxy$C_{1-4}$alkyl)amino$C_{1-4}$ alkylamino$C_{1-4}$alkyl, pyridinyl$C_{1-4}$alkyloxy, hydroxy$C_{1-4}$alkylamino, di(hydroxy$C_{1-4}$alkyl)amino, di($C_{1-4}$alkyl) amino$C_{1-4}$alkylamino, aminothiadiazolyl, aminosulfonylpiperazinyl$C_{1-4}$alkyloxy, or thiophenyl$C_{1-4}$alkylamino;
each $R^5$ and $R^6$ can be placed on the nitrogen in replacement of the hydrogen;
aryl in the above is phenyl, or phenyl substituted with one or more substituents each independently selected from halo, $C_{1-6}$alkyl, $C_{1-6}$alkyloxy, trifluoromethyl, cyano or hydroxycarbonyl.

**2.** A compound as claimed in claim 1 wherein

$R^3$ and $R^4$ are each independently selected from hydrogen, hydroxy, hydroxy$C_{1-6}$alkyl, amino$C_{1-6}$alkyl or aminoaryl;

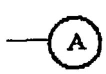

is a radical selected from (a-1), (a-2), (a-3), (a-4), (a-5), (a-6), (a-7), (a-8), (a-9), (a-10), (a-11), (a-12), (a-13), (a-14), (a-15), (a-16), (a-17), (a-18), (a-19), (a-20), (a-21), (a-22), (a-23), (a-24), (a-25), (a-26), (a-27), (a-28), (a-29), (a-30), (a-31), (a-32), (a-33), (a-34), (a-35), (a-36), (a-37), (a-38), (a-39), (a-40), (a-41), (a-42) (a-43) or (a-44);
each $R^5$ and $R^6$ are independently selected from hydrogen; halo; hydroxy; amino; nitro; trihalo$C_{1-6}$alkyl; trihalo$C_{1-6}$alkyloxy; $C_{1-6}$alkyl; $C_{1-6}$alkyloxy; $C_{1-6}$alkyloxy$C_{1-6}$alkyloxy; $C_{1-6}$alkylcarbonyl; $C_{1-6}$alkylsulfonyl;

cyano$C_{1-6}$ alkyl; hydroxy$C_{1-6}$alkyl; hydroxy$C_{1-6}$alkyloxy; hydroxy$C_{1-6}$alkylamino; amino$C_{1-6}$alkyloxy; di($C_{1-6}$alkyl)aminocarbonyl; di(hydroxy$C_{1-6}$alkyl)amino; aryl$C_{1-6}$alkyl)amino; di($C_{1-6}$alkyl)amino$C_{1-6}$alkyloxy; di($C_{1-6}$alkyl)amino$C_{1-6}$alkylamino; arylsulfonyl; arylsulfonylamino; aryloxy; aryl$C_{2-6}$alkenediyl; di($C_{1-6}$alkyl)amino; di($C_{1-6}$alkyl)amino$C_{1-6}$alkyl; di($C_{1-6}$alkyl)amino$C_{1-6}$alkyl($C_{1-6}$alkyl)amino$C_{1-6}$alkyl; cyano; thiophenyl; thiophenyl substituted with di($C_{1-6}$alkyl)amino$C_{1-6}$alkyl($C_{1-6}$alkyl)amino$C_{1-6}$alkyl, di($C_{1-6}$alkyl)amino$C_{1-6}$alkyl, $C_{1-6}$alkylpiperazinyl$C_{1-6}$alkyl or di(hydroxy$C_{1-6}$alkyl)amino$C_{1-6}$alkyl; furanyl; imidazolyl; $C_{1-6}$alkyltriazolyl; tetrazolyl; pyrrolidinyl; piperidinyl$C_{1-6}$alkyloxy; morpholinyl; $C_{1-6}$alkylmorpholinyl; morpholinyl$C_{1-6}$alkyloxy; morpholinyl$C_{1-6}$alkyl; $C_{1-6}$alkylpiperazinyl; $C_{1-6}$alkylpiperazinyl$C_{1-6}$alkyloxy; $C_{1-6}$alkylpiperazinyl$C_{1-6}$alkyl; $C_{1-6}$alkylpiperazinylsulfonyl; aminosulfonylpiperazinyl$C_{1-6}$alkyloxy; aminosulfonylpiperazinyl; aminosulfonylpiperazinyl$C_{1-6}$alkyl; di($C_{1-6}$alkyl)aminosulfonylpiperazinyl; di($C_{1-6}$alkyl)aminosulfonylpiperazinyl$C_{1-6}$alkyl; hydroxy$C_{1-6}$alkylpiperazinyl; hydroxy$C_{1-6}$alkylpiperazinyl$C_{1-6}$alkyl; $C_{1-6}$alkyloxypiperidinyl; $C_{1-6}$alkyloxypiperidinyl$C_{1-6}$alkyl; hydroxy$C_{1-6}$alkyloxy$C_{1-6}$alkylpiperazinyl; hydroxy$C_{1-6}$alkyloxy$C_{1-6}$alkylpiperazinyl$C_{1-6}$alkyl; (hydroxy$C_{1-6}$alkyl)($C_{1-6}$alkyl)amino; (hydroxy$C_{1-6}$alkyl)($C_{1-6}$alkyl)amino$C_{1-6}$alkyl; pyrrolidinyl$C_{1-6}$alkyloxy; pyrazolyl; thiopyrazolyl; pyrazolyl substituted with two substituents selected from $C_{1-6}$alkyl or trihalo$C_{1-6}$alkyl; pyridinyl; pyridinyl substituted with $C_{1-6}$alkyloxy or aryl; pyrimidinyl; quinolinyl; indole; phenyl; phenyl substituted with one, two or three substituents independently selected from halo, amino, $C_{1-6}$alkyl, $C_{1-6}$alkyloxy, hydroxy$C_{1-4}$alkyl, trifluoromethyl, trifluoromethyloxy, hydroxy$C_{1-4}$alkyloxy, $C_{1-4}$alkyloxy$C_{1-4}$alkyloxy, amino$C_{1-4}$alkyloxy, di($C_{1-4}$alkyl)amino$C_{1-4}$alkyloxy, di($C_{1-4}$alkyl)amino, di($C_{1-4}$alkyl)amino$C_{1-4}$alkyl, di($C_{1-4}$alkyl)amino$C_{1-4}$alkyl($C_{1-4}$alkyl)amino$C_{1-4}$alkyl, piperidinyl$C_{1-4}$alkyloxy, pyrrolidinyl$C_{1-4}$alkyloxy, aminosulfonylpiperazinyl, aminosulfonylpiperazinyl$C_{1-4}$alkyl, di($C_{1-4}$alkyl)aminosulfonylpiperazinyl, di($C_{1-4}$alkyl)aminosulfonylpiperazinyl$C_{1-4}$alkyl, hydroxy$C_{1-4}$alkylpiperazinyl, hydroxy$C_{1-4}$alkylpiperazinyl$C_{1-4}$alkyl, $C_{1-4}$alkyloxypiperidinyl, $C_{1-4}$alkyloxypiperidinyl$C_{1-4}$alkyl, hydroxy$C_{1-4}$alkyloxy$C_{1-4}$alkylpiperazinyl, hydroxy$C_{1-4}$alkyloxy$C_{1-4}$alkylpiperazinyl$C_{1-4}$alkyl, (hydroxy$C_{1-4}$alkyl)($C_{1-4}$alkyl)amino, (hydroxy$C_{1-4}$alkyl)($C_{1-4}$alkyl)amino$C_{1-4}$alkyl, pyrrolidinyl$C_{1-4}$alkyloxy, morpholinyl$C_{1-4}$alkyloxy, morpholinyl$C_{1-4}$alkyl, $C_{1-4}$alkylpiperazinyl, $C_{1-4}$alkylpiperazinyl$C_{1-4}$alkyloxy, $C_{1-4}$alkylpiperazinyl$C_{1-4}$alkyl, hydroxy$C_{1-4}$alkylamino, di(hydroxy$C_{1-4}$alkyl)amino, di($C_{1-4}$alkyl)amino$C_{1-4}$alkylamino, aminothiadiazolyl, aminosulfonylpiperazinyl$C_{1-4}$alkyloxy, or thiophenyl$C_{1-4}$alkylamino.

**3.** A compound as claimed in claim 1 wherein t is 0;

$R^1$ is -C(O)N$R^3R^4$, -C(O)-$C_{1-6}$alkanediylS$R^7$, -N$R^8$C(O)N(OH)$R^7$, -N$R^8$C(O)$C_{1-6}$alkanediylS$R^7$ or -N$R^8$C(O)C=N(OH)$R^7$ wherein $R^3$ and $R^4$ are each independently selected from hydrogen, hydroxy, hydroxy$C_{1-6}$alkyl or amino$C_{1-6}$alkyl; $R^2$ is hydrogen, hydroxy, amino, hydroxy$C_{1-6}$alkyl, $C_{1-6}$alkyl, $C_{1-6}$alkyloxy, aryl$C_{1-6}$alkyl, aminocarbonyl, amino$C_{1-6}$alkyl, $C_{1-6}$alkylamino$C_{1-6}$alkyl or di($C_{1-6}$alkyl)amino$C_{1-6}$alkyl; -L- is a bivalent radical selected from -NHC(O)- or -NH$SO_2$-;

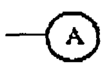

is a radical selected from (a-1), (a-3), (a-4), (a-5), (a-6), (a-7), (a-8), (a-9), (a-10), (a-11), (a-12), (a-13), (a-14), (a-15), (a-16), (a-17), (a-18), (a-19), (a-20), (a-21), (a-22), (a-23), (a-24), (a-25), (a-26), (a-28), (a-29), (a-30), (a-31), (a-32), (a-33), (a-34), (a-35), (a-36), (a-37), (a-38), (a-39), (a-40), (a-41), (a-42), (a-44), (a-45), (a-46), (a-47), (a-48) or (a-51);
each s is independently 0, 1, 2, 3 or 4;

$R^5$ is hydrogen; halo; hydroxy; amino; nitro; trihalo$C_{1-6}$alkyl; trihalo$C_{1-6}$alkyloxy; $C_{1-6}$alkyl; $C_{1-6}$alkyloxy; $C_{1-6}$alkylcarbonyl; $C_{1-6}$alkyloxycarbonyl; $C_{1-6}$alkylsulfonyl; hydroxy$C_{1-6}$alkyl; aryloxy; di($C_{1-6}$alkyl)amino; cyano; thiophenyl; furanyl; furanyl substituted with hydroxy$C_{1-6}$alkyl; benzofuranyl; imidazolyl; oxazolyl; oxazolyl substituted with aryl and $C_{1-6}$alkyl; $C_{1-6}$alkyltriazolyl; tetrazolyl; pyrrolidinyl; pyrrolyl; morpholinyl; $C_{1-6}$alkylmorpholinyl; piperazinyl; $C_{1-6}$alkylpiperazinyl; hydroxy$C_{1-6}$alkylpiperazinyl; $C_{1-6}$alkyloxypiperidinyl; pyrazoly; pyrazolyl substituted with one or two substituents selected from $C_{1-6}$alkyl or trihalo$C_{1-6}$alkyl; pyridinyl; pyridinyl substituted with $C_{1-6}$alkyloxy, aryloxy or aryl; pyrimidinyl; quinolinyl; indole; phenyl; or phenyl substituted with one or two substituents independently selected from halo, $C_{1-6}$alkyl, $C_{1-6}$alkyloxy or trifluoromethyl;
and $R^6$ is hydrogen; halo; hydroxy; amino; nitro; trihalo$C_{1-6}$alkyl; trihalo$C_{1-6}$alkyloxy; $C_{1-6}$alkyl; $C_{1-6}$alkyloxy; $C_{1-6}$alkylcarbonyl; $C_{1-6}$alkyloxycarbonyl; $C_{1-6}$alkylsulfonyl; hydroxy$C_{1-6}$alkyl; aryloxy; di($C_{1-6}$alkyl)amino; cyano; pyridinyl; phenyl; or phenyl substituted with one or two substituents independently selected from halo, $C_{1-6}$alkyl, $C_{1-6}$alkyloxy or trifluoromethyl.

**4.** A compound as claimed in claim 1 and 2 wherein t is 0 or 1; each Q is

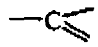 ;

each X is nitrogen; $R^1$ is -C(O)NH(OH); $R^2$ is hydrogen, hydroxy, $C_{1-6}$alkyl, or aryl$C_{1-6}$alkyl; -L- is a bivalent radical selected from NHC(O)- or $-NHSO_2-$;

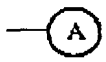

is a radical selected from (a-1) or (a-20); each s is independently 0 or 1; and each $R^5$ is independently selected from hydrogen or phenyl.

**5.** A compound as claimed in claim 1, 2 and 4 wherein t is 1; each Q is

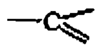 ;

each X is nitrogen; each Y is nitrogen; each Z is -O- or $-CH_2-$; $R^1$ is -C(O)NH(OH); $R^2$ is hydrogen; -L- is a bivalent radical selected from -NHC(O)- or $-NHSO_2-$;

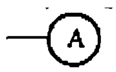

is a radical selected from (a-1) or (a-20); each s is independently 0 or 1; and each $R^5$ is independently selected from hydrogen or phenyl.

**6.** A compound according to claim 1, 2, 4 and 5 selected from compounds No 4, No 10, No 8, No 6, No 1, No 12 and No 14.

| | |
|---|---|
| 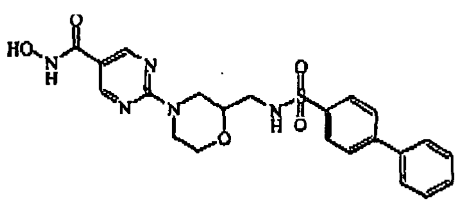 | 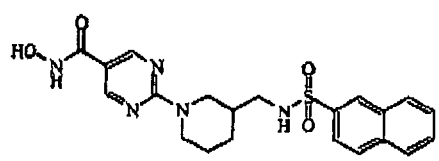 |
| Co. No. 4 | Co. No. 10 |
| 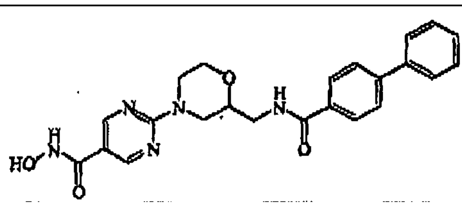 | 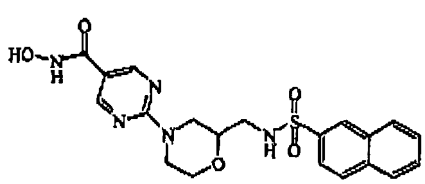 |
| Co. No. 8 | Co. No. 6 |

(continued)

| | |
|---|---|
| 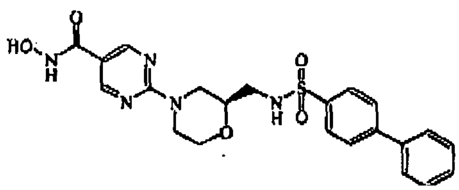 | 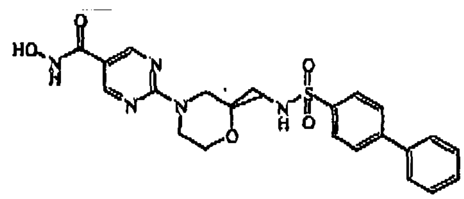 |
| (B); Co. No. 1 | (A); Co. No. 12 |
| 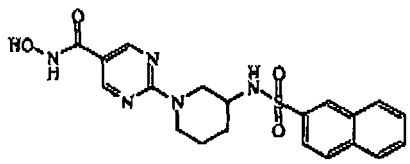 | |
| Co. No. 14 | |

**7.** A pharmaceutical composition comprising pharmaceutically acceptable carriers and as an active ingredient a therapeutically effective amount of a compound as claimed in claim 1 to 6.

**8.** A process of preparing a pharmaceutical composition as claimed in claim 7 wherein the pharmaceutically acceptable carriers and a compound as claimed in claim 1 to 6 are intimately mixed.

**9.** A compound as claimed in any of claims 1 to 6 for use as a medicine.

**10.** Use of a compound as claimed in any of claims 1 to 6 for the manufacture of a medicament for the treatment of proliferative diseases.

**11.** A process for preparing a compound as claimed in claim 1, **characterized by** reacting an intermediate of formula (II) with an appropriate acid, such as for example, trifluoro acetic acid, yielding a hydroxamic acid of formula (I-a), wherein $R^1$ is -C(O)NH(OH).

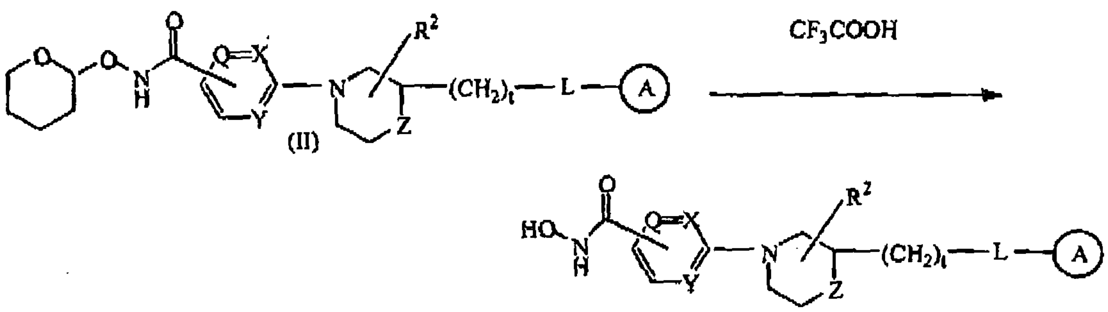


(II)

(I-a)

**12.** A method of detecting or identifying a HDAC in a biological sample comprising detecting or measuring the formation of a complex between a labelled compound as defined in claim 1 and a HDAC.

**13.** A combination of an anti-cancer agents and a HDAC inhibitor as claimed in any of claims 1 to 6.

## Patentansprüche

**1.** Verbindungen der Formel (I),

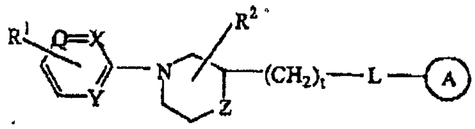 (I)

deren *N*-Oxidformen, deren pharmazeutisch unbedenkliche Additionssalze und deren stereochemisch isomere Formen, wobei
t für 0, 1, 2, 3 oder 4 steht und, wenn t für 0 steht, dies eine direkte Bindung ausdrücken soll;

Q jeweils für Stickstoff oder

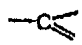

steht;
X jeweils für Stickstoff oder

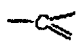

steht;
Y jeweils für Stickstoff oder

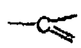

steht;
Z jeweils für -NH-, -O- oder $-CH_2-$ steht;
$R^1$ für $-C(O)NR^3R^4$, $-NHC(O)R^7$, $-C(O)C_{1-6}$-Alkandiyl-$SR^7$, $-NR^8C$ (O) N (OH) $R^7$, $-NR^8C$ $(O)C_{1-6}$-Alkandiyl-$SR^7$ oder $-NR^8C(O)C=N(OH)R^7$ steht,
wobei $R^3$ und $R^4$ jeweils unabhängig voneinander ausgewählt sind aus Wasserstoff, Hydroxy, $C_{1-6}$-Alkyl, Hydroxy-$C_{1-6}$-alkyl, Amino-$C_{1-6}$-alkyl oder Aminoaryl;
$R^7$ für Wasserstoff, $C_{1-6}$-Alkyl, $C_{1-6}$-Alkylcarbonyl, Aryl-$C_{1-6}$-alkyl, $C_{1-6}$-Alkylpyrazinyl, Pyridinon, Pyrrolidinon oder Methylimidazolyl steht; $R^8$ für Wasserstoff oder $C_{1-6}$-Alkyl steht;
$R^2$ für Wasserstoff, Hydroxy, Amino, Hydroxy-$C_{1-6}$-alkyl, $C_{1-6}$-Alkyl, $C_{1-6}$-Alkyloxy, Aryl-$C_{1-6}$-alkyl, Aminocarbonyl, Hydroxycarbonyl, Amino-$C_{1-6}$-alkyl, Aminocarbonyl-$C_{1-6}$-alkyl, Hydroxycarbonyl-$C_{1-6}$-alkyl, Hydroxyaminocarbonyl, $C_{1-6}$-Alkyloxycarbonyl, $C_{1-6}$-Alkylamino-$C_{1-6}$-alkyl oder Di($C_{1-6}$-alkyl)amino-$C_{1-6}$-alkyl steht;
-L- für einen zweiwertigen Rest ausgewählt aus $-NR^9C(O)-$, $-NR^9SO_2-$ oder $-NR^9CH_2-$ steht,
wobei $R^9$ für Wasserstoff, $C_{1-6}$-Alkyl, $C_{3-10}$-Cycloalkyl, Hydroxy-$C_{1-6}$-alkyl, $C_{1-6}$-Alkyloxy-$C_{1-6}$-alkyl oder Di ($C_{1-6}$-alkyl)amino-$C_{1-6}$-alkyl steht;

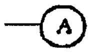

für einen Rest ausgewählt aus

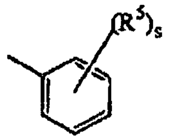
(a-1)

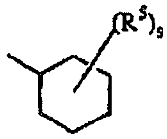
(a-2)

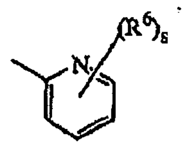
(a-3)

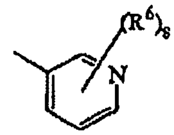
(a-4)

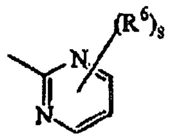
(a-5)

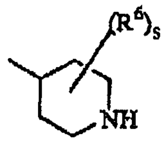
(a-6)

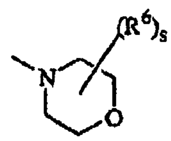
(a-7)

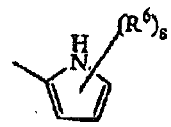
(a-8)

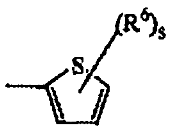
(a-9)

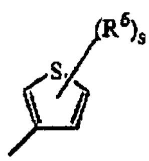
(a-10)

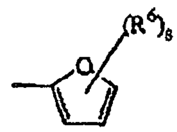
(a-11)

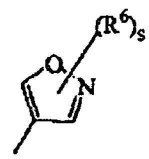
(a-12)

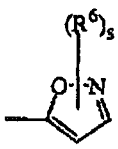
(a-13)

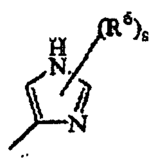
(a-14)

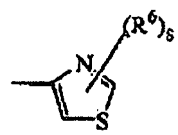
(a-15)

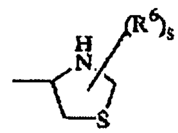
(a-16)

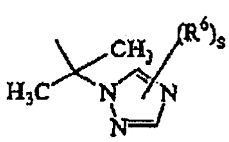
(a-17)

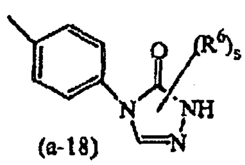
(a-18)

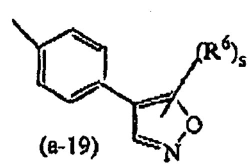
(a-19)

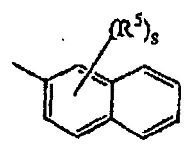
(a-20)

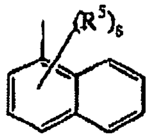
(a-21)

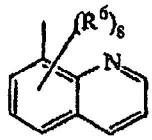
(a-22)

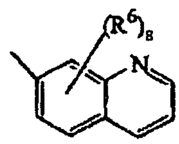
(a-23)

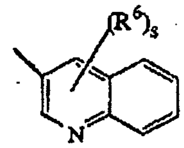
(a-24)

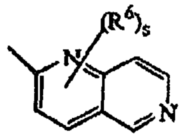
(a-25)

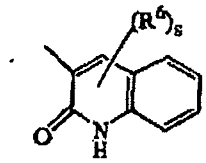
(a-26)

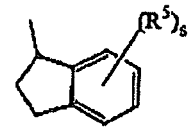
(a-27)

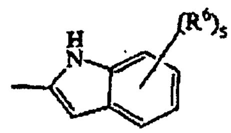
(a-28)

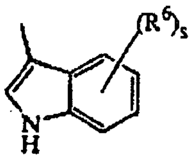
(a-29)

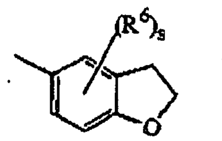
(a-30)

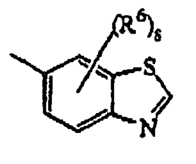
(a-31)

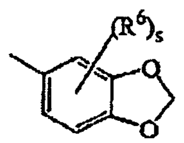
(a-32)

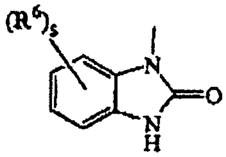
(a-33)

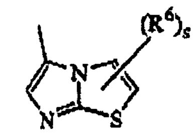
(a-34)

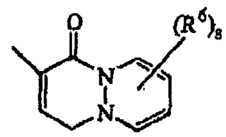
(a-35)

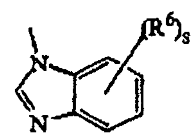
(a-36)

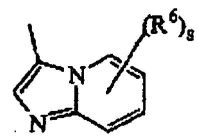
(a-37)

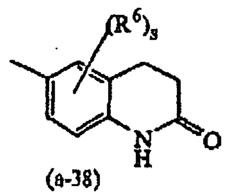
(a-38)

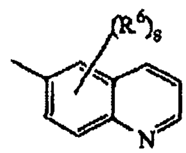
(a-39)

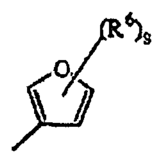
(a-40)

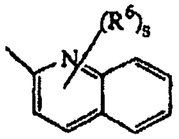
(a-41)

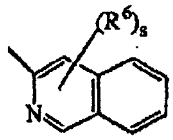
(a-42)

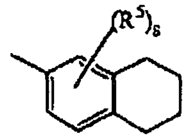
(a-43)

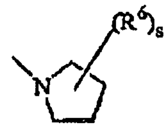
(a-44)

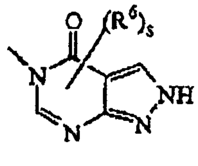

(a-45)

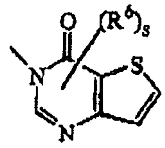

(a-46)

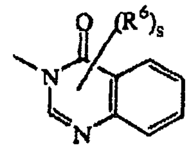

(a-47)

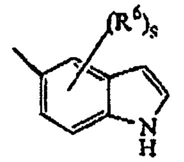

(a-48)

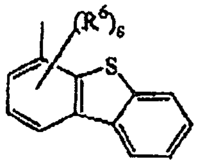

(a-49)

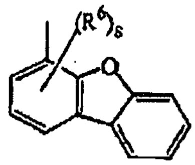

(a-50)

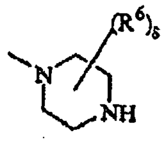

(a-51)

steht,

wobei s jeweils unabhängig für 0, 1, 2, 3, 4 oder 5 steht;

$R^5$ und $R^6$ jeweils unabhängig voneinander aus Wasserstoff; Halogen; Hydroxy; Amino; Nitro; Trihalogen-$C_{1-6}$-alkyl; Trihalogen-$C_{1-6}$-alkyloxy; $C_{1-6}$-Alkyl; $C_{1-6}$-Alkyl substituiert durch Aryl und $C_{3-10}$-Cycloalkyl; $C_{1-6}$-Alkyloxy; $C_{1-6}$-Alkyloxy-$C_{1-6}$-alkyloxy; $C_{1-6}$-Alkylcarbonyl; $C_{1-6}$-Alkyloxycarbonyl; $C_{1-6}$-Alkylsulfonyl; Cyano-$C_{1-6}$-alkyl; Hydroxy-$C_{1-6}$-alkyl; Hydroxy-$C_{1-6}$-alkyloxy; Hydroxy-$C_{1-6}$-alkyl-amino; Amino-$C_{1-6}$-alkyloxy; Di($C_{1-6}$-alkyl)aminocarbonyl; Di (hydroxy-$C_{1-6}$-alkyl)amino; (Aryl) ($C_{1-6}$-alkyl)amino; Di($C_{1-6}$-alkyl)amino-$C_{1-6}$-alkyloxy; Di ($C_{1-6}$-alkyl)amino-$C_{1-6}$-alkylamino; Di ($C_{1-6}$-alkyl)amino-$C_{1-6}$-alkylamino-$C_{1-6}$-alkyl; Arylsulfonyl; Arylsulfonylamino; Aryloxy; Aryloxy- $C_{1-6}$-alkyl; Aryl-$C_{2-6}$-alkendiyl; Di($C_{1-6}$-alkyl)amino; Di($C_{1-6}$-alkyl)amino-$C_{1-6}$-alkyl; Di($C_{1-6}$-alkyl)amino-($C_{1-6}$-alkyl)amino; Di($C_{1-6}$-alkyl)amino-($C_{1-6}$-alkyl)amino-$C_{1-6}$-alkyl; Di($C_{1-6}$-alkyl)amino-$C_{1-6}$-alkyl-($C_{1-6}$-alkyl)amino; Di($C_{1-6}$-alkyl) amino-$C_{1-6}$-alkyl-($C_{1-6}$-alkyl)amino-$C_{1-6}$-alkyl; Aminosulfonyl-amino-($C_{1-6}$-alkyl) amino; Aminosulfonylamino-($C_{1-6}$-alkyl)amino-$C_{1-6}$-alkyl; Di($C_{1-6}$-alkyl)aminosulfonylamino-($C_{1-6}$-alkyl)amino; Di($C_{1-6}$-alkyl)aminosulfonylamino-($C_{1-6}$-alkyl)amino-$C_{1-6}$-alkyl; Cyano; Thiophenyl; Thiophenyl substituiert durch Di($C_{1-6}$-alkyl)amino-$C_{1-6}$-alkyl-($C_{1-6}$-alkyl)amino-$C_{1-6}$-alkyl, Di($C_{1-6}$-alkyl)amino-$C_{1-6}$-alkyl, $C_{1-6}$-Alkylpiperazinyl-$C_{1-6}$-alkyl, Hydroxy-$C_{1-6}$-alkylpiperazinyl-$C_{1-6}$-alkyl, Hydroxy-$C_{1-6}$-alkyloxy-$C_{1-6}$-alkylpiperazinyl-$C_{1-6}$-alkyl, Di($C_{1-6}$-alkyl)aminosulfonylpiperazinyl-$C_{1-6}$-alkyl, $C_{1-6}$-Alkyloxypiperidinyl, $C_{1-6}$-Alkyloxypiperidinyl-$C_{1-6}$-alkyl, Morpholinyl-$C_{1-6}$-alkyl, Hydroxy-$C_{1-6}$-alkyl-($C_{1-6}$-alkyl)amino-$C_{1-6}$-alkyl, oder Di(hydroxy-$C_{1-6}$-alkyl)amino-$C_{1-6}$-alkyl; Furanyl; Furanyl substituiert durch Hydroxy-$C_{1-6}$-alkyl; Benzofuranyl; Imidazolyl; Oxazolyl; Oxazolyl substituiert durch Aryl und $C_{1-6}$-Alkyl; $C_{1-6}$-Alkyltriazolyl; Tetrazolyl; Pyrrolidinyl; Pyrrolyl; Piperidinyl-$C_{1-6}$-alkyloxy; Morpholinyl; $C_{1-6}$-Alkylmorpholinyl; Morpholinyl-$C_{1-6}$-alkyloxy; Morpholinyl-$C_{1-6}$-alkyl; Morpholinyl-$C_{1-6}$-alkylamino; Morpholinyl-$C_{1-6}$-alkylamino-$C_{1-6}$-alkyl; Piperazinyl; $C_{1-6}$-Alkylpiperazinyl; $C_{1-6}$-Alkylpiperazinyl-$C_{1-6}$-alkyloxy; Piperazinyl-$C_{1-6}$-alkyl; Naphthalinylsulfonylpiperazinyl; Naphthalinylsulfonylpiperidinyl; Naphthalinylsulfonyl; $C_{1-6}$-Alkylpiperazinyl-$C_{1-6}$-alkyl; $C_{1-6}$-Alkylpiperazinyl-$C_{1-6}$-alkylamino; $C_{1-6}$-Alkylpiperazinyl-$C_{1-6}$-alkylamino-$C_{1-6}$-alkyl; $C_{1-6}$-Alkylpiperazinylsulfonyl; Aminosulfonylpiperazinyl-$C_{1-6}$-alkyloxy; Aminosulfonylpiperazinyl; Aminosulfonylpiperazinyl-$C_{1-6}$-alkyl; Di($C_{1-6}$-alkyl)aminosulfonylpiperazinyl; Di($C_{1-6}$-alkyl)aminosulfonylpiperazinyl-$C_{1-6}$-alkyl; Hydroxy-$C_{1-6}$-alkylpiperazinyl; Hydroxy-$C_{1-6}$-alkylpiperazinyl-$C_{1-6}$-alkyl; $C_{1-6}$-Alkyloxypiperidinyl; $C_{1-6}$-Alkyloxypiperidinyl-$C_{1-6}$-alkyl; Piperidinylamino-$C_{1-6}$-alkylamino; Piperidinylamino-$C_{1-6}$-alkylamino-$C_{1-6}$-alkyl; ($C_{1-6}$-Alkylpiperidinyl)(hydroxy-$C_{1-6}$-alkyl)amino-$C_{1-6}$-alkylamino; ($C_{1-6}$-Alkylpiperidinyl)(hydroxy-$C_{1-6}$-alkyl)amino-$C_{1-6}$-alkylamino-$C_{1-6}$-alkyl; Hydroxy-$C_{1-6}$-alkyloxy-$C_{1-6}$-alkylpiperazinyl; Hydroxy-$C_{1-6}$-alkyloxy-$C_{1-6}$-alkylpiperazinyl-$C_{1-6}$-alkyl; (Hydroxy-$C_{1-6}$-alkyl) ($C_{1-6}$-alkyl)amino; (Hydroxy-$C_{1-6}$-alkyl)($C_{1-6}$-alkyl)amino-$C_{1-6}$-alkyl; Hydroxy-$C_{1-6}$-alkylamino-$C_{1-6}$-alkyl; Di(hydroxy-$C_{1-6}$-alkyl)amino-$C_{1-6}$-alkyl; Pyrrolidinyl-$C_{1-6}$-alkyl; Pyrrolidinyl-$C_{1-6}$-alkyloxy; Pyrazolyl; Thiopyrazolyl; Pyrazolyl substituiert durch zwei Substituenten ausgewählt aus $C_{1-6}$-Alkyl oder Trihalogen-$C_{1-6}$-alkyl; Pyridinyl; Pyridinyl substituiert durch $C_{1-6}$-Alkyloxy, Aryloxy oder Aryl; Pyrimidinyl; Tetrahydropyrimidinylpiperazinyl; Tetrahydropyrimidinylpiperazinyl-$C_{1-6}$-alkyl; Chinolinyl; Indol; Phenyl; Phenyl substituiert durch einen, zwei oder drei Substituenten unabhängig voneinander ausgewählt aus Halogen, Amino, Nitro, $C_{1-6}$-Alkyl, $C_{1-6}$-Alkyloxy, Hydroxy-$C_{1-4}$-alkyl, Trifluormethyl, Trifluormethyloxy, Hydroxy-$C_{1-4}$-alkyloxy, $C_{1-4}$-Alkylsulfonyl, $C_{1-4}$-Alkyloxy-$C_{1-4}$-alkyloxy, $C_{1-4}$-Alkyloxycarbonyl, Amino-$C_{1-4}$-alkyloxy, Di ($C_{1-4}$-alkyl) amino-$C_{1-4}$-alkyloxy, Di($C_{1-4}$-alkyl)amino, Di($C_{1-4}$-alkyl)aminocarbonyl, Di ($C_{1-4}$-alkyl)amino-$C_{1-4}$-alkyl, Di ($C_{1-4}$-alkyl) amino-$C_{1-4}$-alkylamino-$C_{1-4}$-alkyl, Di ($C_{1-4}$-alkyl) amino-($C_{1-4}$-alkyl) amino, Di($C_{1-4}$-alkyl) amino-($C_{1-4}$-alkyl) amino-$C_{1-4}$-alkyl, Di($C_{1-4}$-alkyl)amino-$C_{1-4}$-alkyl-($C_{1-4}$-alkyl) amino, Di($C_{1-4}$-alkyl)amino-$C_{1-4}$-alkyl-($C_{1-4}$-alkyl) amino-$C_{1-4}$-alkyl, Aminosulfonylamino-($C_{1-4}$-alkyl) amino, Aminosulfonylamino-($C_{1-4}$-alkyl)amino-$C_{1-4}$-alkyl, Di($C_{1-4}$-alkyl)aminosulfonylamino-($C_{1-4}$-alkyl) amino, Di($C_{1-4}$-alkyl)aminosulfonyl-

amino-($C_{1-4}$-alkyl)amino-$C_{1-6}$-alkyl, Cyano, Piperidinyl-$C_{1-4}$-alkyloxy, Pyrrolidinyl-$C_{1-4}$-alkyloxy, Aminosulfonylpiperazinyl, Aminosulfonylpiperazinyl-$C_{1-4}$-alkyl, Di($C_{1-4}$-alkyl)aminosulfonylpiperazinyl, Di($C_{1-4}$-alkyl)aminosulfonylpiperazinyl-$C_{1-4}$-alkyl, Hydroxy-$C_{1-4}$-alkylpiperazinyl, Hydroxy-$C_{1-4}$-alkylpiperazinyl-$C_{1-4}$-alkyl, $C_{1-4}$-Alkyloxypiperidinyl, $C_{1-4}$-Alkyloxypiperidinyl-$C_{1-4}$-alkyl, Hydroxy-$C_{1-4}$-alkyloxy-$C_{1-4}$-alkylpiperazinyl, Hydroxy-$C_{1-4}$-alkyloxy-$C_{1-4}$-alkylpiperazinyl-$C_{1-4}$-alkyl, (Hydroxy-$C_{1-4}$-alkyl) ($C_{1-4}$-alkyl) amino, (Hydroxy-$C_{1-4}$alkyl)($C_{1-4}$alkyl)amino-$C_{1-4}$-alkyl, Hydroxy-$C_{1-4}$-alkylamino-$C_{1-4}$-alkyl, Di (hydroxy-$C_{1-4}$-alkyl) amino-$C_{1-4}$-alkyl, Furanyl, Furanyl substituiert durch -CH=CH-CH=CH-, Pyrrolidinyl-$C_{1-4}$-alkyl, Pyrrolidinyl-$C_{1-4}$-alkyloxy, Morpholinyl, Morpholinyl-$C_{1-4}$-alkyloxy, Morpholinyl-$C_{1-4}$-alkyl, Morpholinyl-$C_{1-4}$-alkylamino, Morpholinyl-$C_{1-4}$-alkylamino-$C_{1-4}$-alkyl, Piperazinyl, $C_{1-4}$-Alkylpiperazinyl, $C_{1-4}$-Alkylpiperazinyl-$C_{1-4}$-alkyloxy, Piperazinyl-$C_{1-4}$-alkyl, $C_{1-4}$-Alkylpiperazinyl-$C_{1-4}$-alkyl, $C_{1-4}$-Alkylpiperazinyl-$C_{1-4}$-alkylamino, $C_{1-4}$-Alkylpiperazinyl-$C_{1-4}$-alkylamino-$C_{1-6}$-alkyl, Pyrimidinylpiperazinyl, Pyrimidinylpiperazinyl-$C_{1-4}$-alkyl, Piperidinylamino-$C_{1-4}$-alkylamino, Piperidinylamino-$C_{1-4}$-alkylamino-$C_{1-4}$-alkyl, ($C_{1-4}$-Alkylpiperidinyl)(hydroxy-$C_{1-4}$-alkyl)amino-$C_{1-4}$-alkylamino, ($C_{1-4}$-Alkylpiperidinyl)(hydroxy-$C_{1-4}$-alkyl)amino-$C_{1-4}$-alkylamino-$C_{1-4}$-alkyl, Pyridinyl-$C_{1-4}$-alkyloxy, Hydroxy-$C_{1-4}$-alkylamino, Di(hydroxy-$C_{1-4}$-alkyl)amino, Di ($C_{1-4}$-alkyl) amino-$C_{1-4}$-alkylamino, Aminothiadiazolyl, Aminosulfonylpiperazinyl-$C_{1-4}$-alkyloxy oder Thiophenyl-$C_{1-4}$-alkylamino ausgewählt sind;

sich $R^5$ und $R^6$ jeweils anstelle von Wasserstoff am Stickstoff befinden können;

Aryl oben für Phenyl oder durch einen oder mehrere Substituenten jeweils unabhängig voneinander ausgewählt aus Halogen, $C_{1-6}$-Alkyl, $C_{1-6}$-Alkyloxy, Trifluormethyl, Cyano oder Hydroxycarbonyl substituiertes Phenyl steht.

**2.** Verbindung nach Anspruch 1, wobei

$R^3$ und $R^4$ jeweils unabhängig voneinander aus Wasserstoff, Hydroxy, Hydroxy-$C_{1-6}$-alkyl, Amino-$C_{1-6}$-alkyl oder Aminoaryl ausgewählt sind;

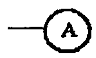

für einen Rest ausgewählt aus (a-1), (a-2), (a-3), (a-4), (a-5), (a-6), (a-7), (a-8), (a-9), (a-10), (a-11), (a-12), (a-13), (a-14), (a-15), (a-16), (a-17), (a-18), (a-19), (a-20), (a-21), (a-22), (a-23), (a-24), (a-25), (a-26), (a-27), (a-28), (a-29), (a-30), (a-31), (a-32), (a-33), (a-34), (a-35), (a-36), (a-37), (a-38), (a-39), (a-40), (a-41), (a-42), (a-43) oder (a-44) steht;

$R^5$ und $R^6$ jeweils unabhängig voneinander aus Wasserstoff; Halogen; Hydroxy; Amino; Nitro; Trihalogen-$C_{1-6}$-alkyl; Trihalogen-$C_{1-6}$-alkyloxy; $C_{1-6}$-Alkyl; $C_{1-6}$-Alkyloxy; $C_{1-6}$-Alkyloxy-$C_{1-6}$-alkyloxy; $C_{1-6}$-Alkylcarbonyl; $C_{1-6}$-Alkylsulfonyl; Cyano-$C_{1-6}$-alkyl; Hydroxy-$C_{1-6}$-alkyl; Hydroxy-$C_{1-6}$-alkyloxy; Hydroxy-$C_{1-6}$-alkylamino; Amino-$C_{1-6}$-alkyloxy; Di($C_{1-6}$-alkyl)aminocarbonyl; Di(hydroxy-$C_{1-6}$-alkyl)amino; Aryl-($C_{1-6}$-alkyl)amino; Di ($C_{1-6}$-alkyl) amino-$C_{1-6}$-alkyloxy; Di($C_{1-6}$-alkyl)amino-$C_{1-6}$-alkylamino; Arylsulfonyl; Arylsulfonylamino; Aryloxy; Aryl-$C_{2-6}$-alkendiyl; Di($C_{1-6}$-alkyl)amino; Di($C_{1-6}$-alkyl)amino-$C_{1-6}$-alkyl; Di ($C_{1-6}$-alkyl) amino-$C_{1-6}$-alkyl-($C_{1-6}$-alkyl)amino-$C_{1-6}$-alkyl; Cyano; Thiophenyl; Thiophenyl substituiert durch Di($C_{1-6}$-alkyl)amino-$C_{1-6}$-alkyl- ($C_{1-6}$-alkyl) amino-$C_{1-6}$-alkyl, Di ($C_{1-6}$-alkyl)amino-$C_{1-6}$-alkyl, $C_{1-6}$-Alkylpiperazinyl-$C_{1-6}$-alkyl oder Di(hydroxy-$C_{1-6}$-alkyl)amino-$C_{1-6}$-alkyl; Furanyl; Imidazolyl; $C_{1-6}$-Alkyltriazolyl; Tetrazolyl; Pyrrolidinyl; Piperidinyl-$C_{1-6}$-alkyloxy; Morpholinyl; $C_{1-6}$-Alkylmorpholinyl; Morpholinyl-$C_{1-6}$-alkyloxy; Morpholinyl-$C_{1-6}$-alkyl; $C_{1-6}$-Alkyl-piperazinyl; $C_{1-6}$-Alkylpiperazinyl-$C_{1-6}$-alkyloxy; $C_{1-6}$-Alkylpiperazinyl-$C_{1-6}$-alkyl; $C_{1-6}$-Alkylpiperazinylsulfonyl; Aminosulfonylpiperazinyl-$C_{1-6}$-alkyloxy; Aminosulfonylpiperazinyl; Aminosulfonylpiperazinyl-$C_{1-6}$-alkyl; Di($C_{1-6}$-alkyl)aminosulfonylpiperazinyl; Di($C_{1-6}$-alkyl)aminosulfonylpiperazinyl-$C_{1-6}$-alkyl; Hydroxy-$C_{1-6}$-alkylpiperazinyl; Hydroxy-$C_{1-6}$-alkylpiperazinyl-$C_{1-6}$-alkyl; $C_{1-6}$-Alkyloxypiperidinyl; $C_{1-6}$-Alkyloxypiperidinyl-$C_{1-6}$-alkyl; Hydroxy-$C_{1-6}$-alkyloxy-$C_{1-6}$-alkylpiperazinyl; Hydroxy$C_{1-6}$-alkyloxy-$C_{1-6}$-alkylpiperazinyl-$C_{1-6}$-alkyl; (Hydroxy-$C_{1-6}$-alkyl) ($C_{1-6}$-alkyl)amino; (Hydroxy-$C_{1-6}$-alkyl) ($C_{1-6}$-alkyl)amino-$C_{1-6}$-alkyl; Pyrrolidinyl-$C_{1-6}$-alkyloxy; Pyrazolyl; Thiopyrazolyl; Pyrazolyl substituiert durch zwei Substituenten ausgewählt aus $C_{1-6}$-Alkyl oder Trihalogen-$C_{1-6}$-alkyl; Pyridinyl; Pyridinyl substituiert durch $C_{1-6}$-Alkyloxy oder Aryl; Pyrimidinyl; Chinolinyl; Indol; Phenyl; Phenyl substituiert durch einen, zwei oder drei Substituenten unabhängig voneinander ausgewählt aus Halogen, Amino, $C_{1-6}$-Alkyl, $C_{1-6}$-Alkyloxy, Hydroxy-$C_{1-4}$-alkyl, Trifluormethyl, Trifluormethyloxy, Hydroxy-$C_{1-4}$-alkyloxy, $C_{1-4}$-Alkyloxy-$C_{1-4}$-alkyloxy, Amino-$C_{1-4}$-alkyloxy, Di($C_{1-4}$-alkyl)amino-$C_{1-4}$-alkyloxy, Di($C_{1-4}$-alkyl) amino, Di($C_{1-4}$-alkyl)amino-$C_{1-4}$-alkyl, Di($C_{1-4}$-alkyl)amino-$C_{1-4}$-alkyl-($C_{1-4}$-alkyl)amino-$C_{1-4}$-alkyl, Piperidinyl-$C_{1-4}$-alkyloxy, Pyrrolidinyl-$C_{1-4}$-alkyloxy, Aminosulfonylpiperazinyl, Aminosulfonylpiperazinyl-$C_{1-4}$-alkyl, Di($C_{1-4}$-alkyl)aminosulfonylpiperazinyl, Di($C_{1-4}$-alkyl)aminosulfonylpiperazinyl-$C_{1-4}$-alkyl, Hydroxy-$C_{1-4}$-alkylpiperazinyl, Hydroxy-

$C_{1-4}$-alkylpiperazinyl-$C_{1-4}$-alkyl, $C_{1-4}$-Alkyloxypiperidinyl, $C_{1-4}$-Alkyloxypiperidinyl-$C_{1-4}$-alkyl, Hydroxy-$C_{1-4}$-alkyloxy-$C_{1-4}$-alkylpiperazinyl, Hydroxy-$C_{1-4}$-alkyloxy-$C_{1-4}$-alkylpiperazinyl-$C_{1-4}$-alkyl, (Hydroxy-$C_{1-4}$-alkyl) ($C_{1-4}$-alkyl)amino, (Hydroxy-$C_{1-4}$-alkyl)($C_{1-4}$-alkyl)amino-$C_{1-4}$-alkyl, Pyrrolidinyl-$C_{1-4}$-alkyloxy, Morpholinyl-$C_{1-4}$-alkyloxy, Morpholinyl-$C_{1-4}$-alkyl, $C_{1-4}$-Alkylpiperazinyl, $C_{1-4}$-Alkylpiperazinyl-$C_{1-4}$-alkyloxy, $C_{1-4}$-Alkylpiperazinyl-$C_{1-4}$-alkyl, Hydroxy-$C_{1-4}$-alkylamino, Di(hydroxy-$C_{1-4}$-alkyl)amino, Di($C_{1-4}$-alkyl)amino-$C_{1-4}$-alkylamino, Aminothiadiazolyl, Aminosulfonylpiperazinyl-$C_{1-4}$-alkyloxy oder Thiophenyl-$C_{1-4}$-alkylamino ausgewählt sind.

**3.** Verbindung nach Anspruch 1, wobei t für 0 steht;

$R^1$ für -C(O)N$R^3R^4$, -C(O)$C_{1-6}$-Alkandiyl-S$R^7$, -N$R^8$C(O)N(OH)$R^7$, -N$R^8$C(O)$C_{1-6}$-Alkandiyl-S$R^7$ oder -N$R^8$C(O)C=N(OH)$R^7$ steht, wobei $R^3$ und $R^4$ jeweils unabhängig voneinander aus Wasserstoff, Hydroxy, Hydroxy-$C_{1-6}$-alkyl oder Amino-$C_{1-6}$-alkyl ausgewählt sind; $R^2$ für Wasserstoff, Hydroxy, Amino, Hydroxy-$C_{1-6}$-alkyl, $C_{1-6}$-Alkyl, $C_{1-6}$-Alkyloxy, Aryl-$C_{1-6}$-alkyl, Aminocarbonyl, Amino-$C_{1-6}$-alkyl, $C_{1-6}$-Alkylamino-$C_{1-6}$-alkyl oder Di($C_{1-6}$-alkyl)amino-$C_{1-6}$-alkyl steht;
-L- für einen zweiwertigen Rest ausgewählt aus -NHC(O)- oder -NH$SO_2$- steht;

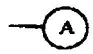

für einen Rest ausgewählt aus (a-1), (a-3), (a-4), (a-5), (a-6), (a-7), (a-8), (a-9), (a-10), (a-11), (a-12), (a-13), (a-14), (a-15), (a-16), (a-17), (a-18), (a-19), (a-20), (a-21), (a-22), (a-23), (a-24), (a-25), (a-26), (a-28), (a-29), (a-30), (a-31), (a-32), (a-33), (a-34), (a-35), (a-36), (a-37), (a-38), (a-39), (a-40), (a-41), (a-42), (a-44), (a-45), (a-46), (a-47), (a-48) oder (a-51) steht;
s jeweils unabhängig für 0, 1, 2, 3 oder 4 steht;

$R^5$ für Wasserstoff; Halogen; Hydroxy; Amino; Nitro; Trihalogen-$C_{1-6}$-alkyl; Trihalogen-$C_{1-6}$-alkyloxy; $C_{1-6}$-Alkyl; $C_{1-6}$-Alkyloxy, $C_{1-6}$-Alkylcarbonyl; $C_{1-6}$-Alkyloxycarbonyl; $C_{1-6}$-Alkylsulfonyl; Hydroxy-$C_{1-6}$-alkyl; Aryloxy; Di($C_{1-6}$-alkyl)amino; Cyano; Thiophenyl; Furanyl; Furanyl substituiert durch Hydroxy-$C_{1-6}$-alkyl; Benzofuranyl; Imidazolyl; Oxazolyl; Oxazolyl substituiert durch Aryl und $C_{1-6}$-alkyl; $C_{1-6}$-Alkyltriazolyl; Tetrazolyl; Pyrrolidinyl; Pyrrolyl; Morpholinyl; $C_{1-6}$-Alkylmorpholinyl; Piperazinyl; $C_{1-6}$-Alkylpiperazinyl; Hydroxy-$C_{1-6}$-alkylpiperazinyl; $C_{1-6}$-Alkyloxypiperidinyl; Pyrazolyl; Pyrazolyl substituiert durch einen oder zwei Substituenten ausgewählt aus $C_{1-6}$-Alkyl oder Trihalogen-$C_{1-6}$-alkyl; Pyridinyl; Pyridinyl substituiert durch $C_{1-6}$-Alkyloxy, Aryloxy oder Aryl; Pyrimidinyl; Chinolinyl; Indol; Phenyl; oder Phenyl substituiert durch einen oder zwei Substituenten unabhängig voneinander ausgewählt aus Halogen, $C_{1-6}$-Alkyl, $C_{1-6}$-Alkyloxy oder Trifluormethyl steht;
und $R^6$ für Wasserstoff; Halogen; Hydroxy; Amino; Nitro; Trihalogen-$C_{1-6}$-alkyl; Trihalogen-$C_{1-6}$-alkyloxy; $C_{1-6}$-Alkyl; $C_{1-6}$-Alkyloxy; $C_{1-6}$-Alkylcarbonyl; $C_{1-6}$-Alkyloxycarbonyl; $C_{1-6}$-Alkylsulfonyl; Hydroxy-$C_{1-6}$-alkyl; Aryloxy; Di($C_{1-6}$-alkyl)amino; Cyano; Pyridinyl; Phenyl; oder Phenyl substituiert durch einen oder zwei Substituenten unabhängig voneinander ausgewählt aus Halogen, $C_{1-6}$-Alkyl, $C_{1-6}$-Alkyloxy oder Trifluormethyl steht.

**4.** Verbindungen nach Anspruch 1 oder 2, wobei t für 0 oder 1 steht; Q jeweils für

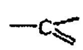

steht; X jeweils für Stickstoff steht; $R^1$ für -C(O)NH(OH) steht; $R^2$ für Wasserstoff, Hydroxy, $C_{1-6}$-Alkyl oder Aryl-$C_{1-6}$-alkyl steht; -L- für einen zweiwertigen Rest ausgewählt aus -NHC(O)- oder -NH$SO_2$- steht;

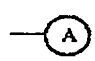

für einen Rest ausgewählt aus (a-1) oder (a-20) steht; s jeweils unabhängig für 0 oder 1 steht; und $R^5$ jeweils unabhängig ausgewählt ist aus Wasserstoff oder Phenyl.

**5.** Verbindung nach einem der Ansprüche 1, 2 und 4, wobei t für 1 steht; Q jeweils für

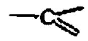

steht; X jeweils für Stickstoff steht; Y jeweils für Stickstoff steht; Z jeweils für -O- oder $-CH_2$-steht; $R^1$ für -C(O)NH (OH) steht; $R^2$ für Wasserstoff steht; -L- für einen zweiwertigen Rest ausgewählt aus -NHC(O)- oder $-NHSO_2$- steht;

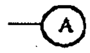

für einen Rest ausgewählt aus (a-1) oder (a-20) steht; s jeweils unabhängig für 0 oder 1 steht; und $R^5$ jeweils unabhängig ausgewählt ist aus Wasserstoff oder Phenyl.

**6.** Verbindung nach einem der Ansprüche 1, 2, 4 und 5 ausgewählt aus den Verbindungen Nr. 4, Nr. 10, Nr. 8, Nr. 6, Nr. 1, Nr. 12 und Nr. 14.

| | |
|---|---|
| 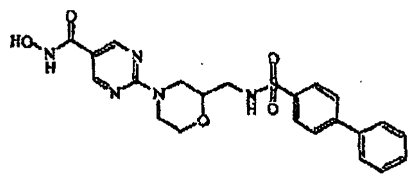 | 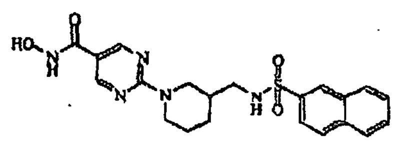 |
| Verb. Nr. 4 | Verb. Nr. 10 |
| 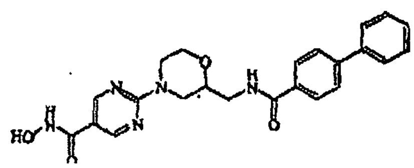 | 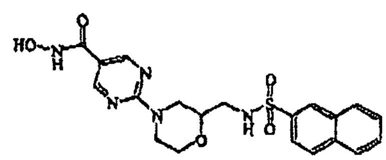 |
| Verb. Nr. 8 | Verb. Nr. 6 |
| 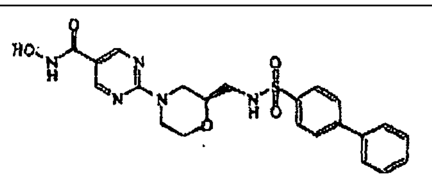 | 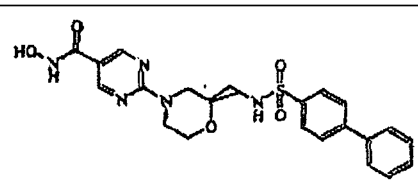 |
| (B); Verb. Nr. 1 | (A); Verb. Nr. 12 |
| 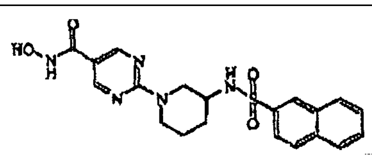 | |
| Verb. Nr. 14 | |

Co.No. = Verb. Nr.

**7.** Pharmazeutische Zusammensetzung, enthaltend pharmazeutisch unbedenkliche Träger und, als Wirkstoff, eine therapeutisch wirksame Menge einer Verbindung nach einem der Ansprüche 1 bis 6.

**8.** Verfahren zur Herstellung einer pharmazeutischen Zusammensetzung nach Anspruch 7, wobei die pharmazeutisch unbedenklichen Träger und eine Verbindung nach einem der Ansprüche 1 bis 6 innig gemischt werden.

**9.** Verbindungen nach einem der Ansprüche 1 bis 6 zur Verwendung als Medizin.

**10.** Verwendung einer Verbindung nach einem der Ansprüche 1 bis 6 zur Herstellung eines Medikaments zur Behandlung von proliferativen Krankheiten.

**11.** Verfahren zur Herstellung einer Verbindung nach Anspruch 1, **dadurch gekennzeichnet, daß** man ein Zwischenprodukt der Formel (II) mit einer geeigneten Säure wie zum Beispiel Trifluoressigsäure zu einer Hydroxansäure der Formel (I-a), in welcher $R^1$ für -C(O)NH(OH) steht, umsetzt.

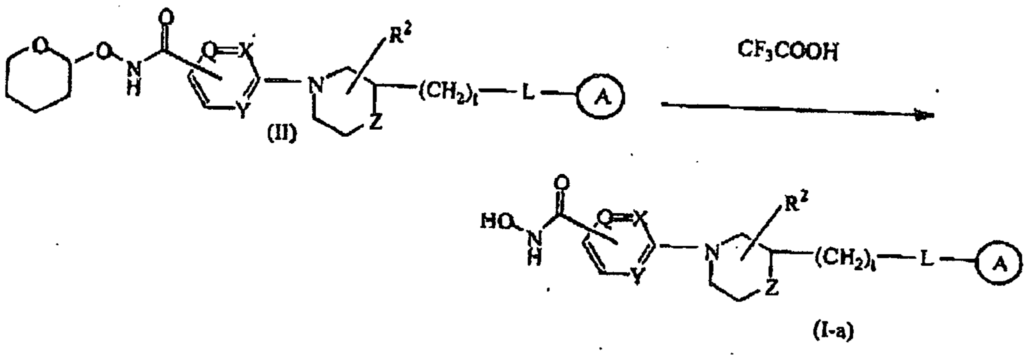


(I-a)

**12.** Verfahren zum Nachweis oder zur Identifizierung einer HDAC in einer biologischen Probe, bei dem man die Bildung eines Komplexes zwischen einer wie in Anspruch 1 definierten markierten Verbindung und einer HDAC nachweist bzw. mißt.

**13.** Kombination eines Antikrebsmittels und eines HDAC-Inhibitors nach einem der Ansprüche 1 bis 6.

**Revendications**

**1.** Composé de formule (I),

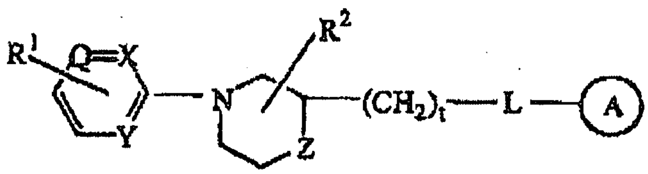 (I)

les formes N-oxyde, les sels d'addition pharmaceutiquement acceptables et les formes stéréochimiquement isomères de celui-ci, dans laquelle :

t est une 0, 1, 2, 3 ou 4 et, lorsque t est 0, alors liaison directe est visée ;
chaque Q est azote ou

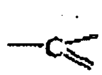 ;

chaque X est azote ou

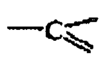 ;

chaque Y est azote ou

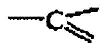 ;

chaque Z est -NH-, -O- ou $-CH_2-$ ;

$R^1$ est $-C(O)NR^3R^4$, $-NHC(O)R^7$, -C(O)-$C_{1-6}$alcanediyl-$SR^7$, $-NR^8C(O)N(OH)R^7$, $-NR^8C(O)C_{1-6}$alcanediyl$SR^7$ ou $-NR^8C(O)C=N(OH)R^7$,

où $R^3$ et $R^4$ sont choisis, chacun indépendamment, parmi hydrogène, hydroxy, $C_{1-6}$alkyle, hydroxy-$C_{1-6}$alkyle, amino$C_{1-6}$alkyle ou aminoaryle ;

$R^7$ est hydrogène, $C_{1-6}$alkyle, $C_{1-6}$alkylcarbonyle, aryl$C_{1-6}$alkyle, $C_{1-6}$alkylpyrazinyle, pyridinone, pyrrolidinone ou méthylimidazolyle ;

$R^8$ est hydrogène ou $C_{1-6}$alkyle ;

$R^2$ est hydrogène, hydroxy, amino, hydroxy$C_{1-6}$alkyle, $C_{1-6}$alkyle, $C_{1-6}$alkyloxy, aryl$C_{1-6}$alkyle, amino-carbonyle, hydroxycarbonyle, amino$C_{1-6}$alkyle, aminocarbonyl$C_{1-6}$alkyle, hydroxycarbonyl$C_{1-6}$-alkyle, hydroxyaminocarbonyle, $C_{1-6}$alkyloxy-carbonyle, $C_{1-6}$alkylamino$C_{1-6}$alkyle ou di($C_{1-6}$-alkyl)amino$C_{1-6}$alkyle ;

-L- est un radical bivalent choisi parmi $-NR^9C(O)-$, $-NR^9SO_2-$ ou $-NR^9CH_2-$, où $R^9$ est hydrogène, $C_{1-6}$alkyle, $C_{3-10}$cycloalkyle, hydroxy$C_{1-6}$alkyle, $C_{1-6}$alkyloxy$C_{1-6}$alkyle ou di($C_{1-6}$alkyl)amino-$C_{1-6}$alkyle ;

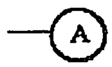

est un radical choisi parmi :

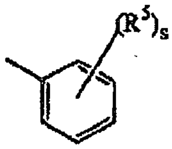

(a-1)

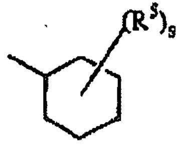

(a-2)

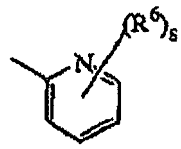

(a-3)

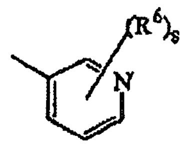

(a-4)

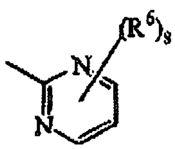

(a-5)

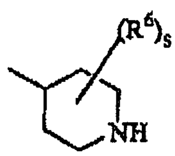

(a-6)

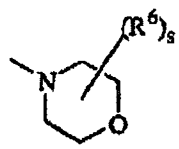

(a-7)

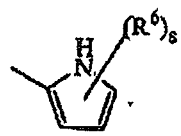

(a-8)

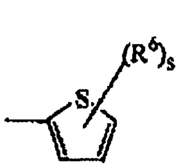

(a-9)

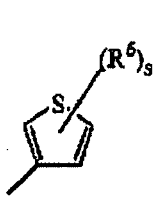

(a-10)

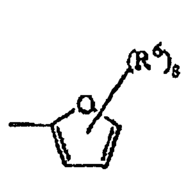

(a-11)

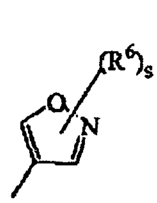

(a-12)

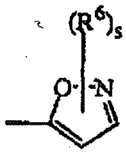
(a-13)

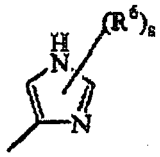
(a-14)

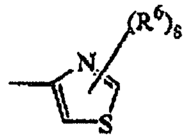
(a-15)

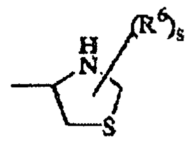
(a-16)

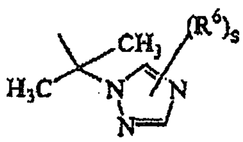
(a-17)

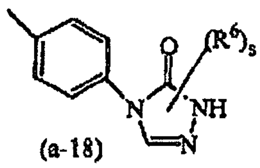
(a-18)

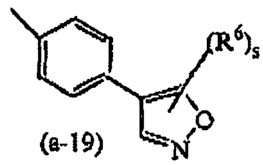
(a-19)

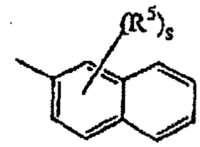
(a-20)

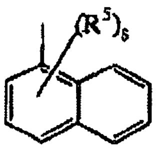
(a-21)

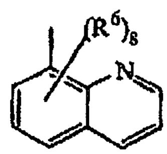
(a-22)

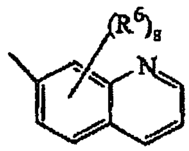
(a-23)

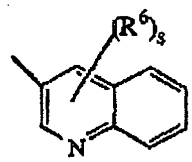
(a-24)

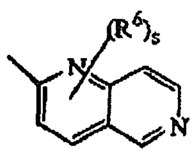
(a-25)

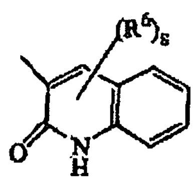
(a-26)

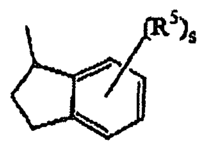
(a-27)

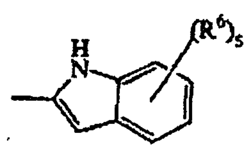
(a-28)

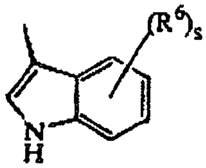
(a-29)

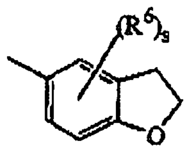
(a-30)

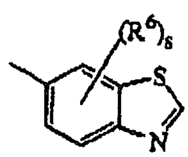
(a-31)

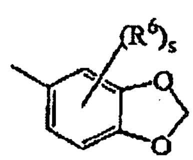
(a-32)

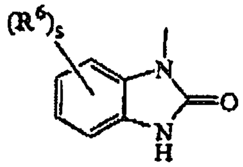

(a-33)

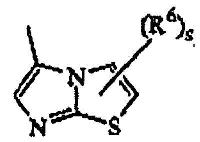

(a-34)

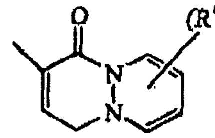

(a-35)

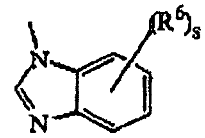

(a-36)

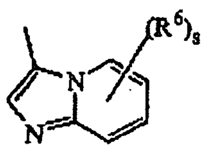

(a-37)

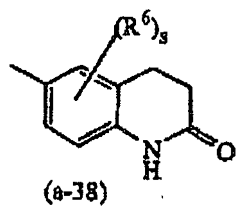

(a-38)

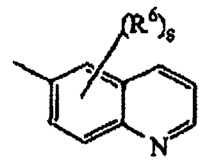

(a-39)

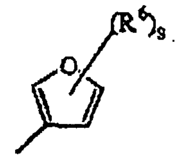

(a-40)

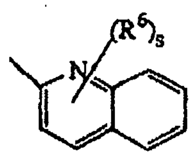

(a-41)

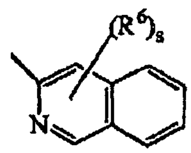

(a-42)

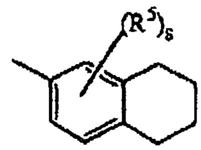

(a-43)

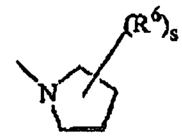

(a-44)

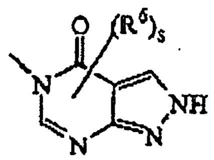

(a-45)

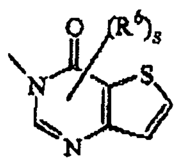

(a-46)

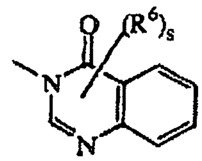

(a-47)

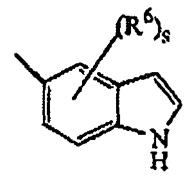

(a-48)

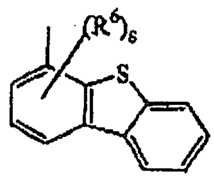

(a-49)

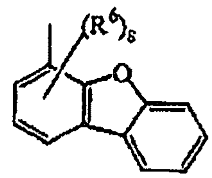

(a-50)

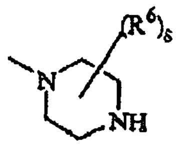

(a-51)

où chaque s est indépendamment 0, 1, 2, 3, 4 ou 5 ;

chaque $R^5$ et $R^6$ sont choisis indépendamment parmi hydrogène ; halogéno ; hydroxy ; amino ; nitro ; trihalogéno$C_{1-6}$alkyle ; trihalogéno-$C_{1-6}$alkyloxy ; $C_{1-6}$alkyle ; $C_{1-6}$alkyle substitué par aryle et $C_{3-10}$cycloalkyle ; $C_{1-6}$alkyloxy ; $C_{1-6}$alkyloxy$C_{1-6}$alkyloxy ; $C_{1-6}$alkylcarbonyle ; $C_{1-6}$alkyloxycarbonyle ; $C_{1-6}$alkylsulfonyle ; cyano$C_{1-6}$alkyle ; hydroxy$C_{1-6}$alkyle ; hydroxy-$C_{1-6}$alkyloxy ; hydroxy$C_{1-6}$alkylamino ; amino-$C_{1-6}$alkyloxy ; di($C_{1-6}$alkyl)aminocarbonyle ; di-(hydroxy$C_{1-6}$alkyl)amino ; (aryl)($C_{1-6}$alkyl)-amino ; di($C_{1-6}$alkyl)amino$C_{1-6}$alkyloxy ; di($C_{1-6}$-alkyl)amino$C_{1-6}$alkylamino; di($C_{1-6}$alkyl)amino-$C_{1-6}$alkylamino$C_{1-6}$alkyle ; arylsulfonyle ; arylsulfonylamino ; aryloxy ; aryloxy$C_{1-6}$alkyle ; aryl$C_{2-6}$alcènediyle ; di($C_{1-6}$alkyl)amino ; di-($C_{1-6}$alkyl)amino$C_{1-6}$alkyle ; di($C_{1-6}$alkyl) amino-($C_{1-6}$alkyl)amine ; di ($C_{1-6}$alkyl)amino ($C_{1-6}$alkyl)-amino$C_{1-6}$alkyle ; di($C_{1-6}$alkyl)amino$C_{1-6}$alkyl-($C_{1-6}$alkyl) amino ; di ($C_{1-6}$alkyl)amino$C_{1-6}$alkyl-($C_{1-6}$alkyl)amino$C_{1-6}$alkyle ; aminosulfonylamino-($C_{1-6}$alkyl)amino ; aminosulfonylamino($C_{1-6}$alkyl)-amino$C_{1-6}$alkyle ; di($C_{1-6}$alkyl)aminosulfonylamino($C_{1-6}$alkyl)amino ; di($C_{1-6}$alkyl)aminosul-

fonylamino($C_{1-6}$alkyl)amino$C_{1-6}$alkyle ; cyano ; thiophényle ; thiophényle substitué par di ($C_{1-6}$alkyl) amino$C_{1-6}$alkyl ($C_{1-6}$alkyl) amino$C_{1-6}$-alkyle, di($C_{1-6}$alkyl)amino$C_{1-6}$alkyle, $C_{1-6}$-alkylpipérazinyl$C_{1-6}$alkyle, hydroxy$C_{1-6}$alkylpipérazinyl$C_{1-6}$alkyle, hydroxy$C_{1-6}$alkyloxy$C_{1-6}$-alkylpipérazinyl$C_{1-6}$alkyle, di($C_{1-6}$alkyl)aminosulfonylpipérazinyl $C_{1-6}$alkyle, $C_{1-6}$alkyloxypipéridinyle, $C_{1-6}$alkyloxypipéridinyl$C_{1-6}$alkyle, morpholinyl$C_{1-6}$alkyle, hydroxy$C_{1-6}$alkyl ($C_{1-6}$-alkyl)amino$C_{1-6}$alkyle, ou di(hydroxy$C_{1-6}$alkyl)-amino$C_{1-6}$alkyle ; furanyle ; furanyle substitué par hydroxy$C_{1-6}$alkyle ; benzofuranyle ; imidazolyle ; oxazolyle ; oxazolyle substitué par aryle et $C_{1-6}$alkyle ; $C_{1-6}$alkyltriazolyle ; tétrazolyle ; pyrrolidinyle ; pyrrolyle ; pipéridinyl$C_{1-6}$alkyloxy ; morpholinyle ; $C_{1-6}$-alkylmorpholinyle ; morpholinyl$C_{1-6}$alkyloxy ; morpholinyl$C_{1-6}$alkyle ; morpholinyl$C_{1-6}$alkylamino ; morpholinyl $C_{1-6}$alkylamino$C_{1-6}$alkyle ; pipérazinyle ; $C_{1-6}$alkylpipérazinyle ; $C_{1-6}$alkylpipérazinyl$C_{1-6}$alkyloxy ; pipérazinyl $C_{1-6}$alkyle ; naphtalénylsulfonylpipérazinyle ; naphtalénylsulfonylpipéridinyle ; naphtalénylsulfonyle ; $C_{1-6}$alkylpipérazinyl$C_{1-6}$alkyle ; $C_{1-6}$alkylpipérazinyl$C_{1-6}$alkylamino ; $C_{1-6}$alkylpipérazinyl-$C_{1-6}$alkylamino$C_{1-6}$alkyle ; $C_{1-6}$ alkylpipérazinylsulfonyle ; aminosulfonylpipérazinyl$C_{1-6}$alkyloxy ; aminosulfonylpipérazinyle ; aminosulfonylpipérazinyl$C_{1-6}$alkyle ; di($C_{1-6}$alkyl)-aminosulfonylpipérazinyle ; di($C_{1-6}$alkyl)aminosulfonylpipérazinyl$C_{1-6}$alkyle ; hydroxy$C_{1-6}$alkylpipérazinyle ; hydroxy$C_{1-6}$alkylpipérazinyl$C_{1-6}$-alkyle ; $C_{1-6}$alkyloxypipéridinyle ; $C_{1-6}$alkyloxypipéridinyl$C_{1-6}$alkyle ; pipéridinylamino$C_{1-6}$-alkylamino ; pipéridinylamino$C_{1-6}$alkylamino-$C_{1-6}$alkyle ; ($C_{1-6}$alkylpipéridinyl)(hydroxy$C_{1-6}$-alkyl)amino$C_{1-6}$alkylamino ; ($C_{1-6}$alkylpipéridinyl)-(hydroxy$C_{1-6}$alkyl)amino$C_{1-6}$alkylamino $C_{1-6}$alkyle ; hydroxy$C_{1-6}$alkyloxy$C_{1-6}$alkylpipérazinyle ; hydroxy-$C_{1-6}$alkyloxy$C_{1-6}$alkylpipérazinyl$C_{1-6}$alkyle ; (hydroxy$C_{1-6}$alkyl)($C_{1-6}$alkyl)amino ; (hydroxy$C_{1-6}$-alkyl)($C_{1-6}$alkyl)amino$C_{1-6}$alkyle ; hydroxy$C_{1-6}$-alkylamino$C_{1-6}$ alkyle ; di(hydroxy$C_{1-6}$alkyl)amino-$C_{1-6}$alkyle ; pyrrolidinyl$C_{1-6}$alkyle ; pyrrolidinyl$C_{1-6}$alkyloxy ; pyrazolyle ; thiopyrazolyle ; pyrazolyle substitué par deux substituants choisis parmi $C_{1-6}$alkyle ou trihalogéno$C_{1-6}$alkyle ; pyridinyle ; pyridinyle substitué par $C_{1-6}$alkyloxy, aryloxy ou aryle ; pyrimidinyle ; tétrahydropyrimidinylpipérazinyle ; tétrahydropyrimidinylpipérazinyl$C_{1-6}$alkyle ; quinoléinyle ; indole ; phényle ; phényle substitué par un, deux ou trois substituants choisis indépendamment parmi halogéno, amino, nitro, $C_{1-6}$alkyle, $C_{1-6}$alkyloxy, hydroxy$C_{1-4}$alkyle, trifluorométhyle, trifluorométhyloxy, hydroxy-$C_{1-4}$alkyloxy, $C_{1-4}$alkylsulfonyle, $C_{1-4}$alkyloxy-$C_{1-4}$alkyloxy, $C_{1-4}$alkyloxycarbonyle, amino$C_{1-4}$-alkyloxy, di ($C_{1-4}$alkyl)amino$C_{1-4}$alkyloxy, di-($C_{1-4}$alkyl) amino, di($C_{1-4}$alkyl)aminocarbonyle, di ($C_{1-4}$alkyl)amino$C_{1-4}$alkyle, di($C_{1-4}$alkyl)amino-$C_{1-4}$alkylamino$C_{1-4}$alkyle, di($C_{1-4}$alkyl)amino-($C_{1-4}$alkyl)amino, di ($C_{1-4}$alkyl) amino($C_{1-4}$alkyl)-amino$C_{1-4}$alkyle, di($C_{1-4}$alkyl)amino$C_{1-4}$alkyl($C_{1-4}$-alkyl) amino, di ($C_{1-4}$alkyl)amino$C_{1-4}$alkyl ($C_{1-4}$-alkyl)amino$C_{1-4}$alkyle, aminosulfonylamino($C_{1-4}$-alkyl) amino, aminosulfonylamino ($C_{1-4}$alkyl)amino-$C_{1-4}$alkyle, di ($C_{1-4}$alkyl)aminosulfonylamino($C_{1-4}$-alkyl) amino, di($C_{1-4}$alkyl)aminosulfonylamino-($C_{1-4}$alkyl)amino$C_{1-6}$alkyle, cyano, pipéridinyl-$C_{1-4}$alkyloxy, pyrrolidinyl$C_{1-4}$alkyloxy, aminosulfonylpipérazinyle, aminosulfonylpipérazinyl-$C_{1-4}$alkyle, di($C_{1-4}$alkyl)aminosulfonylpipérazinyle, di ($C_{1-4}$alkyl) aminosulfonylpipérazinyl$C_{1-4}$alkyle, hydroxy$C_{1-4}$alkylpipérazinyle, hydroxy$C_{1-4}$alkylpipérazinyl$C_{1-4}$alkyle, $C_{1-4}$alkyloxypipéridinyle, $C_{1-4}$alkyloxypipéridinyl$C_{1-4}$alkyle, hydroxy$C_{1-4}$-alkyloxy$C_{1-4}$alkylpipérazinyle, hydroxy$C_{1-4}$alkyloxy$C_{1-4}$alkylpipérazinyl$C_{1-4}$alkyle, (hydroxy$C_{1-4}$-alkyl)($C_{1-4}$alkyl)amino, (hydroxy$C_{1-4}$alkyl)($C_{1-4}$-alkyl)amino$C_{1-4}$alkyle, hydroxy $C_{1-4}$alkylamino-$C_{1-4}$alkyle, di (hydroxy$C_{1-4}$alkyl)amino$C_{1-4}$alkyle, furanyle, furanyle substitué par -CH=CH-CH=CH-, pyrrolidinyl$C_{1-4}$alkyle, pyrrolidinyl$C_{1-4}$alkyloxy, morpholinyle, morpholinyl$C_{1-4}$alkyloxy, morpholinyl$C_{1-4}$alkyle, morpholinyl$C_{1-4}$alkylamino, morpholinyl$C_{1-4}$alkylamino$C_{1-4}$alkyle, pipérazinylé, $C_{1-4}$alkylpipérazinyle, $C_{1-4}$alkylpipérazinyl$C_{1-4}$alkyloxy, pipérazinyl$C_{1-4}$alkyle, $C_{1-4}$alkylpipérazinyl$C_{1-4}$alkyle, $C_{1-4}$alkylpipérazinyl$C_{1-4}$alkylamino, $C_{1-4}$alkylpipérazinyl-$C_{1-4}$alkylamino$C_{1-6}$alkyle, pyrimidinylpipérazinyle, pyrimidinylpipérazinyl$C_{1-4}$alkyle, pipéridinylamino$C_{1-4}$alkylamino, pipéridinylamino$C_{1-4}$alkylamino$C_{1-4}$alkyle, ($C_{1-4}$alkylpipéridinyl)(hydroxy-$C_{1-4}$alkyl)amino $C_{1-4}$alkylamino, ($C_{1-4}$alkylpipéridinyl)(hydroxy$C_{1-4}$alkyl)amino$C_{1-4}$alkylamino$C_{1-4}$-alkyle, pyridinyl$C_{1-4}$alkyloxy, hydroxy-$C_{1-4}$alkylamino, di(hydroxy$C_{1-4}$alkyl)amino, di-($C_{1-4}$alkyl)amino$C_{1-4}$alkylamino, aminothiadiazolyle, aminosulfonylpipérazinyl$C_{1-4}$alkyloxy, ou thiophényl$C_{1-4}$alkylamino ;

chaque $R^5$ et $R^6$ peuvent être placés sur l'azote en remplacement de l'hydrogène ;

aryle, dans ce qui précède, est phényle ou phényle substitué par un ou plusieurs substituants choisis, chacun indépendamment, parmi halogéno, $C_{1-6}$alkyle, $C_{1-6}$alkyloxy, trifluorométhyle, cyano ou hydroxycarbonyle.

**2.** Composé selon la revendication 1, **caractérisé en ce que** :

$R^3$ et $R^4$ sont choisis, chacun indépendamment, parmi hydrogène, hydroxy, hydroxy$C_{1-6}$alkyle, amino-$C_{1-6}$alkyle ou aminoaryle ;

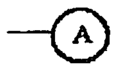

est un radical choisi parmi (a-1), (a-2), (a-3), (a-4), (a-5), (a-6), (a-7), (a-8), (a-9), (a-10), (a-11), (a-12), (a-13), (a-14), (a-15), (a-16), (a-17), (a-18), (a-19), (a-20), (a-21), (a-22), (a-23), (a-24), (a-25), (a-26), (a-27), (a-28), (a-29),

(a-30), (a-31), (a-32), (a-33), (a-34), (a-35), (a-36), (a-37), (a-38), (a-39), (a-40), (a-41), (a-42), (a-43) ou (a-44) ; chaque $R^5$ et $R^6$ sont indépendamment choisis parmi hydrogène ; halogéno ; hydroxy ; amino ; nitro ; trihalogéno$C_{1-6}$alkyle ; trihalogéno$C_{1-6}$alkyloxy ; $C_{1-6}$alkyle ; $C_{1-6}$alkyloxy ; $C_{1-6}$-alkyloxy$C_{1-6}$alkyloxy ; $C_{1-6}$alkylcarbonyle ; $C_{1-6}$-alkylsulfonyle ; cyano$C_{1-6}$alkyle ; hydroxy-$C_{1-6}$alkyle ; hydroxy$C_{1-6}$alkyloxy ; hydroxy$C_{1-6}$-alkylamino ; amino$C_{1-6}$alkyloxy ; di ($C_{1-6}$alkyl)-aminocarbonyle ; di(hydroxy$C_{1-6}$alkyl)amino ; aryl($C_{1-6}$alkyl)amino ; di($C_{1-6}$alkyl) amino$C_{1-6}$-alkyloxy ; di($C_{1-6}$alkyl)amino$C_{1-6}$alkylamino ; arylsulfonyle ; arylsulfonylamino ; aryloxy ; aryl$C_{2-6}$alcènediyle ; di($C_{1-6}$alkyl)amino ; di-($C_{1-6}$alkyl)amino$C_{1-6}$alkyle ; di($C_{1-6}$alkyl)amino-$C_{1-6}$alkyl($C_{1-6}$alkyl) amino$C_{1-6}$alkyle ; cyano ; thiophényle ; thiophényle substitué par di($C_{1-6}$alkyl)amino$C_{1-6}$alkyl($C_{1-6}$alkyl)amino $C_{1-6}$-alkyle, di ($C_{1-6}$alkyl)amino$C_{1-6}$alkyle, $C_{1-6}$alkyl-pipérazinyl$C_{1-6}$alkyle ou di(hydroxy$C_{1-6}$alkyl)-amino$C_{1-6}$alkyle ; furanyle ; imidazolyle ; $C_{1-6}$alkyltriazolyle ; tétrazolyle ; pyrrolidinyle ; pipéridinyl$C_{1-6}$alkyloxy ; morpholinyle ; $C_{1-6}$alkylmorpholinyle ; morpholinyl-$C_{1-6}$alkyloxy ; morpholinyl$C_{1-6}$alkyle ; $C_{1-6}$alkylpipérazinyle ; $C_{1-6}$alkylpipérazinyl$C_{1-6}$alkyloxy ; $C_{1-6}$alkylpipérazinyl$C_{1-6}$alkyle ; $C_{1-6}$alkylpipérazinylsulfonyle ; aminosulfonylpipérazinyl-$C_{1-6}$alkyloxy ; aminosulfonylpipérazinyle ; aminosulfonylpipérazinyl$C_{1-6}$alkyle ; di($C_{1-6}$-alkyl)aminosulfonylpipérazinyle ; di($C_{1-6}$alkyl)-aminosulfonylpipérazinyl$C_{1-6}$alkyle ; hydroxy-$C_{1-6}$alkylpipérazinyle ; hydroxy$C_{1-6}$alkylpipérazinyl$C_{1-6}$alkyle ; $C_{1-6}$alkyloxypipéridinyle ; $C_{1-6}$alkyloxypipéridinyl$C_{1-6}$alkyle ; hydroxy-$C_{1-6}$alkyloxy$C_{1-6}$alkylpipérazinyle ; hydroxy-$C_{1-6}$alkyloxy$C_{1-6}$alkylpipérazinyl$C_{1-6}$alkyle ; (hydroxy$C_{1-6}$alkyl)($C_{1-6}$alkyl)amino ; (hydroxy-$C_{1-6}$alkyl)($C_{1-6}$alkyl) amino$C_{1-6}$alkyle ; pyrrolidinyl$C_{1-6}$alkyloxy ; pyrazolyle ; thiopyrazolyle ; pyrazolyle substitué par deux substituants choisis parmi $C_{1-6}$alkyle ou trihalogéno$C_{1-6}$alkyle ; pyridinyle ; pyridinyle substitué par $C_{1-6}$alkyloxy ou aryle ; pyrimidinyle ; quinoléinyle ; indole ; phényle ; phényle substitué par un, deux ou trois substituants choisis indépendamment parmi halogéno, amino, $C_{1-6}$alkyle, $C_{1-6}$alkyloxy, hydroxy$C_{1-4}$alkyle, trifluorométhyle, trifluorométhyloxy, hydroxy$C_{1-4}$alkyloxy, $C_{1-4}$alkyloxy-$C_{1-4}$alkyloxy, amino$C_{1-4}$alkyloxy, di($C_{1-4}$alkyl)-amino$C_{1-4}$alkyloxy, di ($C_{1-4}$alkyl) amino, di($C_{1-4}$-alkyl)amino$C_{1-4}$alkyle, di ($C_{1-4}$alkyl) amino$C_{1-4}$-alkyl($C_{1-4}$alkyl)amino$C_{1-4}$alkyle, pipéridinyl-$C_{1-4}$alkyloxy, pyrrolidinyl$C_{1-4}$alkyloxy, aminosulfonylpipérazinyle, aminosulfonylpipérazinyl-$C_{1-4}$alkyle, di ($C_{1-4}$alkyl)aminosulfonylpipérazinyle, di($C_{1-4}$alkyl)aminosulfonylpipérazinyl-$C_{1-4}$alkyle, hydroxy$C_{1-4}$alkylpipérazinyle, hydroxy$C_{1-4}$alkylpipérazinyl$C_{1-4}$alkyle, $C_{1-4}$alkyloxypipéridinyle, $C_{1-4}$alkyloxypipéridinyl$C_{1-4}$alkyle, hydroxy $C_{1-4}$alkyloxy$C_{1-4}$alkylpipérazinyle, hydroxy$C_{1-4}$alkyloxy$C_{1-4}$-alkylpipérazinyl$C_{1-4}$alkyle, (hydroxy$C_{1-4}$alkyl)-($C_{1-4}$alkyl)amino, (hydroxy$C_{1-4}$alkyl)($C_{1-4}$alkyl)-amino$C_{1-4}$alkyle, pyrrolidinyl$C_{1-4}$alkyloxy, morpholinyl$C_{1-4}$alkyloxy, morpholinyl$C_{1-4}$alkyle, $C_{1-4}$alkylpipérazinyle, $C_{1-4}$alkylpipérazinyl-$C_{1-4}$alkyloxy, $C_{1-4}$alkylpipérazinyl$C_{1-4}$alkyle, hydroxy$C_{1-4}$alkylamino, di(hydroxy$C_{1-4}$alkyl)amino, di($C_{1-4}$alkyl)amino$C_{1-4}$alkylamino, aminothiadiazolyle, aminosulfonylpipérazinyl$C_{1-4}$alkyloxy ou thiophényl$C_{1-4}$alkylamino.

**3.** Composé selon la revendication 1, **caractérisé en ce que** t est 0 ;

$R^1$ est -C (O)N$R^3R^4$, -C(O)-$C_{1-6}$alcanediylS$R^7$, -N$R^8$C(O)N (OH) $R^7$, -N$R^8$C(O)$C_{1-6}$alcanediylS$R^7$ ou- N$R^8$C (O)C=N(OH) $R^7$, où $R^3$ et $R^4$ sont choisis, chacun indépendamment, parmi hydrogène, hydroxy, hydroxy$C_{1-6}$alkyle ou amino$C_{1-6}$alkyle ;

$R^2$ est hydrogène, hydroxy, amino, hydroxy-$C_{1-6}$alkyle, $C_{1-6}$alkyle, $C_{1-6}$alkyloxy, aryl-$C_{1-6}$alkyle, aminocarbonyle, amino$C_{1-6}$alkyle, $C_{1-6}$alkylamino$C_{1-6}$alkyle ou di ($C_{1-6}$alkyl) amino-$C_{1-6}$alkyle ;

-L- est un radical bivalent choisi parmi -NHC(O)- ou -NHSO$_2$- ;

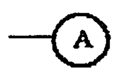

est un radical choisi parmi (a-1), (a-3), (a-4), (a-5), (a-6), (a-7), (a-8), (a-9), (a-10), (a-11), (a-12), (a-13), (a-14), (a-15), (a-16), (a-17), (a-18), (a-19), (a-20), (a-21), (a-22), (a-23), (a-24), (a-25), (a-26), (a-28), (a-29), (a-30), (a-31), (a-32), (a-33), (a-34), (a-35), (a-36), (a-37), (a-38), (a-39), (a-40), (a-41), (a-42), (a-44), (a-45), (a-46), (a-47),

(a-48) ou (a-51) ;

chaque s est indépendamment 0, 1, 2, 3 ou 4 ;

$R^5$ est hydrogène ; halogéno ; hydroxy ; amino ; nitro ; trihalogéno$C_{1-6}$alkyle ; trihalogéno-$C_{1-6}$alkyloxy ; $C_{1-6}$alkyle ; $C_{1-6}$alkyloxy $C_{1-6}$-alkylcarbonyle ; $C_{1-6}$alkyloxycarbonyle ; $C_{1-6}$-alkylsulfonyl ; hydroxy$C_{1-6}$alkyle ; aryloxy ; di($C_{1-6}$alkyl)amino ; cyano ; thiophényle ; furanyle ; furanyle substitué par hydroxy$C_{1-6}$alkyle ; benzofuranyle ; imidazolyle ; oxazolyle ; oxazolyle substitué par aryle et $C_{1-6}$alkyle ; $C_{1-6}$alkyltriazolyle ; tétrazolyle ; pyrrolidinyle ; pyrrolyle ; morpholinyle ; $C_{1-6}$alkylmorpholinyle ; pipérazinyle ; $C_{1-6}$alkylpipérazinyle ; hydroxy$C_{1-6}$alkylpipérazinyle ; $C_{1-6}$alkyloxypipéridinyle ; pyrazolyle ; pyrazolyle substitué par un ou deux substituants choisis parmi $C_{1-6}$alkyle ou trihalogéno$C_{1-6}$alkyle ; pyridinyle ; pyridinyle substitué par $C_{1-6}$alkyloxy,

aryloxy ou aryle ; pyrimidinyle ; quinoléinyle ; indole ; phényle ou phényle substitué par un ou deux substituants choisis indépendamment parmi halogéno, $C_{1-6}$alkyle, $C_{1-6}$alkyloxy ou trifluorométhyle ;
et $R^6$ est hydrogène ; halogéno ; hydroxy ; amino ; nitro ; trihalogéno$C_{1-6}$alkyle ; trihalogéno-$C_{1-6}$alkyloxy ; $C_{1-6}$alkyle ; $C_{1-6}$alkyloxy ; $C_{1-6}$-alkylcarbonyle ; $C_{1-6}$alkyloxycarbonyle ; $C_{1-6}$-alkylsulfonyle ; hydroxy$C_{1-6}$alkyle ; aryloxy ; di($C_{1-6}$alkyl)amino ; cyano ; pyridinyle ; phényle ou phényle substitué par un ou deux substituants choisis indépendamment parmi halogéno, . $C_{1-6}$alkyle, $C_{1-6}$alkyloxy ou trifluorométhyle.

**4.** Composé selon les revendications 1 et 2, **caractérisé en ce que** t est 0 ou 1 ; chaque Q est

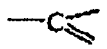 ;

chaque X est azote ; $R^1$ est -C(O)NH(OH) ; $R^2$ est hydrogène, hydroxy, $C_{1-6}$alkyle ou aryl$C_{1-6}$alkyle ; -L- est un radical bivalent choisi parmi -NHC(O)- ou -$NHSO_2$- ;

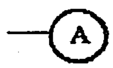

est un radical choisi parmi (a-1) ou (a-20) ; chaque s est indépendamment 0 ou 1 ; et chaque $R^5$ est choisi indépendamment parmi hydrogène ou phényle.

**5.** Composé selon les revendications 1, 2 et 4, **caractérisé en ce que** t est 1 ; chaque Q est

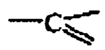 ;

chaque X est azote ; chaque Y est azote ; chaque Z est -O- ou -$CH_2$- ; $R^1$ est -C(O)NH(OH) ; $R^2$ est hydrogène ; -L- est un radical bivalent choisi parmi -NHC(O)- ou -$NHSO_2$- ;

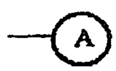

(a-20) ; chaque s est indépendamment 0 ou 1 ; et chaque $R^5$ est choisi indépendamment parmi hydrogène ou phényle.

**6.** Composé selon les revendications 1, 2, 4 et 5, choisi parmi les composés n° 4, n° 10, n° 8, n° 6, n° 1, n° 12 et n° 14 :

| | |
|---|---|
| 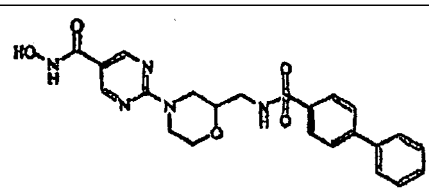 | 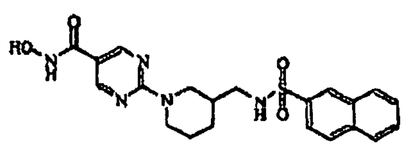 |
| Co. n° 4 | Co. n° 10 |

(suite)

| | |
|---|---|
| 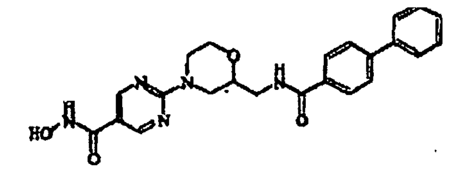 | 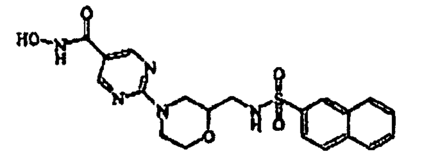 |
| Co. n° 8 | Co. n° 6 |
| 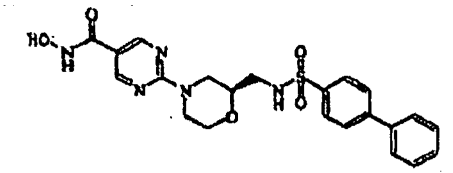 | 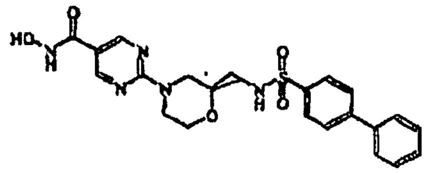 |
| (B) ; Co. n° 1 | (A); Co. n° 12 |
| 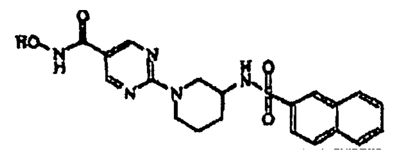 | |
| Co. n° 14 | |

**7.** Composition pharmaceutique comprenant des supports pharmaceutiquement acceptables et, à titre de principe actif, une quantité thérapeutiquement efficace d'un composé selon les revendications 1 à 6.

**8.** Procédé de préparation d'une composition pharmaceutique selon la revendication 7, **caractérisé en ce que** les supports pharmaceutiquement acceptables et un composé selon les revendications 1 à 6 sont mélangés intimement.

**9.** Composé selon l'une quelconque des revendications 1 à 6, destiné à être utilisé comme médicament.

**10.** Utilisation d'un composé selon l'une quelconque des revendications 1 à 6, pour la fabrication d'un médicament destiné au traitement de maladies prolifératives.

**11.** Procédé de préparation d'un composé selon la revendication 1, **caractérisé par** la réaction d'un intermédiaire de formule (II) avec un acide approprié tel que, par exemple, l'acide trifluoro-acétique, donnant un acide hydroxamique de formule (I-a), où $R^1$ est -C(O)NH(OH).

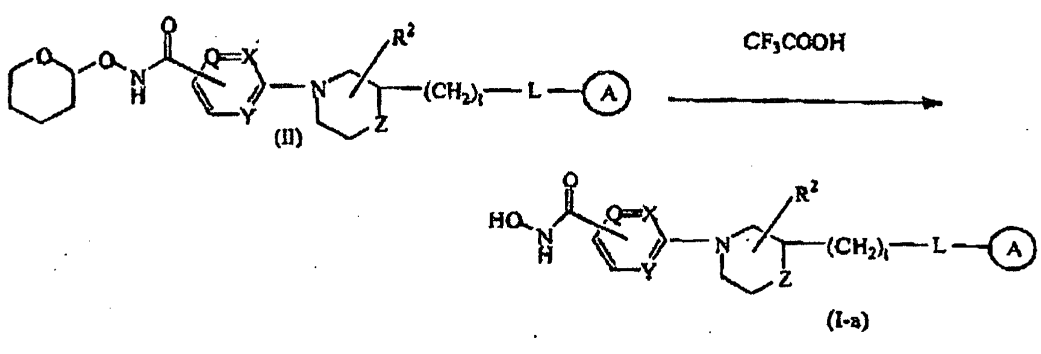

(II)
(I-a)

**12.** Méthode de détection ou d'identification d'une HDAC dans un échantillon biologique comprenant la détection ou la mesure de la formation d'un complexe entre un composé marqué, tel que défini dans la revendication 1, et une HDAC.

**13.** Combinaison d'un agent anticancéreux et d'un inhibiteur d'HDAC selon l'une quelconque des revendications 1 à 6.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0827742 A, Geerts
- WO 0138322 A
- WO 0170675 A


### Non-patent literature cited in the description


- **MARKS et al.** *Nature Reviews: Cancer,* 2001, vol. 1, 194-202
- **FINNIN et al.** *Nature,* 1999, vol. 401, 188-193
- **B.BUNIN.** The Combinatorial Index. Academic Press
- **NOVABIOCHEM'S.** Catalogue & Peptide Synthesis Handbook. Novabiochem AG, 1999
- **MOSMANN TIM.** *Journal of Immunological Methods,* 1983, vol. 65, 55-63
- Probit Analyses. **FINNEY, D.J.** Graded Responses. Cambridge University Press, 1962
- **GORROD et al.** *Xenobiotica,* 1975, vol. 5, 453-462